# EUROPÄISCHE PATENTSCHRIFT

(11) **EP 0 931 070 B1**
(45) Veröffentlichungstag und Bekanntmachung des Hinweises auf die Patenterteilung: **19.03.2003**
(21) Anmeldenummer: 97941998.3
(22) Anmeldetag: 09.09.1997
(51) Int. Cl.: C07D 215/14, A01N 43/42, C07D 215/18, C07D 215/36, C07D 217/22, C07D 215/22, C07D 215/26

(54) **HETAROYLCYCLOHEXANDIONDERIVATE MIT HERBIZIDER WIRKUNG**
HETAROYL CYCLOHEXANEDIONE DERIVATIVES WITH HERBICIDAL EFFECT
DERIVES D'HETAROYLCYCLOHEXANDIONE A ACTION HERBICIDE

(30) Priorität: 20.09.1996 DE 19638486
(43) Veröffentlichungstag der Anmeldung: 28.07.1999
(73) Patentinhaber: BASF AKTIENGESELLSCHAFT, 67056 Ludwigshafen (DE)
(72) Erfinder: OTTEN, Martina, D-67069 Ludwigshafen (DE); GÖTZ, Norbert, D-67547 Worms (DE); VON DEYN, Wolfgang, D-67435 Neustadt (DE); ENGEL, Stefan, D-65510 Idstein (DE); KARDORFF, Uwe, D-68159 Mannheim (DE); PLATH, Peter, D-67227 Frankenthal (DE); HILL, Regina, Luise, D-67346 Speyer (DE); WITSCHEL, Matthias, D-67061 Ludwigshafen (DE); MISSLITZ, Ulf, D-67433 Neustadt (DE); WESTPHALEN, Karl-Otto, D-67346 Speyer (DE); WALTER, Helmut, D-67283 Obrigheim (DE)
(86) Internationale Anmeldenummer: EP9704894
(87) Internationale Veröffentlichungsnummer: WO98012180

(56) Entgegenhaltungen:
- EP-A- 0 283 261
- EP-A- 0 563 817

## Beschreibung

Die vorliegende Erfindung betrifft neue Hetaroylderivate der Formel I 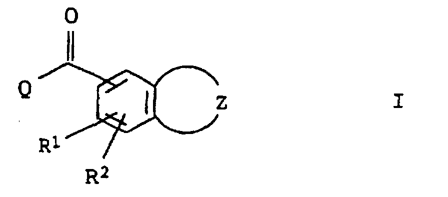 in der die Variablen folgende Bedeutung haben:
- R¹, R²: Wasserstoff, Nitro, Halogen, Cyano, Rhodano, Hydroxy, Mercapto, C₁-C₆-Alkyl, C₁-C₆-Halogenalkyl, C₂-C₆-Alkenyl, C₂-C₆-Alkinyl, C₁-C₆-Alkoxy, C₁-C₆-Halogenalkoxy, C₂-C₆-Alkenyloxy, C₂-C₆-Alkinyloxy, C₁-C₆-Alkylthio, C₁-C₆-Halogenalkylthio, C₂-C₆-Alkenylthio, C₂-C₆-Alkinylthio, C₁-C₆-Alkylsulfinyl, C₁-C₆-Halogenalkylsulfinyl, C₂-C₆-Alkenylsulfinyl, C₂-C₆-Alkinylsulfinyl, C₁-C₆-Alkylsulfonyl, C₁-C₆-Halogenalkylsulfonyl, C₂-C₆-Alkenylsulfonyl, C₂-C₆-Alkinylsulfonyl, C₁-C₆-Alkoxysulfonyl, C₁-C₆-Halogenalkoxysulfonyl, C₂-C₆-Alkenyloxysulfonyl, C₂-C₆-Alkinyloxysulfonyl, Phenyl, Phenyloxy, Phenylthio, Phenylsulfinyl oder Phenylsulfonyl, wobei die fünf letztgenannten Substituenten partiell oder vollständig halogeniert sein können und ein bis drei der folgenden Gruppen tragen können:
Nitro, Cyano, Hydroxy, C₁-C₄-Alkyl, C₁-C₄-Halogenalkyl, C₁-C₄-Alkoxy, C₁-C₄-Halogenalkoxy;
- Z: ein Baustein aus der Gruppe Z¹ bis Z¹² 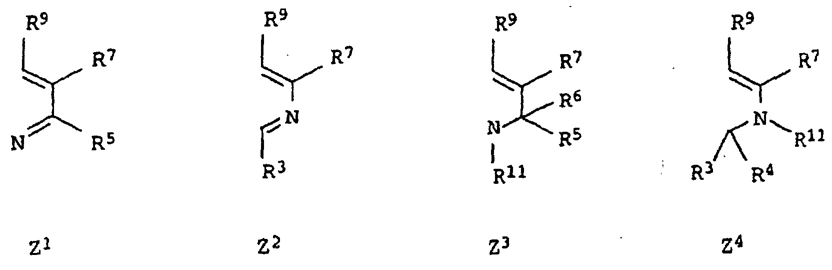 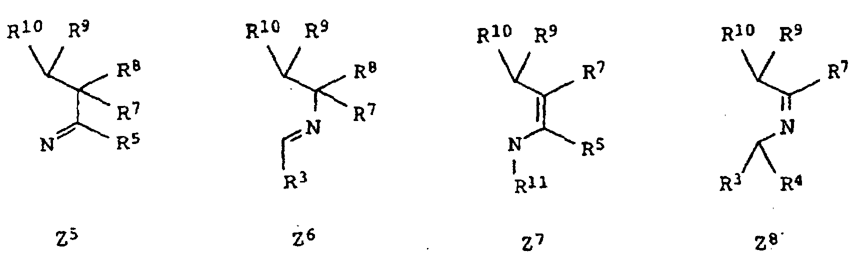 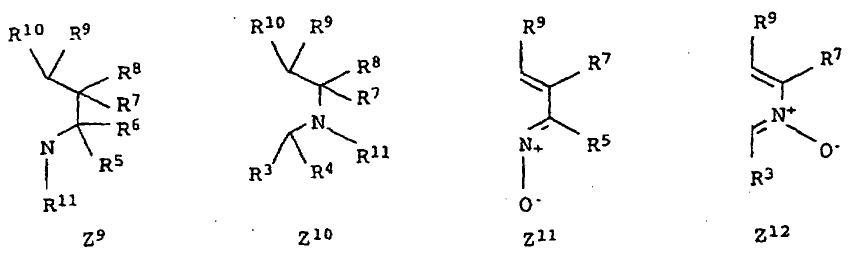 wobei
R³, R⁵, R⁷, R⁹ Nitro, Cyano, Hydroxy, Mercapto, C₂-C₄-Alkenyl, C₂-C₄-Alkinyl, C₂-C₄-Alkenyloxy, C₂-C₄-Alkinyloxy, C₂-C₄-Alkenylthio, C₂-C₄-Alkinylthio, C₁-C₄-Alkylsulfinyl, C₁-C₄-Halogenalkylsulfinyl, C₂-C₄-Alkenylsulfinyl, C₂-C₄-Alkinylsulfinyl, C₁-C₄-Alkylsulfonyl, C₁-C₄-Halogenalkylsulfonyl, C₂-C₄-Alkenylsulfonyl, C₂-C₄-Alkinylsulfonyl, C₁-C₄-Alkoxysulfonyl, C₁-C₄-Halogenalkoxysulfonyl, C₂-C₄-Alkenyloxysulfonyl, C₂-C₄-Alkinyloxysulfonyl, -NR¹²R¹³, -CO₂R¹², -CONR¹²R¹³, Phenyl, Phenoxy, Phenylthio, Phenylsulfinyl oder Phenylsulfonyl bedeuten, wobei die fünf letztgenannten Substituenten partiell oder vollständig halogeniert sein können und ein bis drei der folgenden Gruppen tragen können:
Nitro, Cyano, Hydroxy, C₁-C₄-Alkyl, C₁-C₄-Halogenalkyl, C₁-C₄-Alkoxy, C₁-C₄-Halogenalkoxy;
sowie die unter R⁴ genannten Reste;
R⁴, R^{6,} R⁸, R¹⁰ Wasserstoff, Halogen, C₁-C₄-Alkyl, C₁-C₄-Halogenalkyl, C₁-C₄-Alkoxy, C₁-C₄-Halogenalkoxy, C₁-C₄-Alkylthio oder C₁-C₄-Halogenalkylthio bedeuten; oder
eine -CR³R⁴-, -CR⁵R⁶-, -CR⁷R⁸-, -CR⁹R¹⁰-Einheit durch C=O oder C=NR¹³ ersetzt sein kann;
R¹¹ Wasserstoff, C₁-C₆-Alkyl, C₁-C₆-Halogenalkyl, C₃-C₆-Alkenyl, C₃-C₆-Alkinyl, C₁-C₆-Alkylcarbonyl, C₁-C₆-Halogenalkylcarbonyl, -CO₂R¹², -CONR¹²R¹³ oder SO₂R¹² bedeutet;
R¹² Wasserstoff, C₁-C₆-Alkyl, C₁-C₆-Halogenalkyl, C₃-C₆-Alkenyl, C₃-C₆-Alkinyl oder Phenyl bedeutet, wobei der letztgenannte Rest partiell oder vollständig halogeniert sein kann und ein bis drei der folgenden Reste tragen kann:
Nitro, Cyano, Hydroxy, C₁-C₄-Alkyl, C₁-C₄-Halogenalkyl, C₁-C₄-Alkoxy, C₁-C₄-Halogenalkoxy;
R¹³ C₁-C₆-Alkoxy, C₁-C₆-Halogenalkoxy, C₃-C₆-Alkenyloxy, C₃-C₆-Alkinyloxy oder ein unter R¹² genannter Rest bedeutet;
- Q: ein in 2-Stellung verknüpfter Cyclohexan-1,3-dionring der Formel II 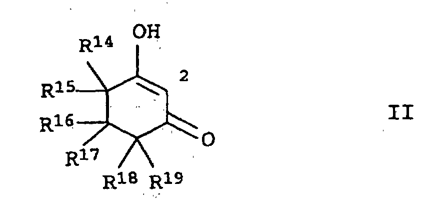
wobei
- R¹⁴, R¹⁵, R¹⁷ und R¹⁹: für Wasserstoff oder C₁-C₄-Alkyl stehen;
- R¹⁶: für Wasserstoff, C₁-C₄-Alkyl oder C₃-C₄-Cycloalkyl steht, wobei die beiden letztgenannten Gruppen gegebenenfalls einen bis drei der folgenden Substituenten tragen können:
Halogen, C₁-C₄-Alkylthio oder C₁-C₄-Alkoxy;
oder
für Tetrahydropyran-3-yl, Tetrahydropyran-4-yl, Tetrahydrothiopyran-3-yl, 1,3-Dioxolan-2-yl, 1,3-Dioxan-2-yl, 1,3-Oxathiolan-2-yl, 1,3-Oxathian-2-yl, 1,3-Dithiolan2-yl oder 1,3-Dithian-2-yl steht, wobei die 6 letztgenannten Reste gegebenenfalls durch ein bis drei C₁-C₄-Alkylreste substituiert sein können;
- R¹⁸: für Wasserstoff, C₁-C₄-Alkyl oder C₁-C₆-Alkoxycarbonyl steht;
oder
- R¹⁶ und R¹⁹: gemeinsam eine Bindung oder einen drei- bis sechsgliedrigen carbocyclischen Ring bilden;
oder
die -CR¹⁶R¹⁷-Einheit durch C=O ersetzt sein kann
sowie deren landwirtschaftlich brauchbaren Salze.

Außerdem betrifft die Erfindung Verfahren zur Herstellung von Verbindungen der Formel I, Mittel welche diese enthalten sowie die Verwendung dieser Derivate oder diese enthaltende Mittel zur Schadpflanzenbekämpfung.

Aus der Literatur, beispielsweise aus EP-A 283 261, sind 2-Hetaroylcyclohexandione bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die Hetaroylderivate der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in. Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht. Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat; Propionat und Butyrat.

Der in 2-Stellung verknüpfte Cyclohexan-1,3-dionring der Formel II, 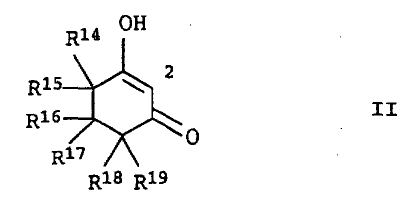 steht auch stellvertretend für die tautomeren Formen II' und II''.

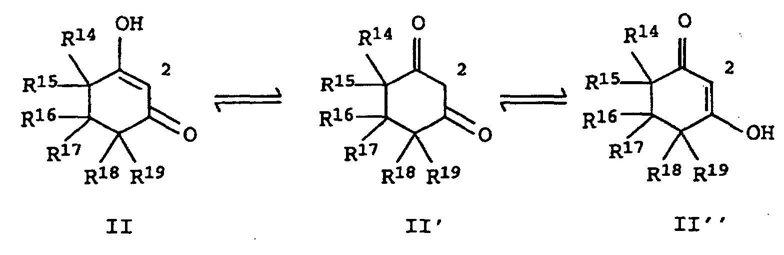

Die für die Substituenten R¹-R¹⁹ oder als Reste an Phenylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, Alkoxysulfonyl-, Halogenalkoxysulfonyl-, Alkylcarbonyl-, Halogenalkylcarbonyl, Alkoxycarbonyl-, Alkenyl-, Alkenyloxy-, Alkenylthio-, Alkenylsulfinyl-, Alkenylsulfonyl-, Alkenyloxysulfonyl-, Alkinyl-, Alkinyloxy-, Alkinylthio-, Alkinylsulfinyl-, Alkinylsulfonyl- und Alkinyloxysulfonyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl, sowie die Alkylteile von C₁-C₄-Alkylcarbonyl: Methyl, Ethyl, Propyl, 1-Methylethyl, Butyl, 1-Methylpropyl, 2-Methylpropyl und 1,1-Dimethylethyl;
- C₁-C₆-Alkyl, sowie die Alkylteile von C₁-C₆-Alkylcarbonyl: C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl, sowie die Halogenalkylteile von C₁-C₄-Halogenalkylcarbonyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl, sowie die Halogenalkylteile von C₁-C₆-Halogenalkylcarbonyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Alkoxy, sowie die Alkoxyteile in C₁-C₄-Alkoxycarbonyl; Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy, sowie die Alkoxyteile in C₁-C₆-Alkoxycarbonyl; C₁-C₄-Alkoxy wie voranstehend genannt, sowie Pentoxy. 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Hrompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₆-Halogenalkoxy: C₁-C₄-Halogenalkoxy wie voranstehend genannt, sowie 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy und Dodecafluorhexoxy;
- C₁-C₄-Alkylthio: Methylthio, Ethylthio, Propylthio, 1-Methylethylthio, Butylthio, 1-Methylpropylthio, 2-Methylpropylthio und 1,1-Dimethylethylthio;
- C₁-C₆-Alkylthio: C₁-C₄-Alkylthio wie voranstehend genannt, sowie Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
- C₁-C₄-Halogenalkylthio: einen C₁-C₄-Alkylthiorest wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio. 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio und Nonafluorbutylthio;
- C₁-C₆-Halogenalkylthio: C₁-C₄-Halogenalkylthio wie voranstehend genannt, sowie 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio und Dodecafluorhexylthio;
- C₁-C₄-Alkylsulfinyl (C₁-C₄-Alkyl-S(=O)-): Methylsulfinyl, Ethylsulfinyl, Propylsulfinyl, 1-Methylethylsulfinyl, Butylsulfinyl, 1-Methylpropylsulfinyl, 2-Methylpropylsulfinyl und 1,1-Dimethylethylsulfinyl;
- C₁-C₆-Alkylsulfinyl: C₁-C₄-Alkylsulfinyl wie voranstehend genannt, sowie Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulf inyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
- C₁-C₄-Halogenalkylsulfinyl: C₁-C₄-Alkylsulfinylrest wie voranstehend genannt, der partiell oder vollständig durch Fluor, Chlor, Brom und/oder Iod substituiert ist, also Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl und Nonafluorbutylsulfinyl;
- C₁-C₆-Halogenalkylsulfinyl: C₁-C₄-Halogenalkylsulfinyl wie voranstehend genannt, sowie 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl und Dodecafluorhexylsulfinyl;
- C₁-C₄-Alkylsulfonyl (C₁-C₄-Alkyl-S(=O)₂-): Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₆-Alkylsulfonyl: C₁-C₄-Alkylsulfonyl wie voranstehend genannt, sowie Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, 1,1-Dimethylpropylsulfonyl, 1,2-Dimethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₄-Halogenalkylsulfonyl: einen C₁-C₄-Alkylsulfonylrest wie voranstehend genannt, der partiell oder vollständig durch Fluor, Chlor, Brom und/oder Iod substituiert ist, also Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2,2,2-Trichlorethylsulfonyl, 2-Chlor-2-fluorethylsulfonyl, 2-Chlor-2,2-difluorethylsulfonyl, 2,2-Dichlor-2-fluorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl,3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl und Nonafluorbutylsulfonyl;
- C₁-C₆-Halogenalkylsulfonyl: C₁-C₄-Halogenalkylsulfonyl wie voranstehend genannt, sowie 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;
- C₁-C₄-Alkoxysulfonyl: Methoxysulfonyl, Ethoxysulfonyl, Propoxysulfonyl, 1-Methylethoxysulfonyl, Butoxysulfonyl, 1-Methylpropoxysulfonyl, 2-Methylpropoxysulfonyl und 1,1-Dimethylethoxysulfonyl;
- C₁-C₆-Alkoxysulfonyl; C₁-C₄-Alkoxysulfonyl wie voranstehend genannt, sowie Pentoxysulfonyl, 1-Methylbutoxysulfonyl, 2-Methylbutoxysulfonyl, 3-Methylbutoxysulfonyl, 1,1-Dimethylpropoxysulfonyl, 1,2-Dimethylpropoxysulfonyl, 2,2-Dimethylpropoxysulfonyl, 1-Ethylpropoxysulfonyl, Hexoxysulfonyl, 1-Methylpentoxysulfonyl, 2-Methylpentoxysulfonyl, 3-Methylpentoxysulfonyl, 4-Methylpentoxysulfonyl, 1,1-Dimethylbutoxysulfonyl, 1,2-Dimethylbutoxysulfonyl, 1,3-Dimethylbutoxysulfonyl, 2,2-Dimethylbutoxysulfonyl, 2,3-Dimethylbutoxysulfonyl, 3,3-Dimethylbutoxysulfonyl, 1-Ethylbutoxysulfonyl, 2-Ethylbutoxysulfonyl, 1,1,2-Trimethylpropoxysulfonyl, 1,2,2-Trimethylpropoxysulfonyl, 1-Ethyl-1-methylpropoxysulfonyl und 1-Ethyl-2-methylpropoxysulfonyl;
- C₁-C₄-Halogenalkoxysulfonyl: einen C₁-C₄-Alkoxysulfonylrest wie voranstehend genannt, der partiell oder vollständig durch Fluor, Chlor, Brom und/oder Iod substituiert ist, also z.B. Fluormethoxysulfonyl, Difluormethoxysulfonyl, Trifluormethoxysulfonyl, Chlordifluormethoxysulfonyl, Bromdifluormethoxysulfonyl, 2-Fluorethoxysulfonyl, 2-Chlorethoxysulfonyl, 2-Bromethoxysulfonyl, 2-Iodethoxysulfonyl, 2,2-Difiuorethoxysulfonyl, 2,2,2-Trifluorethoxysulfonyl, 2-Chlor-2-fluorethoxysulfonyl, 2-Chlor-2,2-difluorethoxysulfonyl, 2,2-Dichlor-2-fluorethoxysulfonyl, 2,2,2-Trichlorethoxysulfonyl, Pentafluorethoxysulfonyl, 2-Fluorpropoxysulfonyl, 3-Fluorpropoxysulfonyl, 2-Chlorpropoxysulfonyl, 3-Chlorpropoxysulfonyl, 2-Brompropoxysulfonyl, 3-Brompropoxysulfonyl, 2,2-Difluorpropoxysulfonyl, 2,3-Difluorpropoxysulfonyl, 2,3-Dichlorpropoxysulfonyl, 3,3,3-Trifluorpropoxysulfonyl, 3,3,3-Trichlorpropoxysulfonyl, 2,2,3,3,3-Pentafluorpropoxysulfonyl, Heptafluorpropoxysulfonyl, 1-(Fluormethyl)-2-fluorethoxysulfonyl, 1-(Chlormethyl)-2-chlorethoxysulfonyl, 1-(Brommethyl)-2-bromethoxysulfonyl, 4-Fluorbutoxysulfonyl, 4-Chlorbutoxysulfonyl, 4-Brombutoxysulfonyl und 4-Iodbutoxysulfonyl;
- C₁-C₆-Halogenalkoxysulfonyl: C₁-C₄-Halogenalkoxysulfonyl wie voranstehend genannt, sowie 5-Fluorpentoxysulfonyl, 5-Chlorpentoxysulfonyl, 5-Brompentoxysulfonyl, 5-Iodpentoxysulfonyl, Undecafluorpentoxysulfonyl, 6-Fluorhexoxysulfonyl, 6-Chlorhexoxysulfonyl, 6-Bromhexoxysulfonyl, 6-Iodhexoxysulfonyl und Dodecafluorhexoxysulfonyl;
- C₂-C₄-Alkenyl, sowie die Alkenylteile von C₂-C₄-Alkenyloxy, C₂-C₄-Alkenylthio, C₂-C₄-Alkenylsulfinyl, C₂-C₄-Alkenylsulfonyl, C₂-C₄-Alkenyloxysulfonyl: Ethenyl, Prop-1-en-1-yl, Prop-2-en-1-yl, 1-Methylethenyl, Buten-1-yl, Buten-2-yl, Buten-3-yl 1-Methyl-prop-1-en-1-yl, 2-Methyl-prop-1-en-1-yl, 1-Methyl-prop-2-en-1-yl und 2-Methyl-prop-2-en-1-yl;
- C₂-C₆-Alkenyl, sowie die Alkenylteile von C₂-C₆-Alkenyloxy. C₂-C₆-Alkenylthio, C₂-C₆-Alkenylsulfinyl, C₂-C₆-Alkenylsulfonyl, C₂-C₆-Alkenyloxysulfonyl: C₂-C₄-Alkenyl wie voranstehend genannt, sowie Penten-1-yl, Penten-2-yl, Penten-3-yl, Penten-4-yl, 1-Methyl-but-1-en-1-yl, 2-Methylbut-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methylbut-3-en-1-yl, 1,1-Dimethyl-prop-2-en-1-yl, 1,2-Dimethylprop-1-en-1-yl, 1,2-Dimethyl-prop-2-en-1-yl, 1-Ethylprop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methylpent-1-en-1-yl, 4-Methyl-pent-1-en-1-yl, 1-Methyl-pent-2-en-1-yl 2-Methyl-pent-2-en-1-yl, 3-Methyl-pent-2-en-1-yl, 4-Methyl-pent-2-en-1-yl, 1-Methyl-pent-3-en-1-yl, 2-Methylpent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl;
- C₂-C₄-Alkinyl sowie die Alkinylreste von C₂-C₄-Alkinyloxy, C₂-C₄-Alkinylthio, C₂-C₄-Alkinylsulfinyl, C₂-C₄-Alkinylsulfonyl, C₂-C₄-Alkinyloxysulfonyl: Ethinyl, Prop-1-in-1-yl, Prop-2-in-1-yl, But-1-in-1-yl, But-1-in-3-yl, But-1-in-4-yl und But-2-in-1-yl;
- C₂-C₆-Alkinyl, sowie die Alkinylreste von C₂-C₆-Alkinyloxy, C₂-C₆-Alkinylthio, C₂-C₆-Alkinylsulfinyl, C₂-C₆-Alkinylsulfonyl, C₂-C₆-Alkinyloxysulfonyl: C₂-C₄-Alkinyl wie voranstehend genannt, sowie Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methylbut-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methylpent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl;
- C₃-C₄-Cycloalkyl: Cyclopropyl und Cyclobutyl;

Alle Phenylringe sind vorzugsweise unsubstituiert oder tragen ein bis drei Halogenatome und/oder eine Nitrogruppe, einen Cyanorest, einen Methyl-, Trifluormethyl-, Methoxy- oder Trifluormethoxysubstituenten.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, Cyano, Rhodano, Hydroxy, Mercapto, C₁-C₆-Alkyl, C₁-C₆-Halogenalkyl, C₂-C₆-Alkenyl, C₂-C₆-Alkinyl, C₁-C₆-Alkoxy, C₁-C₆-Halogenalkoxy, C₂-C₆-Alkenyloxy, C₂-C₆-Alkinyloxy, C₁-C₆-Alkylthio, C₁-C₆-Halogenalkylthio, C₂-C₆-Alkenylthio, C₂-C₆-Alkinylthio, C₁-C₆-Alkylsulfinyl, C₁-C₆-Halogenalkylsulfinyl, C₂-C₆-Alkenylsulfinyl, C₂-C₆-Alkinylsulfinyl, C₁-C₆-Alkylsulfonyl, C₁-C₆-Halogenalkylsulfonyl, C₂-C₆-Alkenylsulfonyl, C₂-C₆-Alkinylsulfonyl, C₁-C₆-Alkoxysulfonyl, C₁-C₆-Halogenalkoxysulfonyl, C₂-C₆-Alkenyloxysulfonyl, C₂-C₆-Alkinyloxysulfonyl, Phenyl, Phenyloxy, Phenylthio, Phenylsulfinyl oder Phenylsulfonyl, wobei die fünf letztgenannten Substituenten partiell oder vollständig halogeniert sein können und ein bis drei der folgenden Gruppen tragen können:
Nitro, Cyano, Hydroxy, C₁-C₄-Alkyl, C₁-C₄-Halogenalkyl, C₁-C₄-Alkoxy, C₁-C₄-Halogenalkoxy;
besonders bevorzugt Nitro, Halogen, Hydroxy, C₁-C₆-Alkyl, C₁-C₆-Halogenalkyl, C₁-C₆-Alkoxy, C₁-C₆-Halogenalkoxy, C₁-C₆-Alkylsulfonyl, C₁-C₆-Halogenalkylsulfonyl oder Phenyl, wobei letzgenannter Rest unsubstituiert ist oder ein bis drei Halogenatome und/oder eine Nitrogruppe, einen Cyanorest, einen Methyl-, Trifluormethyl-, Methoxy- oder Trifluormethoxysubstituenten tragen kann;
insbesonders bevorzugt Nitro, Fluor, Chlor, Brom, Hydroxy, Methyl, Ethyl, Trifluormethyl, Methoxy, Ethoxy, Difluormethoxy, Trifluormethoxy, Methylsulfonyl, Ethylsulfonyl, Trifluormethylsulfonyl, Pentafluorethylsulfonyl oder Phenyl;
- R²: Wasserstoff, Halogen oder C₁-C₆-Alkyl;
besonders bevorzugt Wasserstoff, Chlor, Brom oder Methyl;
- Z: Z¹, Z², Z³, Z⁴, Z⁵, Z⁶, Z⁷, Z⁸, Z⁹, Z¹⁰, Z¹¹ oder Z¹²;
- R³, R⁵, R⁷, R⁹: Wasserstoff, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Nitro, Cyano, Hydroxy, Mercapto, C₂-C₄-Alkenyl, C₂-C₄-Alkinyl, C₂-C₄-Alkenyloxy, C₂-C₄-Alkinyloxy, C₂-C₄-Alkenylthio, C₂-C₄-Alkinylthio, C₁-C₄-Alkylsulfinyl, C₁-C₄-Halogenalkylsulfinyl, C₂-C₄-Alkenylsulfinyl, C₂-C₄-Alkinylsulfinyl, C₁-C₄-Alkylsulfonyl, C₁-C₄-Halogenalkylsulfonyl, C₂-C₄-Alkenylsulfonyl, C₂-C₄-Alkinylsulfonyl, C₁-C₄-Alkoxysulfonyl, C₁-C₄-Halogenalkoxysulfonyl, C₂-C₄-Alkenyloxysulfonyl, C₂-C₄-Alkinyloxysulfinyl, -NR¹²R¹³, -CO₂R¹², -CONR¹²R¹³, Phenyl, Phenoxy, Phenylthio, Phenylsulfinyl oder Phenylsulfonyl bedeuten, wobei die fünf letztgenannten Substituenten partielle oder vollständig halogeniert sein können und ein bis drei der folgenden Gruppen tragen können:
Nitro, Cyano, Hydroxy, C₁-C₄-Alkyl, C₁-C₄-Halogenalkyl, C₁-C₄-Alkoxy, C₁-C₄-Halogenalkoxy;
besonders bevorzugt Wasserstoff, Halogen, C₁-C₄-Alkyl, C₁-C₄-Halogenalkyl, C₁-C₄-Alkoxy, C₁-C₄-Halogenalkoxy, Nitro, Cyano, Hydroxy, C₁-C₆-Alkoxycarbonyl oder Phenyl, wobei letztgenannter Rest unsubstituiert ist oder ein bis drei Halogenatome und/oder eine Nitrogruppe, einen Cyanorest, einen Methyl-, Trifluormethyl-, Methoxy- oder Trifluormethoxysubstituenten tragen kann;
insbesonders bevorzugt Wasserstoff, Fluor, Chlor, Brom, Methyl, Ethyl, Trifluormethyl, Methoxy, Ethoxy, Trifluormethoxy, Difluormethoxy, Nitro, Cyano, Hydroxy, Methoxycarbonyl, Ethoxycarbonyl oder Phenyl;
- R⁴, R⁶, R⁸, R¹⁰: Wasserstoff, Halogen oder C₁-C₄-Alkyl;
besonderes bevorzugt Wasserstoff, Fluor, Chlor, Methyl oder Ethyl;
inbesondere bevorzugt Wasserstoff;
- R¹¹: Wasserstoff, C₁-C₆-Alkyl, C₁-C₆-Halogenalkyl, C₁-C₆-Alkylcarbonyl, C₁-C₆-Halogenalkylcarbonyl, C₁-C₆-Alkylsulfonyl, C₁-C₆-Halogenalkylsulfonyl oder Phenylsulfonyl, wobei der letztgenannte Phenylrest gegebenenfalls durch einen C₁-C₄-Alkylrest substituiert sein kann;
besonders bevorzugt Methyl, Ethyl, Difluormethyl, Trifluormethyl, Methylcarbonyl, Ethylcarbonyl, Isopropylcarbonyl, Trifluormethylcarbonyl, Methylsulfonyl, Trifluormethylsulfonyl, Phenylsulfonyl oder 4-Methylphenylsulfonyl;
- R¹²: Wasserstoff oder C₁-C₆-Alkyl;
besonders bevorzugt Wasserstoff, Methyl oder Ethyl;
- R¹³: Wasserstoff, C₁-C₆-Alkyl, C₁-C₆-Alkoxy, C₃-C₆-Alkenyloxy oder C₃-C₆-Alkinyloxy;
besonders bevorzugt Methyl, Ethyl, Methoxy, Ethoxy, 2-Propen-1-yloxy, 2-Propin-1-yloxy oder 1-Methyl-2-propin-1-yloxy;
- R¹⁴,R¹⁵, R¹⁷, R¹⁹: Wasserstoff oder C₁-C₄-Alkyl;
besonders bevorzugt Wasserstoff, Methyl oder Ethyl;
- R¹⁶: Wasserstoff, C₁-C₄-Alkyl, C₃-C₄-Cycloalkyl, wobei die beiden letztgenannten Gruppen gegebenenfalls ein bis drei der folgenden Substituenten tragen können: Halogen, C₁-C₄-Alkoxy oder C₁-C₄-Alkylthio;
Tetrahydropyran-3-yl, Tetrahydropyran-4-yl, Tetrahydrothiopyran-3-yl, 1,3-Dioxolan-2-yl, 1,3-Dioxan-2-yl, 1,3-Oxathiolan-2-yl, 1,3-Oxathian-2-yl, 1,3-Dithian-2-yl oder 1,3-Dithiolan-2-yl, wobei die sechs letztgenannten Gruppen gegebenenfalls bis zu drei C₁-C₄-Alkylreste tragen können; besonders bevorzugt Wasserstoff, Methyl, Ethyl, Cyclopropyl, Di(methoxy)methyl, Di(ethoxy)methyl, 2-Ethylthiopropyl, Tetrahydropyran-3-yl, Tetrahydropyran-4-yl, 1,3-Dioxolan-2-yl, 1,3-Dioxan-2-yl, 5,5-Dimethyl-1-3-dioxan-2-yl, 1,3-Oxathiolan-2-yl, 1,3-Oxathian-2-yl, 1,3-Dithiolan-2-yl, 5,5-Dimethyl-1,3-dithian-2-yl oder 1-Methylthiocyclopropyl;
- R¹⁸: Wasserstoff, C₁-C₄-Alkyl oder C₁-C₄-Alkoxycarbonyl;
besonders bevorzugt Wasserstoff, Methyl oder Methoxycarbonyl.

Ebenso kann in Betracht kommen, daß R¹⁶ und R¹⁹ eine π-Bindung ausbilden, so daß ein Doppelbindungssystem entsteht.

Gewünschtenfalls kann die -CR¹⁶R¹⁷-Einheit durch C=O ersetzt werden.

Bevorzugt sind Verbindungen der Formel I, wobei die Variable Z die Bedeutung Z¹, Z², Z¹¹ oder Z¹² hat.

Ebenso bevorzugt sind die Verbindungen der Formel I, wobei die Variable Z die Bedeutung Z³, Z⁴, Z⁵, Z⁶, Z⁷ oder Z⁸ hat.

Ebenso bevorzugt sind die Verbindungen der Formel I, wobei die Variable Z die Bedeutung Z⁹ oder Z¹⁰ hat.

Insbesondere bevorzugt sind Verbindungen der Formel Ia-Ic (Z=Z¹) und Id-Ie (Z=Z²) sowie deren N-Oxide Ia'-Ic' (Z=Z¹¹) und Id'-Ie' (Z=Z¹²), ebenso insbesondere bevorzugt sind Verbindungen der Formeln If (Z=Z⁹) und Ig (Z=Z¹⁰).

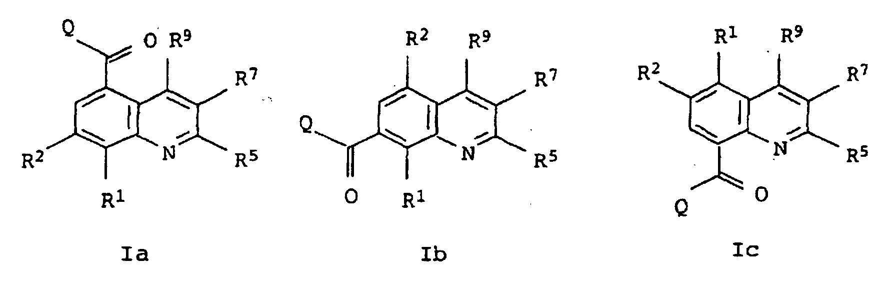

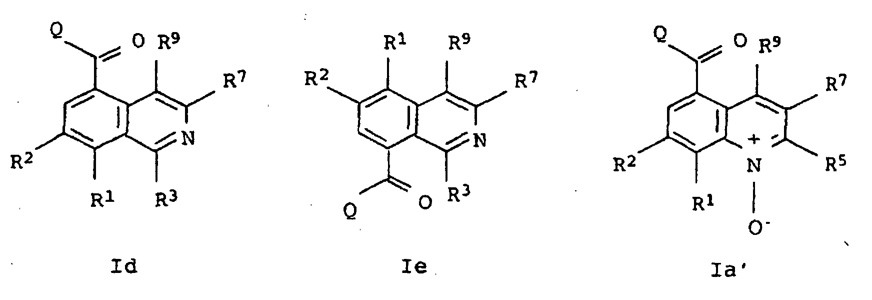

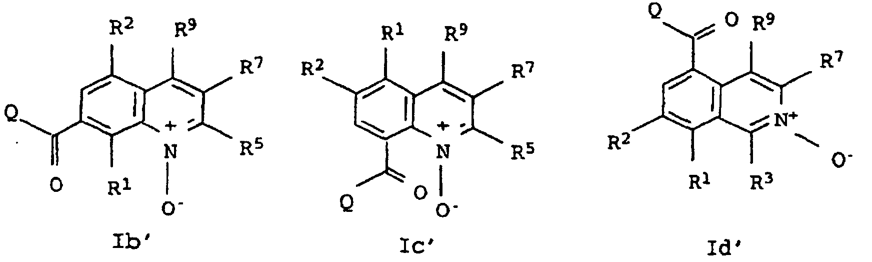

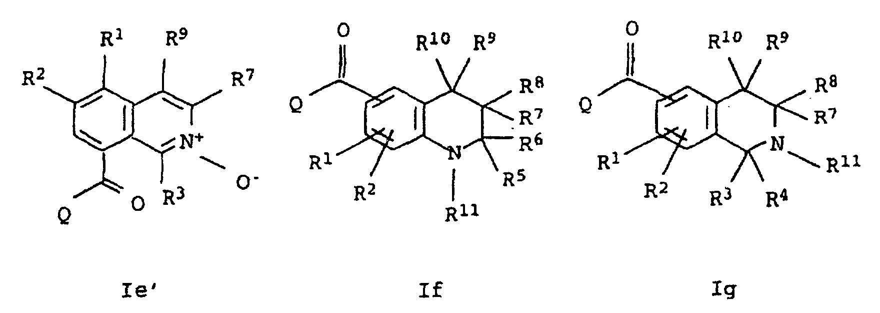

Weiterhin sind die Verbindungen Ia, Ib, Ic, Id und Ie bevorzugt.

Ebenso sind die Verbindungen If und Ig, wobei CR³R⁴, CR⁵R⁶, CR⁷R⁸ und/oder CR⁹R¹⁰ nicht durch C=O oder C=NR¹³ ersetzt werden können, bevorzugt.

Weiterhin sind die Verbindungen If, wobei CR⁵R⁶ durch C=O oder C=NR¹³ ersetzt ist, bevorzugt.

Weiterhin sind die Verbindungen Ig, wobei CR³R⁴, CR⁷R⁸ und/oder CR⁸R¹⁰ durch C=O oder C=NR¹³ ersetzt ist, bevorzugt.

Außerordentlich bevorzugt sind die in Tabelle 1 aufgeführten Verbindungen Ia1 (=̂ I mit R², R¹⁴, R¹⁵, R¹⁶, R¹⁷, R¹⁸, R¹⁹ = H, wobei das "Q-CO-Fragment" in Position a, R¹ in Position d und Z¹ über die Positionen b und c gebunden sind).

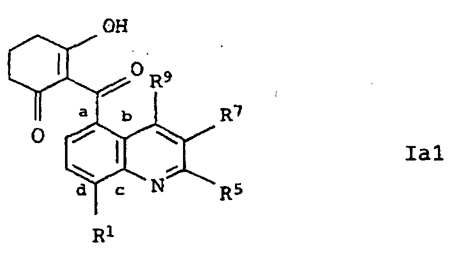

**Tabelle 1**

| Nr. | R¹ | R⁵ | R⁷ | R⁹ |
|---|---|---|---|---|
| Ia1.001 | Br | H | H | H |
| Ia1.002 | Cl | H | H | H |
| Ia1.003 | SO₂CH₃ | H | H | H |
| Ia1.004 | CH₃ | H | H | H |
| Ia1.005 | OH | H | H | H |
| Ia1.006 | OCH₃ | H | H | H |
| Ia1.007 | CF₃ | H | H | H |
| Ia1.008 | NO₂ | H | H | H |
| Ia1.009 | F | H | H | H |
| Ia1.010 | OCF₃ | H | H | H |
| Ia1.011 | C₆H₅ | H | H | H |
| Ia1.012 | Br | H | CH₃ | H |
| Ia1.013 | Cl | H | CH₃ | H |
| Ia1.014 | SO₂CH₃ | H | CH₃ | H |
| Ia1.015 | CH₃ | H | CH₃ | H |
| Ia1.016 | OH | H | CH₃ | H |
| Ia1.017 | OCH₃ | H | CH₃ | H |
| Ia1.018 | CF₃ | H | CH₃ | H |
| Ia1.019 | NO₂ | H | CH₃ | H |
| Ia1.020 | F | H | CH₃ | H |
| Ia1.021 | OCF₃ | H | CH₃ | H |
| Ia1.022 | C₆H₅ | H | CH₃ | H |
| Ia1.023 | Br | CH₃ | H | H |
| Ia1.024 | Cl | CH₃ | H | H |
| Ia1.025 | SO₂CH₃ | CH₃ | H | H |
| Ia1.026 | CH₃ | CH₃ | H | H |
| Ia1.027 | OH | CH₃ | H | H |
| Ia1.028 | OCH₃ | CH₃ | H | H |
| Ia1.029 | CF₃ | CH₃ | H | H |
| Ia1.030 | NO₂ | CH₃ | H | H |
| Ia1.031 | F | CH₃ | H | H |
| Ia1.032 | OCF₃ | CH₃ | H | H |
| Ia1.033 | C₆H₅ | CH₃ | H | H |
| Ia1.034 | Br | H | H | CH₃ |
| Ia1.035 | Cl | H | H | CH₃ |
| Ia1.036 | SO₂CH₃ | H | H | CH₃ |
| Ia1.037 | CH₃ | H | H | CH₃ |
| Ia1.038 | OH | H | H | CH₃ |
| Ia1.039 | OCH₃ | H | H | CH₃ |
| Ia1.040 | CF₃ | H | H | CH₃ |
| Ia1.041 | NO₂ | H | H | CH₃ |
| Ia1.042 | F | H | H | CH₃ |
| Ia1.043 | OCF₃ | H | H | CH₃ |
| Ia1.044 | C₆H₅ | H | H | CH₃ |
| Ia1.045 | Br | CH₃ | CH₃ | CH₃ |
| Ia1.046 | Cl | CH₃ | CH₃ | CH₃ |
| Ia1.047 | SO₂CH₃ | CH₃ | CH₃ | CH₃ |
| Ia1.048 | CH₃ | CH₃ | CH₃ | CH₃ |
| Ia1.049 | OH | CH₃ | CH₃ | CH₃ |
| Ia1.050 | OCH₃ | CH₃ | CH₃ | CH₃ |
| Ia1.051 | CF₃ | CH₃ | CH₃ | CH₃ |
| Ia1.052 | NO₂ | CH₃ | CH₃ | CH₃ |
| Ia1.053 | F | CH₃ | CH₃ | CH₃ |
| Ia1.054 | OCF₃ | CH₃ | CH₃ | CH₃ |
| Ia1.055 | C₆H₅ | CH₃ | CH₃ | CH₃ |
| Ia1.056 | Br | H | Cl | H |
| Ia1.057 | Cl | H | Cl | H |
| Ia1.058 | SO₂CH₃ | H | Cl | H |
| Ia1.059 | CH₃ | H | Cl | H |
| Ia1.060 | OH | H | Cl | H |
| Ia1.061 | OCH₃ | H | Cl | H |
| Ia1.062 | CF₃ | H | Cl | H |
| Ia1.063 | NO₂ | H | Cl | H |
| Ia1.064 | F | H | Cl | H |
| Ia1.065 | OCF₃ | H | Cl | H |
| Ia1.066 | C₆H₅ | H | Cl | H |
| Ia1.067 | Br | Cl | H | H |
| Ia1.068 | Cl | Cl | H | H |
| Ia1.069 | SO₂CH₃ | Cl | H | H |
| Ia1.070 | CH₃ | Cl | H | H |
| Ia1.071 | OH | Cl | H | H |
| Ia1.072 | OCH₃ | Cl | H | H |
| Ia1.073 | CF₃ | Cl | H | H |
| Ia1.074 | NO₂ | Cl | H | H |
| Ia1.075 | F | Cl | H | H |
| Ia1.076 | OCF₃ | Cl | H | H |
| Ia1.077 | C₆H₅ | Cl | H | H |
| Ia1.078 | Br | H | H | Cl |
| Ia1.079 | Cl | H | H | Cl |
| Ia1.080 | SO₂CH₃ | H | H | Cl |
| Ia1.081 | CH₃ | H | H | Cl |
| Ia1.082 | OH | H | H | Cl |
| Ia1.083 | OCH₃ | H | H | Cl |
| Ia1.084 | CF₃ | H | H | Cl |
| Ia1.085 | NO₂ | H | H | Cl |
| Ia1.086 | F | H | H | Cl |
| Ia1.087 | OCF₃ | H | H | Cl |
| Ia1.088 | C₆H₅ | H | H | Cl |
| Ia1.089 | Br | Cl | Cl | Cl |
| Ia1.090 | Cl | Cl | Cl | Cl |
| Ia1.091 | SO₂CH₃ | Cl | Cl | Cl |
| Ia1.092 | CH₃ | Cl | Cl | Cl |
| Ia1.093 | OH | Cl | Cl | Cl |
| Ia1.094 | OCH₃ | Cl | Cl | Cl |
| Ia1.095 | CF₃ | Cl | Cl | Cl |
| Ia1.096 | NO₂ | Cl | Cl | Cl |
| Ia1.097 | F | Cl | Cl | Cl |
| Ia1.098 | OCF₃ | Cl | Cl | Cl |
| Ia1.099 | C₆H₅ | Cl | Cl | Cl |
| Ia1.100 | Br | C₆H₅ | H | H |
| Ia1.101 | Cl | C₆H₅ | H | H |
| Ia1.102 | SO₂CH₃ | C₆H₅ | H | H |
| Ia1.103 | CH₃ | C₆H₅ | H | H |
| Ia1.104 | OH | C₆H₅ | H | H |
| Ia1.105 | OCH₃ | C₆H₅ | H | H |
| Ia1.106 | CF₃ | C₆H₅ | H | H |
| Ia1.107 | NO₂ | C₆H₅ | H | H |
| Ia1.108 | F | C₆H₅ | H | H |
| Ia1.109 | OCF₃ | C₆H₅ | H | H |
| Ia1.110 | C₆H₅ | C₆H₅ | H | H |
| Ia1.111 | Br | CH₃ | OH | H |
| Ia1.112 | Cl | CH₃ | OH | H |
| Ia1.113 | SO₂CH₃ | CH₃ | OH | H |
| Ia1.114 | CH₃ | CH₃ | OH | H |
| Ia1.115 | OH | CH₃ | OH | H |
| Ia1.116 | OCH₃ | CH₃ | OH | H |
| Ia1.117 | CF₃ | CH₃ | OH | H |
| Ia1.118 | NO₂ | CH₃ | OH | H |
| Ia1.119 | F | CH₃ | OH | H |
| Ia1.120 | OCF₃ | CH₃ | OH | H |
| Ia1.121 | C₆H₅ | CH₃ | OH | H |
| Ia1.122 | Br | CF₃ | H | H |
| Ia1.123 | Cl | CF₃ | H | H |
| Ia1.124 | SO₂CH₃ | CF₃ | H | H |
| Ia1.125 | CH₃ | CF₃ | H | H |
| Ia1.126 | OH | CF₃ | H | H |
| Ia1.127 | OCH₃ | CF₃ | H | H |
| Ia1.128 | CF₃ | CF₃ | H | H |
| Ia1.129 | NO₂ | CF₃ | H | H |
| Ia1.130 | F | CF₃ | H | H |
| Ia1.131 | OCF₃ | CF₃ | H | H |
| Ia1.132 | C₆H₅ | CF₃ | H | H |
| Ia1.133 | Br | CH₃ | H | OH |
| Ia1.134 | Cl | CH₃ | H | OH |
| Ia1.135 | SO₂CH₃ | CH₃ | H | OH |
| Ia1.136 | CH₃ | CH₃ | H | OH |
| Ial.137 | OH | CH₃ | H | OH |
| Ia1.138 | OCH₃ | CH₃ | H | OH |
| Ia1.139 | CF₃ | CH₃ | H | OH |
| Ia1.140 | NO₂ | CH₃ | H | OH |
| Ia1.141 | F | CH₃ | H | OH |
| Ia1.142 | OCF₃ | CH₃ | H | OH |
| Ia1.143 | C₆H₅ | CH₃ | H | OH |
| Ia1.144 | Br | H | H | OCH₃ |
| Ia1.145 | Cl | H | H | OCH₃ |
| Ia1.146 | SO₂CH₃ | H | H | OCH₃ |
| Ia1.147 | CH₃ | H | H | OCH₃ |
| Ia1.148 | OH | H | H | OCH₃ |
| Ia1.149 | OCH₃ | H | H | OCH₃ |
| Ia1.150 | CF₃ | H | H | OCH₃ |
| Ia1.151 | NO₂ | H | H | OCH₃ |
| Ia1.152 | F | H | H | OCH₃ |
| Ia1.153 | H | H | H | OCH₃ |
| Ia1.154 | OCF₃ | H | H | OCH₃ |
| Ia1.155 | C₆H₅ | H | H | OCH₃ |
| Ia1.156 | Br | Cl | Cl | CH₃ |
| Ia1.157 | Cl | Cl | Cl | CH₃ |
| Ia1.158 | SO₂CH₃ | Cl | Cl | CH₃ |
| Ia1.159 | CH₃ | Cl | Cl | CH₃ |
| Ia1.160 | OH | Cl | Cl | CH₃ |
| Ia1.161 | OCH₃ | Cl | Cl | CH₃ |
| Ia1.162 | CF₃ | Cl | Cl | CH₃ |
| Ia1.163 | NO₂ | Cl | Cl | CH₃ |
| Ia1.164 | F | Cl | Cl | CH₃ |
| Ia1.165 | OCF₃ | Cl | Cl | CH₃ |
| Ia1.166 | C₆H₅ | Cl | Cl | CH₃ |
| Ia1.167 | Br | CF₃ | H | Br |
| Ia1.168 | Cl | CF₃ | H | Br |
| Ial.169 | SO₂CH₃ | CF₃ | H | Br |
| Ia1.170 | CH₃ | CF₃ | H | Br |
| Ia1.171 | OH | CF₃ | H | Br |
| Ia1.172 | OCH₃ | CF₃ | H | Br |
| Ia1.173 | CF₃ | CF₃ | H | Br |
| Ia1.174 | NO₂ | CF₃ | H | Br |
| Ia1.175 | F | CF₃ | H | Br |
| Ia1.176 | H | CF₃ | H | Br |
| Ia1.177 | OCF₃ | CF₃ | H | Br |
| Ia1.178 | C₆H₅ | CF₃ | H | Br |
| Ia1.179 | Br | OH | CN | H |
| Ia1.180 | Cl | OH | CN | H |
| Ia1.181 | SO₂CH₃ | OH | CN | H |
| Ia1.182 | CH₃ | OH | CN | H |
| Ia1.183 | OH | OH | CN | H |
| Ia1.184 | OCH₃ | OH | CN | H |
| Ia1.185 | CF₃ | OH | CN | H |
| Ia1.186 | NO₂ | OH | CN | H |
| Ia1.187 | F | OH | CN | H |
| Ia1.188 | OCF₃ | OH | CN | H |
| Ia1.189 | C₆H₅ | OH | CN | H |
| Ia1.190 | Br | H | CF₃ | H |
| Ia1.191 | Cl | H | CF₃ | H |
| Ia1.192 | SO₂CH₃ | H | CF₃ | H |
| Ia1.193 | CH₃ | H | CF₃ | H |
| Ia1.194 | OH | H | CF₃ | H |
| Ia1.195 | OCH₃ | H | CF₃ | H |
| Ia1.196 | CF₃ | H | CF₃ | H |
| Ia1.197 | NO₂ | H | CF₃ | H |
| Ia1.198 | F | H | CF₃ | H |
| Ia1.199 | OCF₃ | H | CF₃ | H |
| Ia1.200 | C₆H₅ | H | CF₃ | H |
| Ia1.201 | Br | H | H | NO₂ |
| Ia1.202 | Cl | H | H | NO₂ |
| Ia1.203 | SO₂CH₃ | H | H | NO₂ |
| Ia1.204 | CH₃ | H | H | NO₂ |
| Ia1.205 | OH | H | H | NO₂ |
| Ia1.206 | OCH₃ | H | H | NO₂ |
| Ia1.207 | CF₃ | H | H | NO₂ |
| Ia1.208 | NO₂ | H | H | NO₂ |
| Ia1.209 | F | H | H | NO₂ |
| Ia1.210 | OCF₃ | H | H | NO₂ |
| Ia1.211 | C₆H₅ | H | H | NO₂ |

Desweiteren sind die folgenden Hetaroylderivate der Formel I außerordentlich bevorzugt:
- die Verbindungen Ia2.001-Ia2.211, die sich von den entsprechenden Verbindungen Ia1.001-Ia1.211 dadurch unterscheiden, daß R¹⁶ für Methyl steht: 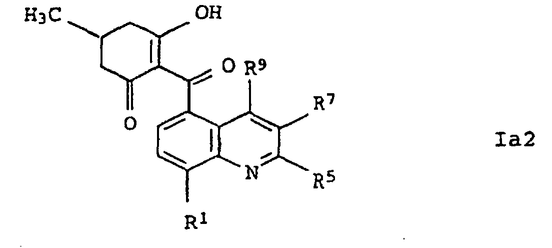
- die Verbindungen Ia3.001-Ia3.211, die sich von den entsprechenden Verbindungen Ia1.001-Ia1.211 dadurch unterscheiden, daß R¹⁶ und R¹⁷ jeweils für Methyl stehen: 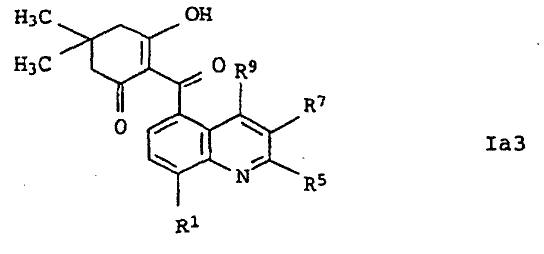
- die Verbindungen Ia4.001-Ia4.211, die sich von den entsprechenden Verbindungen Ia1.001-Ia1.211 dadurch unterscheiden, daß R¹⁸ und R¹⁹ jeweils für Methyl stehen: 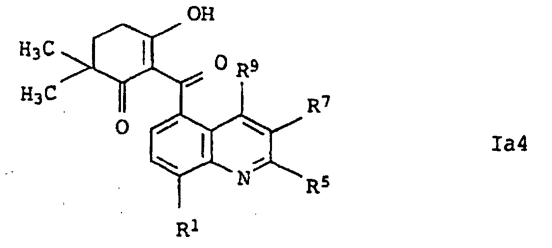
- die Verbindungen Ia5.001-Ia5.211, die sich von den entsprechenden Verbindungen Ia1.001-Ia1.211 dadurch unterscheiden, daß -CR¹⁶R¹⁷-Einheit durch C=O ersetzt ist: 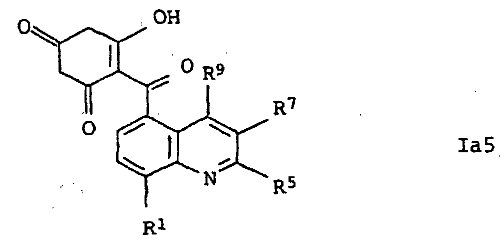
- die Verbindungen Ia6.001-Ia6.211, die sich von den entsprechenden Verbindungen Ia1.001-Ia1.211 dadurch unterscheiden, daß R¹⁴, R¹⁸ und R¹⁹ für Methyl stehen und die -CR¹⁶R¹⁷- Einheit durch C=O ersetzt ist: 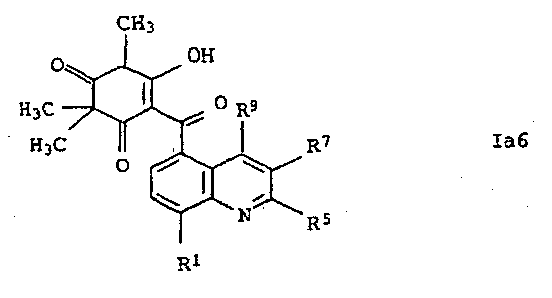
- die Verbindungen Ia7.001-Ia7.211, die sich von den entsprechenden Verbindungen Ia1.001-Ia1.211 dadurch unterscheiden, daß R¹⁴, R¹⁵, R¹⁸ und R¹⁹ für Methyl stehen und die -CR¹⁶R¹⁷-Einheit durch C=O ersetzt ist: 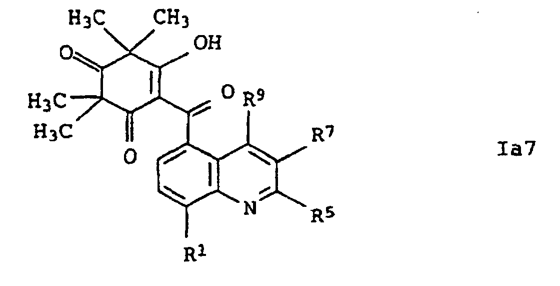
- die Verbindungen Ia'1.001-Ia'.1.211, die sich von den entsprechenden Verbindungen Ia1.001-Ia1.211 dadurch unterscheiden, daß es sich um das N-Oxid handelt (Z = Z¹¹): 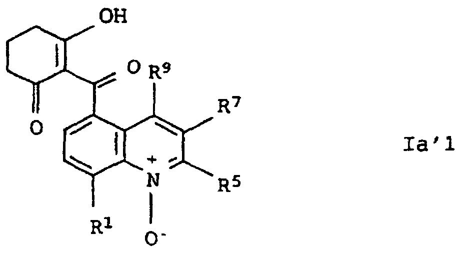
- die Verbindungen Ia'2.001-Ia'2.211, die sich von den entsprechenden Verbindungen Ia1.001-Ia1.211 dadurch unterscheiden, daß R¹⁶ für Methyl steht und es sich um das N-Oxid handelt (Z = Z¹¹): 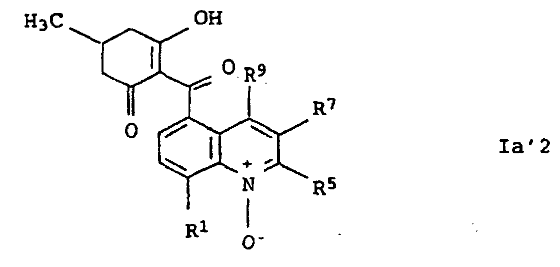
- die Verbindungen Ia'3.001-Ia'3.211, die sich von den entsprechenden Verbindungen Ia1.001-Ia1.211 dadurch unterscheiden, daß R¹⁶ und R¹⁷ jeweils für Methyl stehen und es sich um das N-Oxid handelt (Z = Z¹¹) : 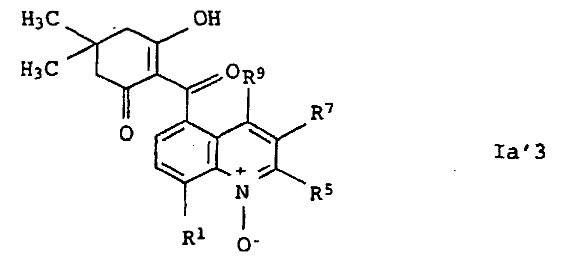
- die Verbindungen Ia'4.001-Ia'4.211, die sich von den entsprechenden Verbindungen Ia1.001-Ia1.211 dadurch unterscheiden, daß R¹⁸ und R¹⁹ jeweils für Methyl stehen und es sich um das N-Oxid handelt (Z = Z¹¹) : 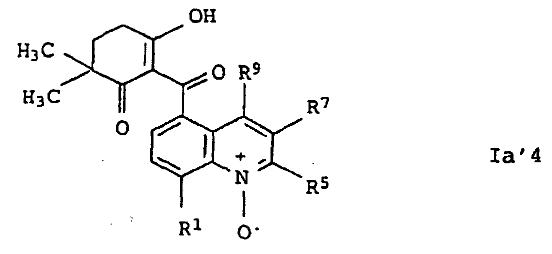
- die Verbindungen Ia'5.001-Ia'5.211, die sich von den entsprechenden Verbindungen Ia1.001-Ia1.211 dadurch unterscheiden, daß die -CR¹⁶R¹⁷-Einheit durch C=O ersetzt ist und daß es sich um das N-Oxid handelt (Z=Z¹¹): 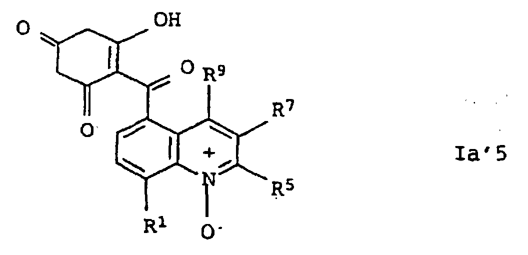
- die Verbindungen Ia'6.001-Ia'6.211, die sich von den entsprechenden Verbindungen Ia1.001-Ia1.211 dadurch unterscheiden, daß R¹⁴, R¹⁸ und R¹⁹ für Methyl stehen, die -CR¹⁶R¹⁷-Einheit durch C=O ersetzt ist und daß es sich um das N-Oxid handelt (Z=Z¹¹): 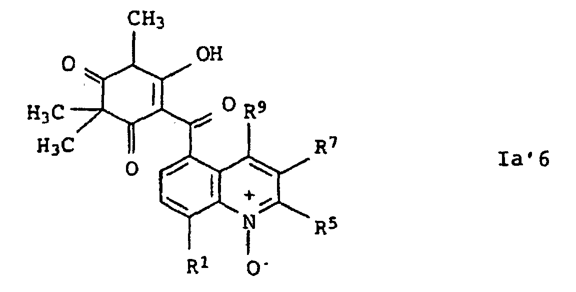
- die Verbindungen Ia'7.001-Ia'7.211, die sich von den entsprechenden Verbindungen Ia1.001-Ia1.211 dadurch unterscheiden, daß R¹⁴, R¹⁵, R¹⁸ und R¹⁹ für Methyl stehen, die -CR¹⁶R¹⁷-Einheit durch C=O ersetzt ist und daß es sich um das N-Oxid handelt (Z=Z¹¹):

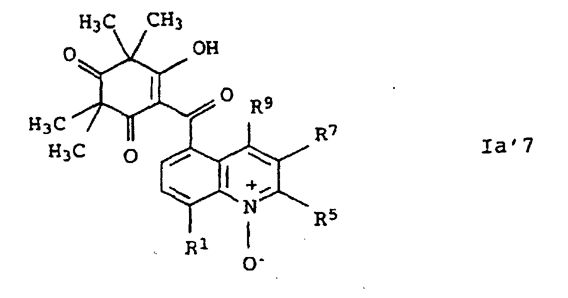

Ebenso sind die in der folgenden Tabelle 2 aufgeführten Verbindungen Ib1 (=̂ I mit R¹⁴, R¹⁵, R¹⁶, R¹⁷, R¹⁸, R¹⁹ = H, wobei das "Q-CO-Pragment" in Position e, R¹ in Position d, R² in Position a und Z¹ über die Positionen b und c gebunden sind) außerordentlich bevorzugt.

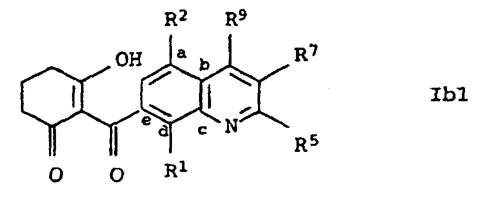

**Tabelle 2**

| Nr. | R¹ | R² | R⁵ | R⁷ | R⁹ |
|---|---|---|---|---|---|
| Ib1.01 | CH₃ | H | H | H | H |
| Ib1.02 | CH₃ | H | H | H | CH₃ |
| Ib1.03 | CH₃ | H | H | CH₃ | H |
| Ib1.04 | CH₃ | H | CH₃ | H | H |
| Ib1.05 | CH₃ | CH₃ | H | H | H |
| Ib1.06 | CH₃ | H | H | H | Cl |
| Ib1.07 | CH₃ | H | H | Cl | H |
| Ib1.08 | CH₃ | H | Cl | H | H |
| Ib1.09 | CH₃ | Cl | H | H | H |
| Ib1.10 | CH₃ | H | H | H | CF₃ |
| Ib1.11 | CH₃ | H | H | CF₃ | H |
| Ib1.12 | CH₃ | H | CF₃ | H | H |
| Ib1.13 | Cl | H | H | H | H |
| Ib1.14 | Cl | H | H | H | CH₃ |
| Ib1.15 | Cl | H | H | CH₃ | H |
| Ib1.16 | Cl | H | CH₃ | H | H |
| Ib1.17 | Cl | H | H | H | Cl |
| Ib1.18 | Cl | H | H | Cl | H |
| Ib1.19 | Cl | H | Cl | H | H |
| Ib1.20 | Cl | H | Cl | Cl | Cl |
| Ib1.21 | CH₃ | H | CH₃ | CH₃ | CH₃ |

Desweiteren sind die folgenden Hetaroylderivate der Formel I außerordentlich bevorzugt:
- die Verbindungen Ib2.01-Ib2.21, die sich von den entsprechenden Verbindungen Ib1.01-Ib1.21 dadurch unterscheiden, daß R¹⁶ für Methyl steht: 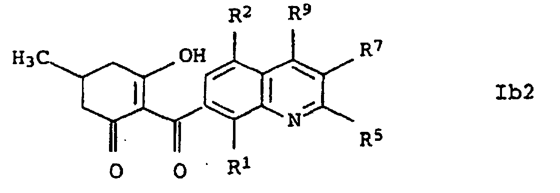
- die Verbindungen Ib3.01-Ib3.21 die sich von den entsprechenden Verbindungen Ib1.01-Ib1.21 dadurch unterscheiden, daß R¹⁶ und R¹⁷ jeweils für Methyl stehen: 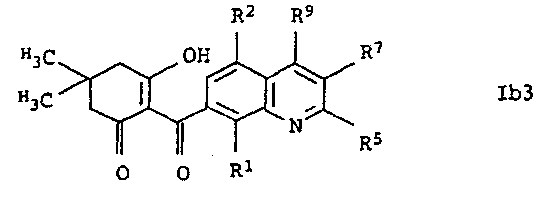
- die Verbindungen Ib4.01-Ib4.21, die sich von den entsprechenden Verbindungen Ib1.01-Ib1.21 dadurch unterscheiden, daß R¹⁸ und R¹⁹ jeweils für Methyl stehen: 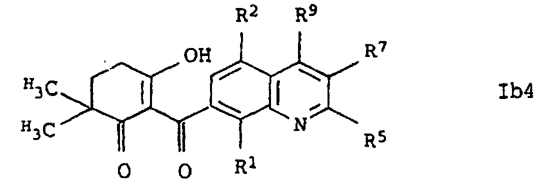
- die Verbindungen Ib5.01-Ib5.21, die sich von den entsprechenden Verbindungen Ib1.01-Ib1.21 dadurch unterscheiden, daß die -CR¹⁶R¹⁷-Einheit durch C=0 ersetzt ist: 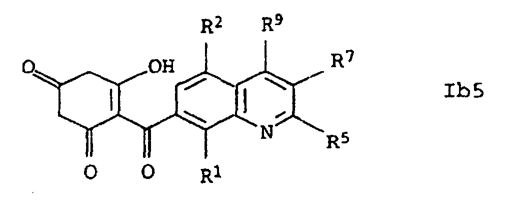
- die Verbindungen Ib6.01-Ib6.21, die sich von den entsprechenden Verbindungen Ib1.01 -Ib1.21 dadurch unterscheiden, daß R¹⁴, R¹⁸ und R¹⁹ für Methyl stehen und die -CR¹⁶R¹⁷-Einheit durch C=0 ersetzt ist: 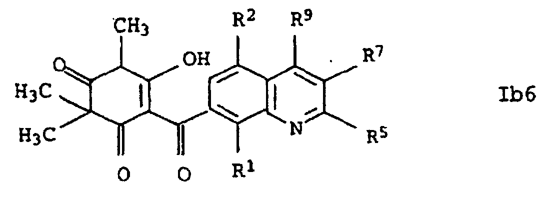
- die Verbindungen Ib7.01-Ib7.21, die sich von den entsprechenden Verbindungen Ib1.01-Ib1.21 dadurch unterscheiden, daß R¹⁴, R¹⁵, R¹⁸ und R¹⁹ für Methyl stehen und die -CR¹⁶R¹⁷-Einheit durch C=O ersetzt ist:

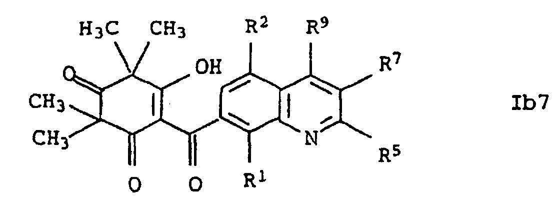

Daneben sind die in der folgenden Tabelle 3 aufgeführten Verbindungen Ic1 (=̂ I mit R², R¹⁴, R¹⁵, R¹⁶, R¹⁷, R¹⁸, R¹⁹ = H, wobei das "Q-CO-Fragment" in Position d, R¹ in Position a und Z¹ über die Positionen b und c gebunden sind) außerordentlich bevorzugt:

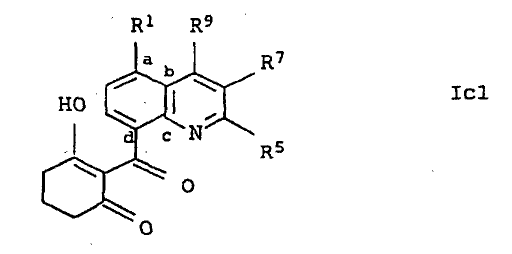

**Tabelle 3**

| Nr. | R¹ | R⁵ | R⁷ | R⁹ |
|---|---|---|---|---|
| Ic1.01 | Br | CH₃ | H | H |
| Ic1.02 | Cl | CH₃ | H | H |
| Ic1.03 | SO₂CH₃ | CH₃ | H | H |
| Ic1.04 | CH₃ | CH₃ | H | H |
| Ic1.05 | OH | CH₃ | H | H |
| Ic1.06 | OCH₃ | CH₃ | H | H |
| Ic1.07 | CF₃ | CH₃ | H | H |
| Ic1.08 | NO₂ | CH₃ | H | H |
| Ic1.09 | F | CH₃ | H | H |
| Ic1.10 | OCF₃ | CH₃ | H | H |
| Ic1.11 | C₆H₅ | CH₃ | H | H |
| Ic1.12 | Br | CF₃ | H | H |
| Ic1.13 | Cl | CF₃ | H | H |
| Ic1.14 | SO₂CH₃ | CF₃ | H | H |
| Ic1.15 | CH₃ | CF₃ | H | H |
| Ic1.16 | OH | CF₃ | H | H |
| Ic1.17 | OCH₃ | CF₃ | H | H |
| IC1.18 | CF₃ | CF₃ | H | H |
| Ic1.19 | NO₂ | CF₃ | H | H |
| Ic1.20 | F | CF₃ | H | H |
| Ic1.21 | OCF₃ | CF₃ | H | H |
| Ic1.22 | C₆H₅ | CF₃ | H | H |
| Ic1.23 | Br | H | H | H |
| Ic1.24 | Cl | H | H | H |
| Ic1.25 | SO₂CH₃ | H | H | H |
| Ic1.26 | CH₃ | H | H | H |
| Ic1.27 | OH | H | H | H |
| Ic1.28 | OCH₃ | H | H | H |
| Ic1.29 | CF₃ | H | H | H |
| Ic1.30 | NO₂ | H | H | H |
| Ic1.31 | F | H | H | H |
| Ic1.32 | OCF₃ | H | H | H |
| Ic1.33 | C₆H₅ | H | H | H |
| Ic1.34 | Br | Cl | H | H |
| Ic1.35 | Cl | Cl | H | H |
| Ic1.36 | SO₂CH₃ | Cl | H | H |
| Ic1.37 | CH₃ | Cl | H | H |
| Ic1.38 | OH | Cl | H | H |
| Ic1.39 | OCH₃ | Cl | H | H |
| Ic1.40 | CF₃ | Cl | H | H |
| Ic1.41 | NO₂ | Cl | H | H |
| Ic1.42 | F | Cl | H | H |
| Ic1.43 | OCF₃ | Cl | H | H |
| Ic1.44 | C₆H₅ | Cl | H | H |

Desweiteren sind die folgenden Hetaroylderivate der Formel I außerordentlich bevorzugt:
- die Verbindungen Ic2.01-Ic2.44, die sich von den Verbindungen Ic1.01-Ic1.44 dadurch unterscheiden, daß R¹⁶ für Methyl steht: 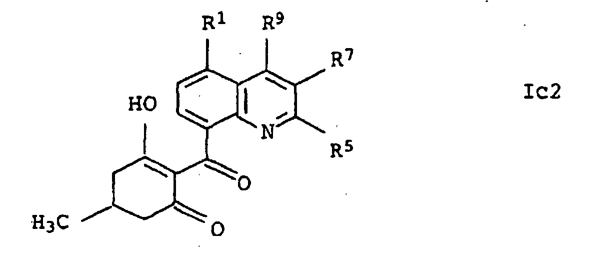
- die Verbindungen Ic3.01-Ic3.44, die sich von den Verbindungen Ic1.01-Ic1.44 dadurch unterscheiden, daß R¹⁶ und R¹⁷ jeweils für Methyl stehen: 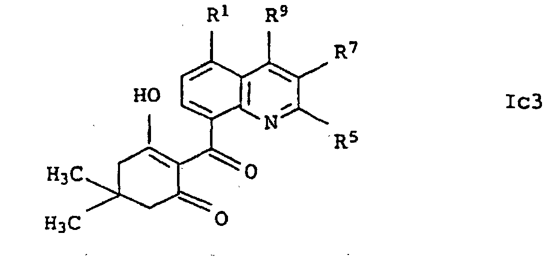
- die Verbindungen Ic4.01-Ic4.44, die sich von den Verbindungen Ic1.01-Ic1.44 dadurch unterscheiden, daß R¹⁸ und R¹⁹ jeweils für Methyl stehen: 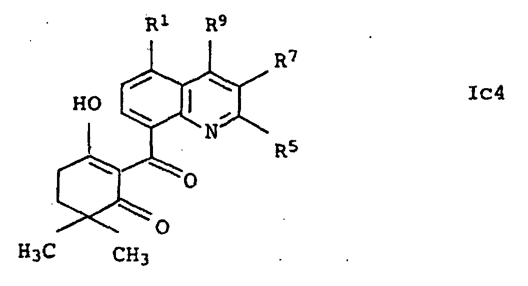
- die Verbindungen Ic5.01-Ic5.44, die sich von den entsprechenden Verbindungen Ic1.01-Ic1.44 dadurch unterscheiden, daß die -CR¹⁶R¹⁷-Einheit durch C=O ersetzt ist: 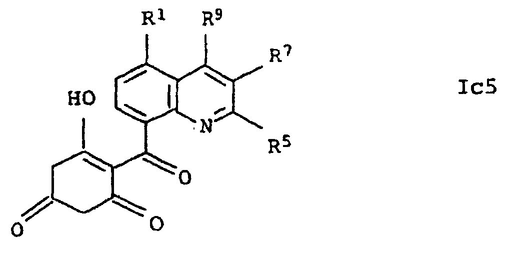
- die Verbindungen Ic6.01-Ic6.44, die sich von den entsprechenden Verbindungen Ic1.01-Ic1.44 dadurch unterscheiden, daß R¹⁴, R¹⁸ und R¹⁹ für Methyl stehen und die -CR¹⁶R¹⁷-Einheit durch C=O ersetzt ist: 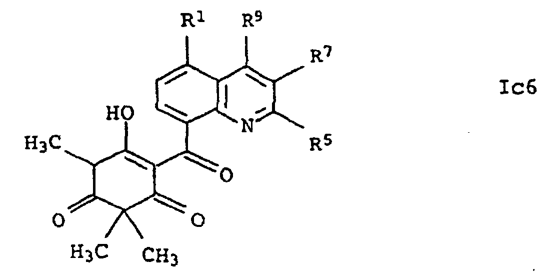
- die Verbindungen Ic7.01-Ic7.44, die sich von den entsprechenden Verbindungen Ic1.01-Ic1.44 dadurch unterschieden, daß R¹⁴, R¹⁵, R¹⁸ und R¹⁹ für Methyl stehen und die -CR¹⁶R¹⁷-Einheit durch C=O ersetzt ist:

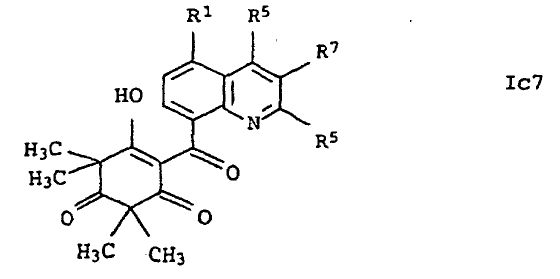

Daneben sind die in der folgenden Tabelle 4 aufgeführten Verbindungen Id1 (=̂ I mit R², R¹⁴, R¹⁵, R¹⁶, R¹⁷, R¹⁸, R¹⁹ = H, wobei das "Q-CO-Fragment" in Position a, R¹ in Position d und Z² über die Positionen b und c gebunden sind) außerordentlich bevorzugt:

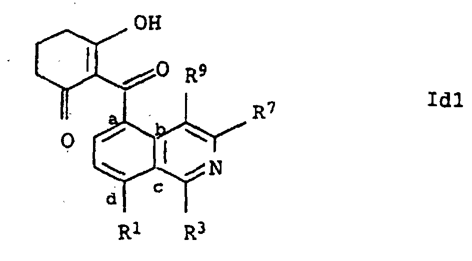

**Tabelle 4**

| Nr. | R¹ | R³ | R⁷ | R⁹ |
|---|---|---|---|---|
| Id1.01 | Br | H | H | H |
| Id1.02 | Cl | H | H | H |
| Id1.03 | SO₂CH₃ | H | H | H |
| Id1.04 | CH₃ | H | H | H |
| Id1.05 | OH | H | H | H |
| Id1.06 | OCH₃ | H | H | H |
| Id1.07 | CF₃ | H | H | H |
| Id1.08 | NO₂ | H | H | H |
| Id1.09 | F | H | H | H |
| Id1.10 | OCF₃ | H | H | H |
| Id1.11 | Br | CH₃ | H | H |
| Id1.12 | Cl | CH₃ | H | H |
| Id1.13 | SO₂CH₃ | CH₃ | H | H |
| Id1.14 | CH₃ | CH₃ | H | H |
| Id1.15 | OH | CH₃ | H | H |
| Id1.16 | OCH₃ | CH₃ | H | H |
| Id1.17 | CF₃ | CH₃ | H | H |
| Id1.18 | NO₂ | CH₃ | H | H |
| Id1.19 | F | CH₃ | H | H |
| Id1.20 | H | CH₃ | H | H |
| Id1.21 | OCF₃ | CH₃ | H | H |
| Id1.22 | Br | CH₃ | CH₃ | H |
| Id1.23 | Cl | CH₃ | CH₃ | H |
| Id1.24 | SO₂CH₃ | CH₃ | CH₃ | H |
| Id1.25 | CH₃ | CH₃ | CH₃ | H |
| Id1.26 | OH | CH₃ | CH₃ | H |
| Id1.27 | OCH₃ | CH₃ | CH₃ | H |
| Id1.28 | CF₃ | CH₃ | CH₃ | H |
| Id1.29 | NO₂ | CH₃ | CH₃ | H |
| Id1.30 | F | CH₃ | CH₃ | H |
| Id1.31 | OCF₃ | CH₃ | CH₃ | H |
| Id1.32 | Br | Cl | Cl | Cl |
| Id1.33 | Cl | Cl | Cl | Cl |
| Id1.34 | SO₂CH₃ | Cl | Cl | Cl |
| Id1.35 | CH₃ | Cl | Cl | Cl |
| Id1.36 | OH | Cl | Cl | Cl |
| Id1.37 | OCH₃ | Cl | Cl | Cl |
| Id1.38 | CF₃ | Cl | Cl | Cl |
| Id1.39 | NO₂ | Cl | Cl | Cl |
| Id1.40 | F | Cl | Cl | Cl |
| Id1.41 | OCF₃ | Cl | Cl | Cl |
| Id1.42 | Br | OCH₃ | Cl | H |
| Id1.43 | Cl | OCH₃ | Cl | H |
| Id1.44 | SO₂CH₃ | OCH₃ | Cl | H |
| Id1.45 | CH₃ | OCH₃ | Cl | H |
| Id1.46 | OH | OCH₃ | Cl | H |
| Id1.47 | OCH₃ | OCH₃ | Cl | H |
| Id1.48 | CF₃ | OCH₃ | Cl | H |
| Id1.49 | NO₂ | OCH₃ | Cl | H |
| Id1.50 | F | OCH₃ | Cl | H |
| Id1.51 | OCF₃ | OCH₃ | Cl | H |
| Id1.52 | Br | H | OCH₃ | H |
| Id1.53 | Cl | H | OCH₃ | H |
| Id1.54 | SO₂CH₃ | H | OCH₃ | H |
| Id1.55 | CH₃ | H | OCH₃ | H |
| Id1.56 | OH | H | OCH₃ | H |
| Id1.57 | OCH₃ | H | OCH₃ | H |
| Id1.58 | CF₃ | H | OCH₃ | H |
| Id1.59 | NO₂ | H | OCH₃ | H |
| Id1.60 | F | H | OCH₃ | H |
| Id1.61 | OCF₃ | H | OCH₃ | H |
| Id1.62 | Br | CH₃ | CH₃ | CH₃ |
| Id1.63 | Cl | CH₃ | CH₃ | CH₃ |
| Id1.64 | SO₂CH₃ | CH₃ | CH₃ | CH₃ |
| Id1.65 | CH₃ | CH₃ | CH₃ | CH₃ |
| Id1.66 | OH | CH₃ | CH₃ | CH₃ |
| Id1.67 | OCH₃ | CH₃ | CH₃ | CH₃ |
| Id1.68 | CF₃ | CH₃ | CH₃ | CH₃ |
| Id1.69 | NO₂ | CH₃ | CH₃ | CH₃ |
| Id1.70 | F | CH₃ | CH₃ | CH₃ |
| Id1.71 | OCF₃ | CH₃ | CH₃ | CH₃ |
| Id1.72 | Br | Cl | H | H |
| Id1.73 | Cl | Cl | H | H |
| Id1.74 | SO₂CH₃ | Cl | H | H |
| Id1.75 | CH₃ | Cl | H | H |
| Id1.76 | OH | Cl | H | H |
| Id1.77 | OCH₃ | Cl | H | H |
| Id1.78 | CF₃ | Cl | H | H |
| Id1.79 | NO₂ | Cl | H | H |
| Id1.80 | F | Cl | H | H |
| Id1.81 | OCF₃ | Cl | H | H |
| Id1.82 | Br | Cl | Cl | H |
| Id1.83 | Cl | Cl | Cl | H |
| Id1.84 | SO₂CH₃ | Cl | Cl | H |
| Id1.85 | CH₃ | Cl | Cl | H |
| Id1.86 | OH | Cl | Cl | H |
| Id1.87 | OCH₃ | Cl | Cl | H |
| Id1.88 | CF₃ | Cl | Cl | H |
| Id1.89 | NO₂ | Cl | Cl | H |
| Id1.90 | F | Cl | Cl | H |
| Id1.91 | OCF₃ | Cl | Cl | H |

Desweiteren sind die folgenden Hetaroylderivate der Formel I außerordentlich bevorzugt:
- die Verbindungen Id2.01-Id2.91, die sich von den Verbindungen Id1.01-Id1.91 dadurch unterscheiden, daß R¹⁶ für Methyl steht: 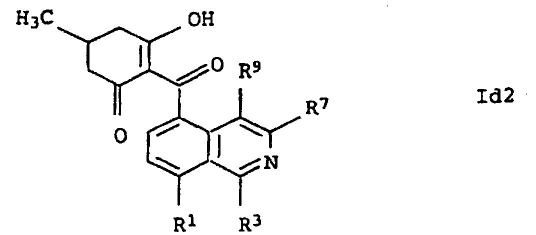
- die Verbindungen Id.3.01-Id3.91, die sich von den Verbindungen Id1.01-Id1.91 dadurch unterscheiden, daß R¹⁶ und. R¹⁷ für Methyl stehen: 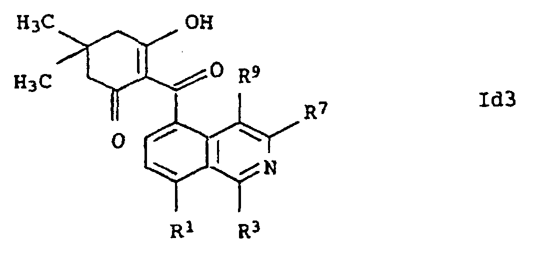
- die Verbindungen Id4.01-Id4.91, die sich von den Verbindungen Id1.01-Id1.91 dadurch unterscheiden, daß R¹⁸ und R¹⁹ jeweils für Methyl stehen: 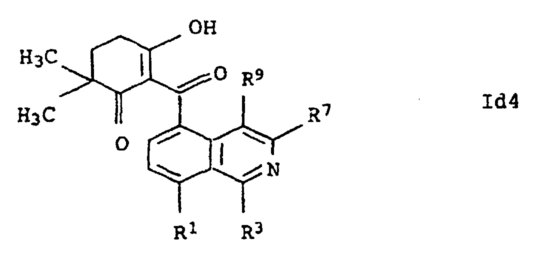
- die Verbindungen Id5.01-Id5.91, die sich von den entsprechenden Verbindungen Id1.01-Id1.91 dadurch unterschieden, daß die -CR¹⁶R¹⁷-Einheit durch C=O ersetzt ist: 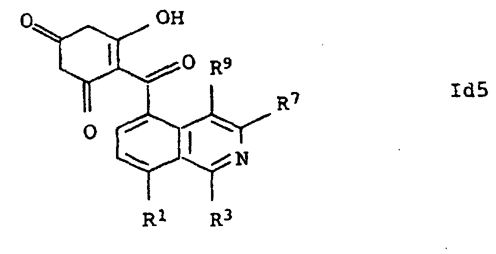
- die Verbindungen Id6.01-Id6.91, die sich von den entsprechenden Verbindungen Id1.01-Id1.91 dadurch unterscheiden, daß R¹⁴, R¹⁸ und R¹⁹ für Methyl stehen und die -CR¹⁶R¹⁷-Einheit durch C=O ersetzt ist: 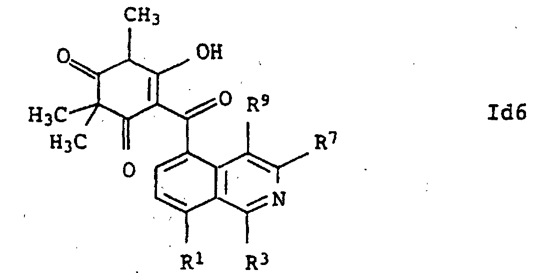
- die Verbindungen Id7.01-Id7.91, die sich von den entsprechenden Verbindungen Id1.01-Id1.91 dadurch unterscheiden, daß R¹⁴, R¹⁵, R¹⁸ und R¹⁹ für Methyl stehen und die -CR¹⁶R¹⁷-Einheit durch C=O ersetzt ist:

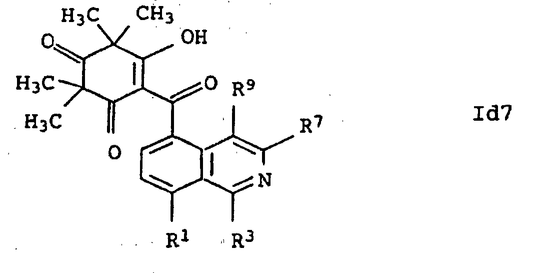

Die Hetaroylderivate der Formel I sind auf verschiedene Art und weise erhältlich, beispielsweise nach folgendem Verfahren:
Umsetzung von Cyclohexandionen der Formel II mit einer aktivierten Carbonsäure IIIa oder einer Carbonsäure IIIb, die vorzugsweise in Situ aktiviert wird, zu dem Acylierungsprodukt und anschließende Umlagerung.

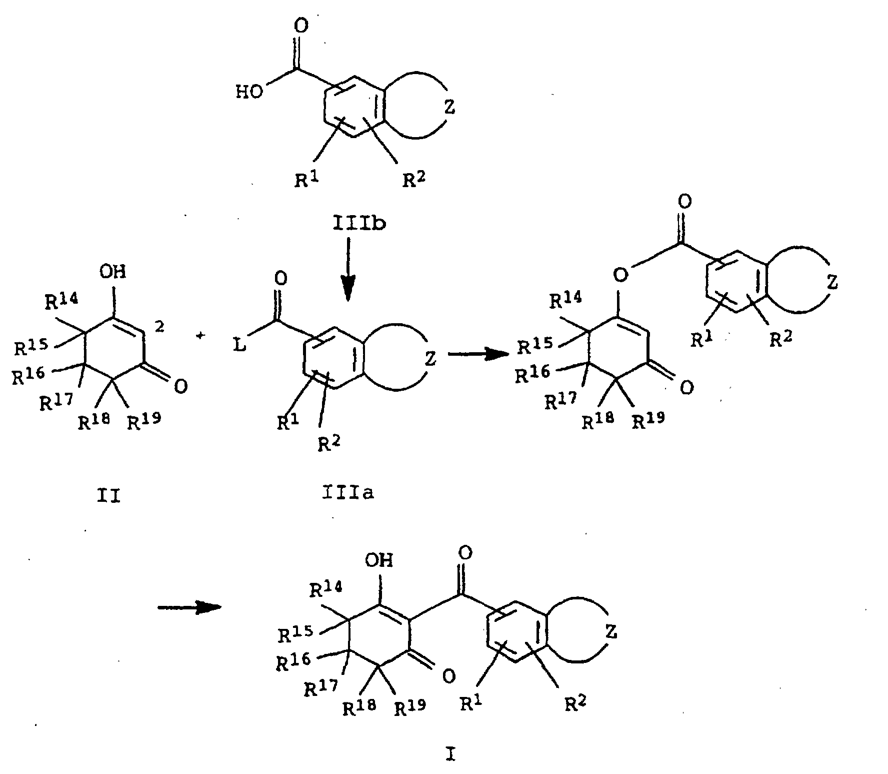

L steht für eine nucleophil verdrängbare Abgangsgruppe, wie Halogen z.B. Brom, Chlor, Heterocyclyl, z.B. Imidazolyl, Pyridyl, Carboxylat, z.B. Acetat, Trifluoracetat etc.

Die aktivierte Hetaroylcarbonsäure kann direkt eingesetzt werden, wie im Fall der Hetaroylhalogenide oder in situ erzeugt werden, z.B. mit Dicyclohexylcarbodiimid, Triphenylphosphin/Azodicarbonsäureester, 2-Pyridindisulfit/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis. 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels kann der rohe Enolester ohne weitere Reinigung zur Umlagerung eingesetzt werden.

Die Umlagerung der Enolester zu den Verbindungen der Formel I erfolgt zweckmäßigerweise bei Temperaturen von 20 bis 40°C in einem Lösungsmittel und in Gegenwart einer Hilfsbase sowie mit Hilfe einer Cyanoverbindung als Katalysator.

Als Lösungsmittel können z.B. Acetonitril, Methylenchlorid, 1,2-Dichlorethan, Essigsäureethylester, Toluol oder Gemische hiervon verwendet werden. Bevorzugtes Lösungsmittel ist Acetonitril.

Geeignete Hilfsbasen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Enolester, eingesetzt werden. Bevorzugt wird Triethylamin verwendet, vorzugsweise in doppelt äquimolaren Verhältnis in Bezug auf den Enolester.

Als "Umlagerungskatalysator" kommen anorganische Cyanide, wie Natriumcyanid, Kaliurocyanid und organische Cyanoverbindungen, wie Acetoncyanhydrin, Trimethylsilylcyanid in Betracht. Sie werden in einer Menge von 1 bis 50 Molprozent, bezogen auf den Enolester, eingesetzt. Vorzugsweise werden Acetoncyanhydrin oder Trimethylsilylcyanid, z.B. in einer Menge von 5 bis 15, vorzugsweise 10 Molprozent, bezogen auf den Enolester, eingesetzt.

Die Aufarbeitung kann in an sich bekannter Weise erfolgen. Das Reaktionsgemisch wird z.B. mit verdünnter Mineralsäure, wie z.B. 5%ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt. (Beispiele für die Darstellung von Enolestern von Cyclohexan-1,3-dionen und für die cyanidkatalysierte Umlagerung der Enolester sind z.B. in EP-A 186 118, US 4 780 127 genannt).

Die als Ausgangsmaterialien verwendeten Cyclohexan-1,3-dion der Formel II sind bekannt oder können nach an sich bekannten Verfahren hergestellt werden (z.B. EP-A 71 707, EP-A 142 741, EP-A 243 313, US 4 249 937; WO 92/13821).

Die Carbonsäurehalogenide der Formel IIIa (mit L = Br, Cl) können auf an sich bekannte Art und Weise durch Umsetzung der Carbonsäuren der Formel IIIb mit Halogenierungsreagentien wie Thionylchlorid, Thionylbromid, Phosgen, Diphosgen, Triphosgen, Oxalylchlorid, Oxalylbromid hergestellt werden.

Die Carbonsäuren der Formel IIIb sind an sich bekannt oder können auf an sich bekannte Art und Weise dargestellt werden (The Chemistry of Heterocyclic Compounds, Vol. 32, "Quinolines, Part I, II and III", Editor E. Taylor, Verlag Wiley & Sons; The Chemistry of Heterocyclic Compounds, Vol. 38, "Isoquinolines, Part I and II", Editor A. Weissemberger and E. Taylor, Verlag Wiley & Sons; T. Eicher, S. Hauptmann, "Chemie der Heterocyclen", Thieme Verlag 1994)

Beispielsweise können ggf. substituierte Aminobenzoesäuren durch Umsetzung mit Glycerin, ggf. substituierten Glycerinderivaten oder α,β-ungesättigten Carbonylverbindungen nach Skraup zu den entsprechenden Chinolincarbonsäuren umgesetzt werden (vgl. EP-A 294 685, DE-A 33 26 225) (Schema 1)

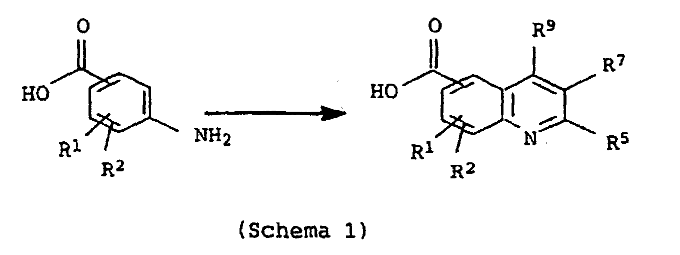

Ebenso ist es möglich, ggf. substituierte Aniline mit Glycerin, ggf. substituierten Glycerinderivaten oder α,β-ungesättigten Carbonylverbindungen umzusetzen. Nach Halogenierung und Austausch der Halogenfunktion durch Cyanid (z.B. mittels Kupfer(I)cyanid) wird das Nitril zur entsprechenden Chinolincarbonsäure verseift (vgl. Khim. Greterotsikl. Soedin 1980, 3, 366 (=̂ CA 93, 71504)). (Schema 2)

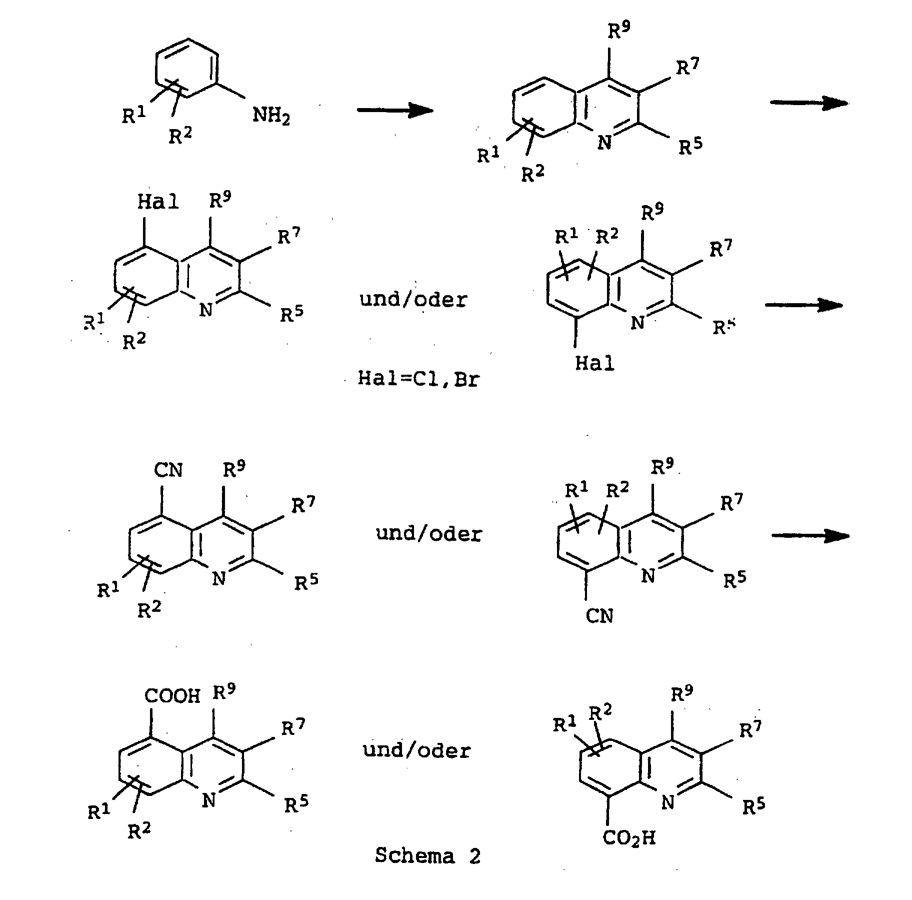

Nicht literaturbekannte Aniline können durch Reduktion aus den entsprechenden Nitrobenzolen erhalten werden. Hierfür eignen sich z.B. katalytische Hydrierung wie an Raneynickel, Pt/C, Pd/C, Rh/C oder auch Reduktion mit Eisenpulver, Zinkpulver etc. in einem Gemisch aus org. Säure, z.B. Essigsäure, Propionsäure und protischen Lösungsmittel wie Methanol, Ethanol oder Wasser.

Die Nitrobenzole können durch Nitrierung, Substituionsreaktionen etc. aufgebaut werden. Schema 3 stellt exemplarisch eine Synthese-Sequenz dar.

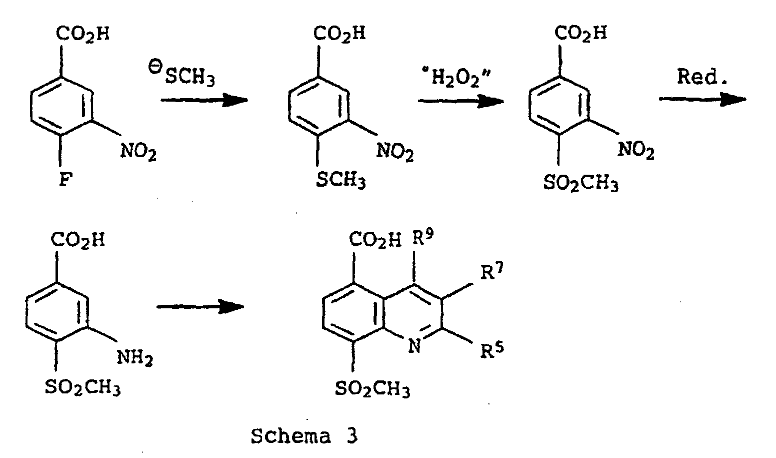

Isochinolincarbonsäuren können z.B. aus halogenierten Isochinolinen, anschließendem Halogen/Cyanid-Austausch (Chem. Ber. 1919, 52, 1749) und nachfolgender Verseifung aufgebaut werden. (Schema 4)

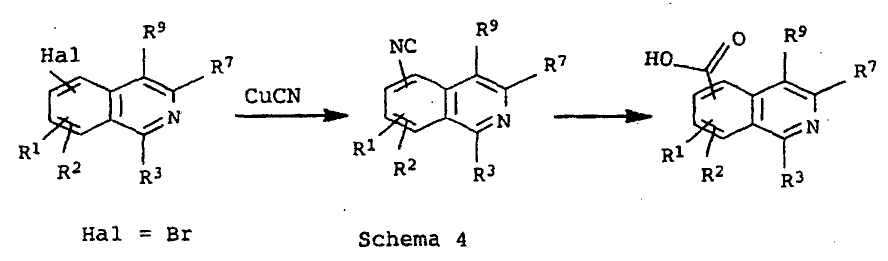

Ebenso ist es möglich aus nitrierten Isochinolinen die entsprechenden Aminoisochinoline mittels Reduktion (wie oben genannt) darzustellen. Anschließende Diazotierung, Sandmeyer-Reaktion mit Cyanid und verseifung führen zu Isochinolincarbonsäuren (Schema 5).

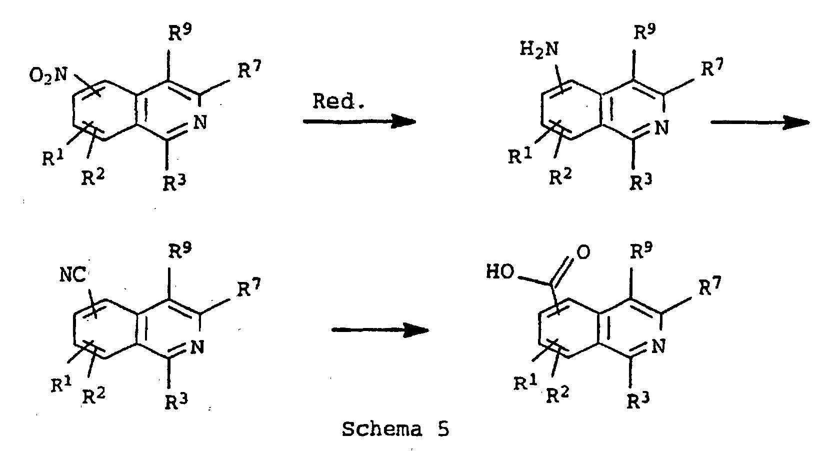

Halogenierte bzw. nitrierte Isochinoline können gemäß EP-A 633 262 dargestellt werden. Weiterhin ist es möglich, halogenierte Isochinoline ausgehend von ggf. substituierten Benzaldehyden, mittels Umsetzung mit Aminoacetaldehydacetal und anschließender Halogenierung (Helv. Chim. Acta 1985, 68, 1828) zu erhalten (Schema 6).

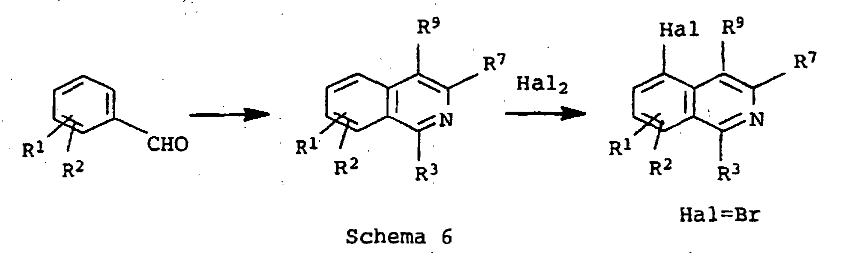

Die N-Oxide der Chinolin- bzw. Isochinolincarbonsäuren können durch Oxidation mit Wasserstoffperoxid aus den entsprechenden Chinolin- bzw. Isochinolincarbonsäuren erhalten werden. Es kann von Vorteil sein, die entsprechenden Säuren zuerst in den C₁-C₆-Alkyl-Ester überzuführen, die Oxidation mit Wasserstoffperoxid durchzuführen und anschließend den Ester zu verseifen.

2,3-Dihydrochinolin-Derivate können u.a. durch Cyclisierung von γ-funktionalisierten, N-Alkylanilinen ggf. mit Hilfe von Lewisoder Protonensäuren, erhalten werden (Heterocycles 1986, 24, 2109; J. Am. Chem. Soc. 1949, 71, 1901).

Tetrahydroisochinolin Derivate können aus Isochinolinen mittels Reduktion mit Wasserstoff, ggf. unter Metallkatalyse, z.B. an Pt in Essigsäure, erhalten werden. Es ist aber auch möglich, Isochinoline mit Dimethylsulfat umzusetzen und diese durch Reduktion mit Natriumboranat in Tetrahydroisochinolin-Derivate überzuführen.

### Herstellbeispiele

### 2-(8-Bromchinolin-5-yl)-carbonyl-1,3-cyclohexandion (Verbindung 5.02)

- Stufe 1:: 8-Brom-5-chinolincarbonsäurechlorid
2,3 g 8-Brom-5-chinolincarbonsäure wurden zusammen mit 40 ml Toluol, 1 Tropfen Dimethylformamid und 1,2 g Thionylchlorid 1 Stunde auf Rückflußtemperatur erwärmt. Danach wurde das Lösungsmittel abdestilliert und das erhaltene Säurechlorid direkt weiter umgesetzt.
- Stufe 2:: 0,9 g 1,3-Cyclohexandion, 10 ml Methylenchlorid und 0,9 g Triethylamin wurden vorgelegt und bei 0-10°C 2,1 g Säurechlorid aus Stufe 1 in 30 ml Methylenchlorid zugetropft. Es wurde 1 Stunde bei Raumtemperatur nachgerührt. Die Reaktionslösung wurde dann mit wasser verdünnt, mit Salzsäure sauer gestellt und mit Methylenchlorid extrahiert. Die organische Phase wurde getrocknet und eingeengt. Das o-acylierte Zwischenprodukt wurde chromatographisch an Kieselgel gereinigt.
Ausbeute: 1,2 g
(Schmelzpunkt: 118°C)
- Stufe 3:: 1,1g des O-acylierten Zwischenproduktes aus Stufe 2 wurden in 30 ml Acetonitril gelöst und dann mit 1,1 g Triethylamin und 0,2 g Acetoncyanhydrin versetzt. Man rührte 1 Stunde. Danach wurde die Reaktionslösung auf 2 N Salzsäure gegossen und mit Essigsäureethylester extrahiert. Man behandelte die organische Phase anschließend mit Natriumcarbonat-Lösung, stellt die wäßrige alkalische Phase sauer und extrahiert wieder mit Essigsäureethylester. Die organische Phase wurde getrocknet, eingeengt und durch Chromatographie an Kieselgel gereinigt.
Ausbeute: 0,13 g
(Schmelzpunkt: 180°C)

### 2-(5-Nitrochinolin-8-yl)-carbonyl-cyclohexan-1,3-dion (Verbindung 8.01)

1,0 g 5-Nitro-8-chinolincarbonsäure wurden mit 0,5 g 1,3-Cyclohexandion und 1,0 g Dicyclohexylcarbodiimid etwa 12 Stunden in 15 ml Acetonitril bei Raumtemperatur gerührt. Anschließend gab man 0,7 g Triethylamin und 0,2 ml Acetoncyanhydrin zu. Nach 4 Stunden gab man die Reaktionslösung auf wäßrige Natriumcarbonat-Lösung, extrahierte mit Essisäureethylester, stellte die wäßrige Phase mit Salzsäure sauer und extrahierte mit Essigsäureethylester. Man trocknete die organische Phase, destillierte das Lösungsmittel ab und chromatographierte an Kieselgel.
Ausbeute: 0,14 g
(¹H-NMR (CDCl₃, δ in ppm): 2,10 (2H); 2,36 (2H); 2,85 (2H); 7,62 (2H); 8,43 (1H); 8,93 (1H); 9,06 (1H); 16,39 (1H))

In den folgenden Tabellen 5-16 sind neben den vorstehend beschriebenen Hetaroylderivaten der Formel I noch weitere aufgeführt. die in analoger Weise hergestellt wurden oder herstellbar sind:

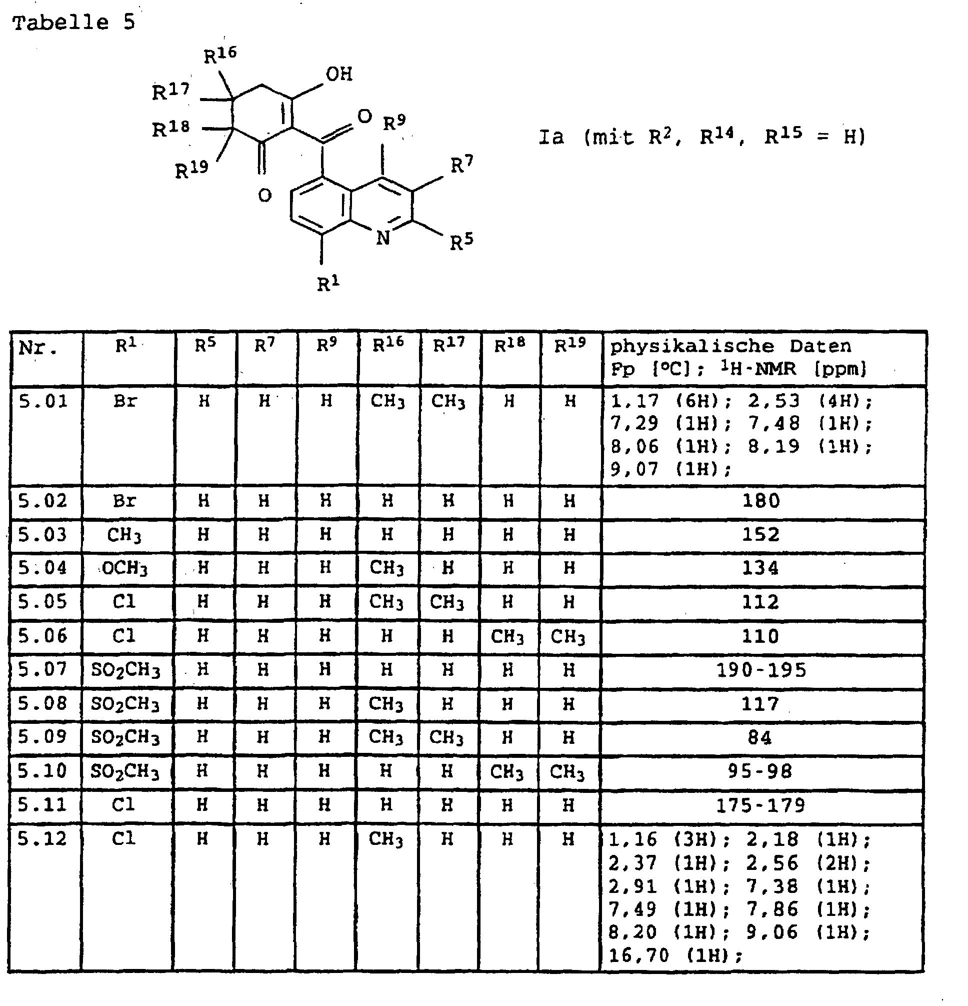

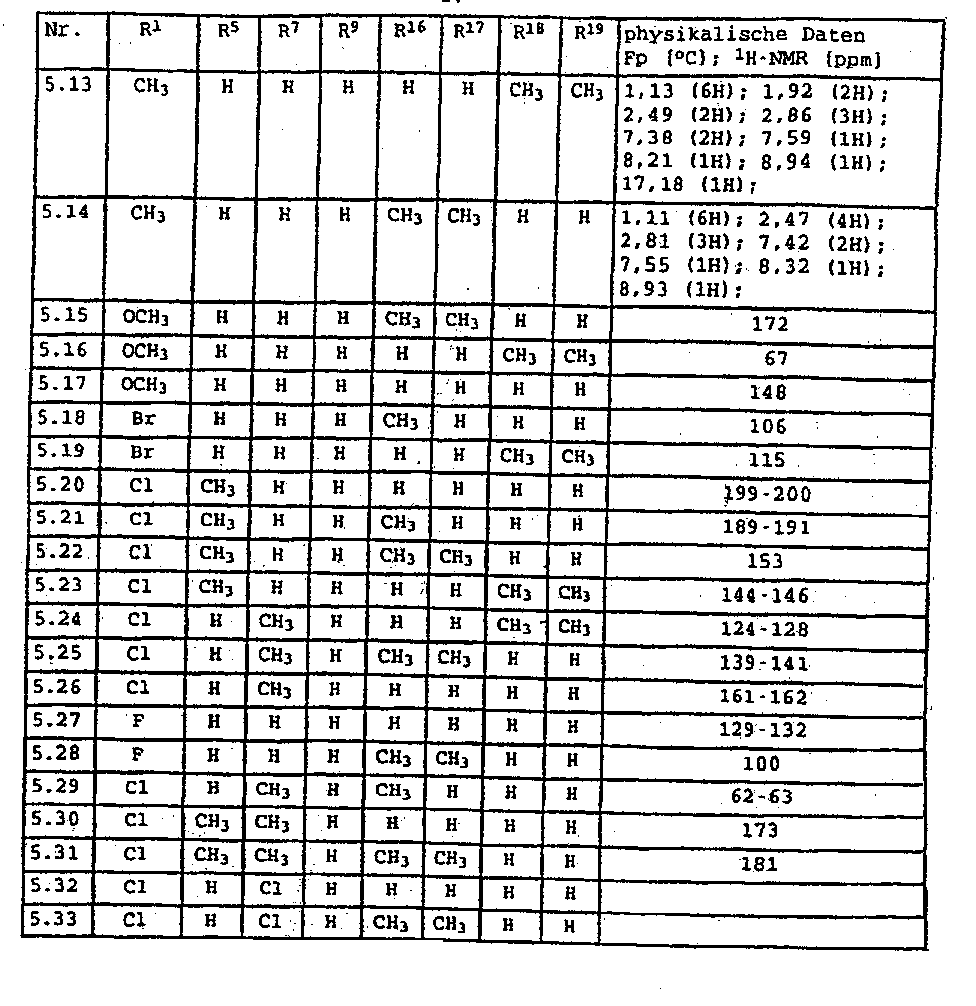

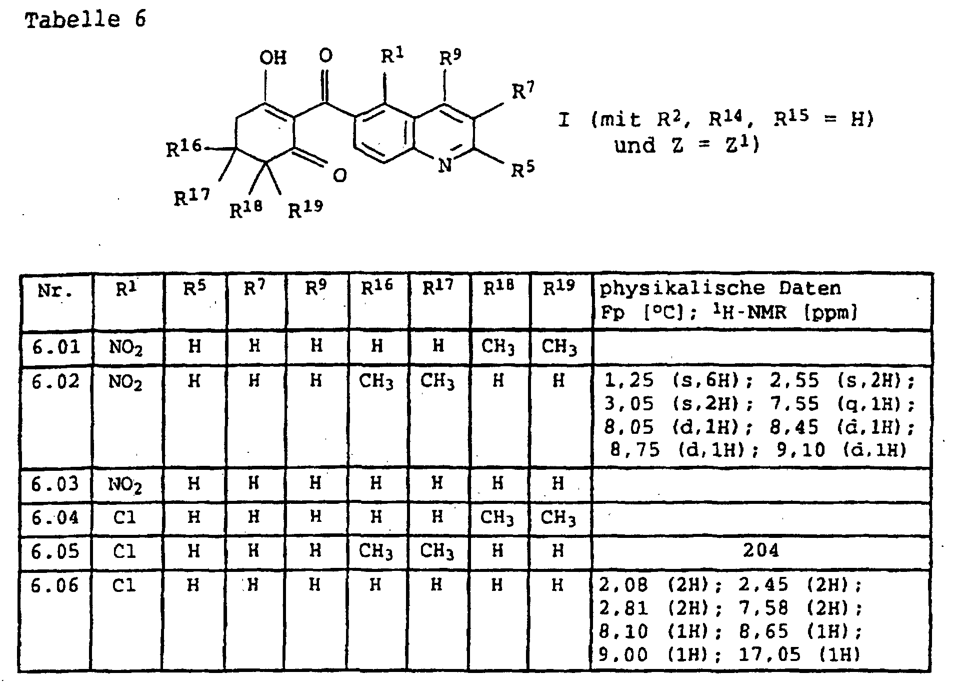

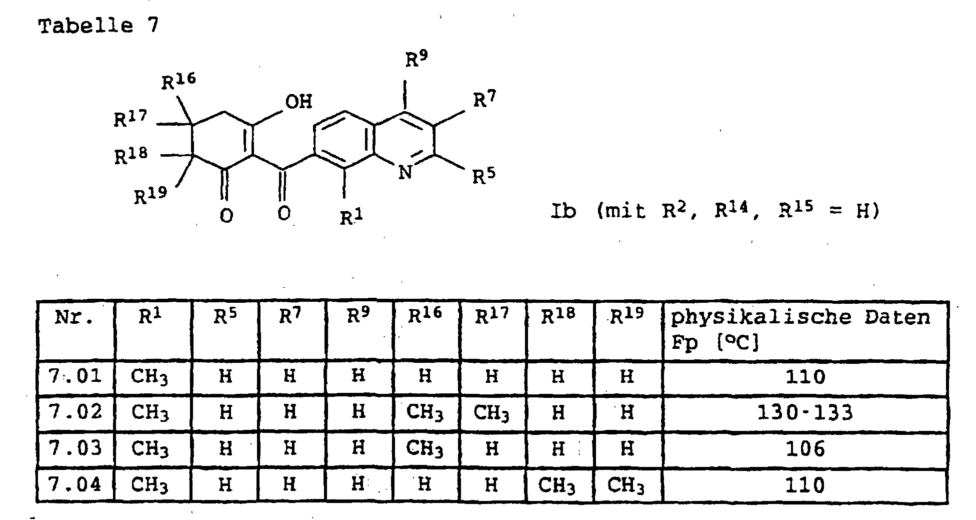

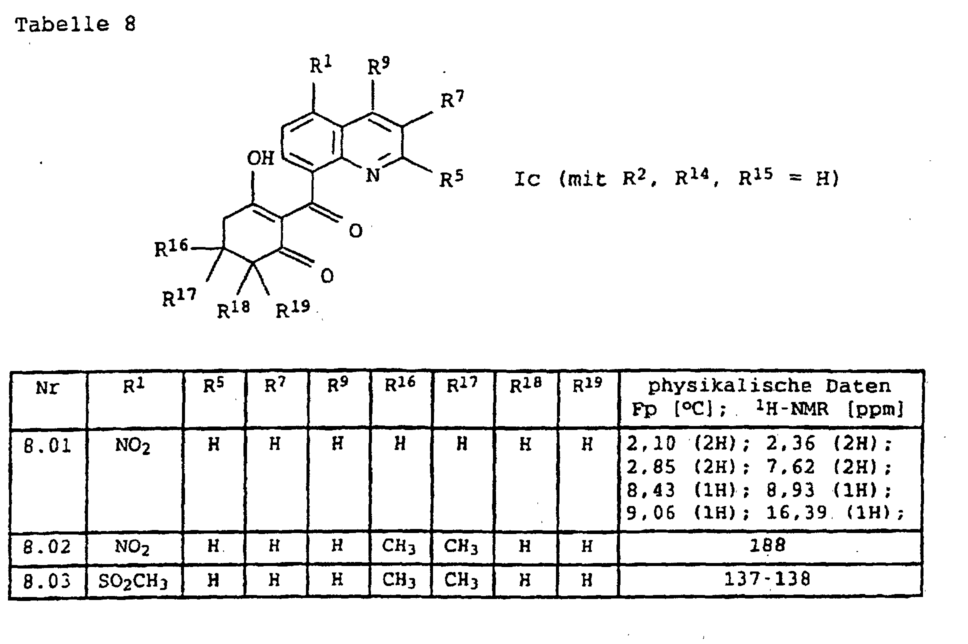

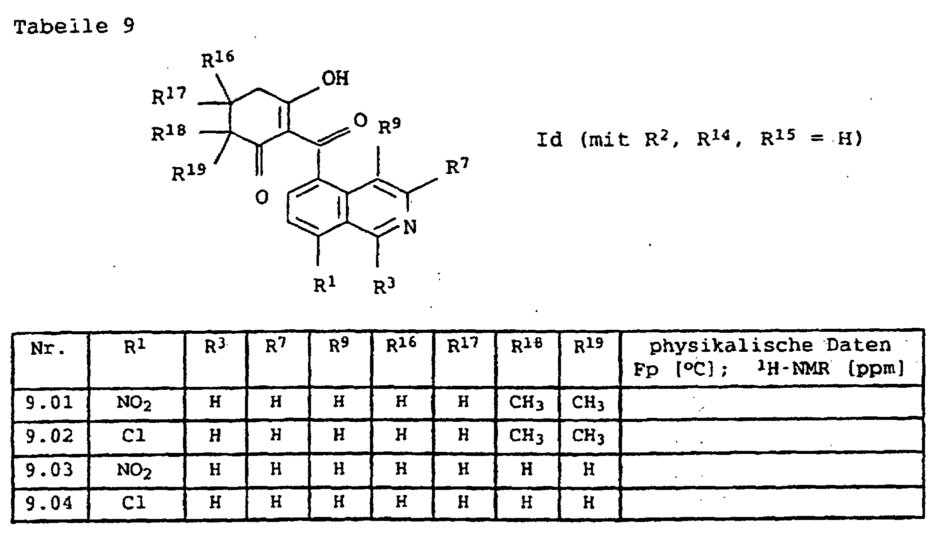

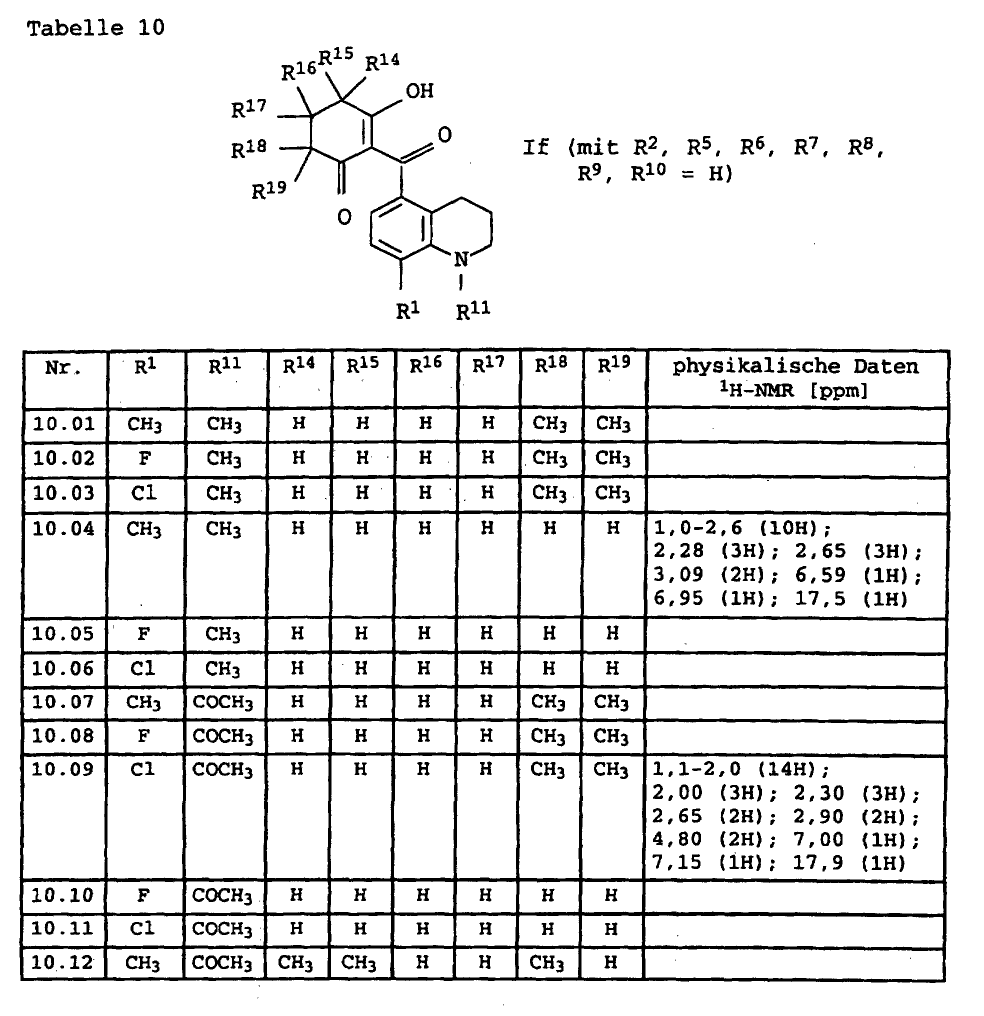

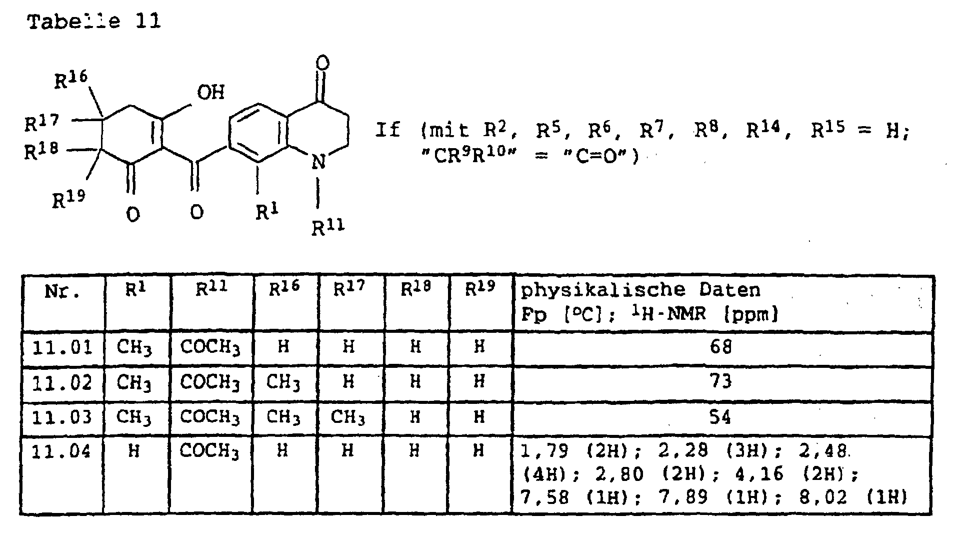

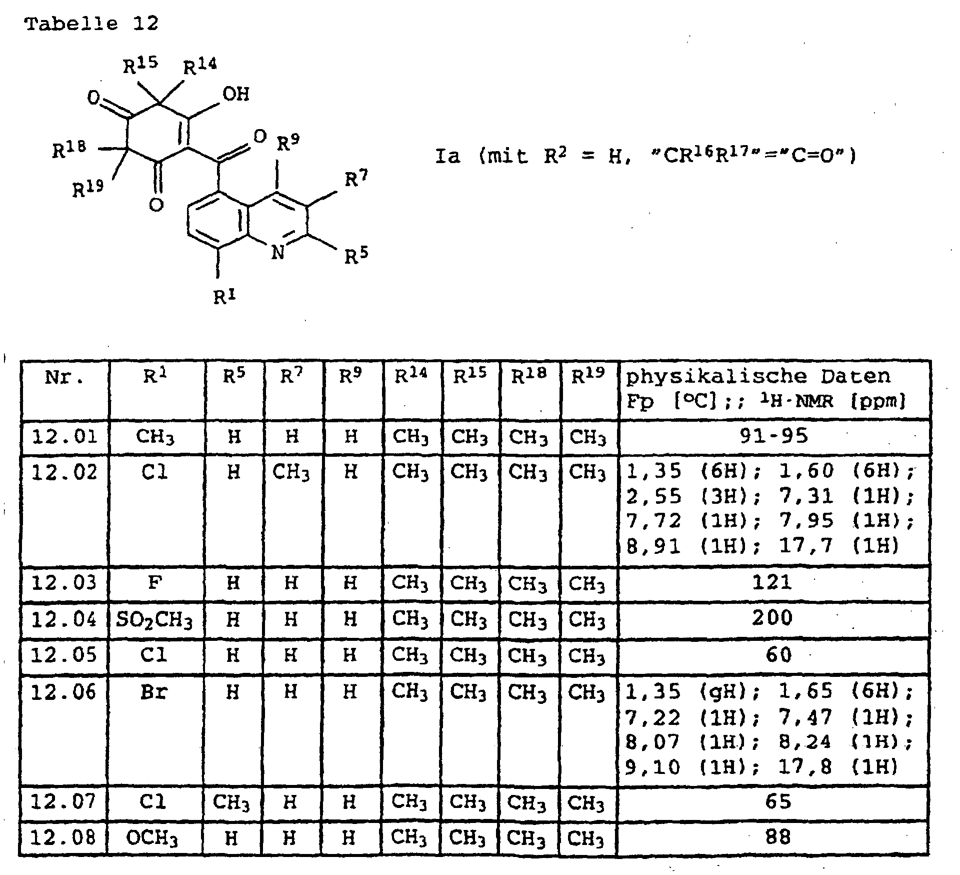

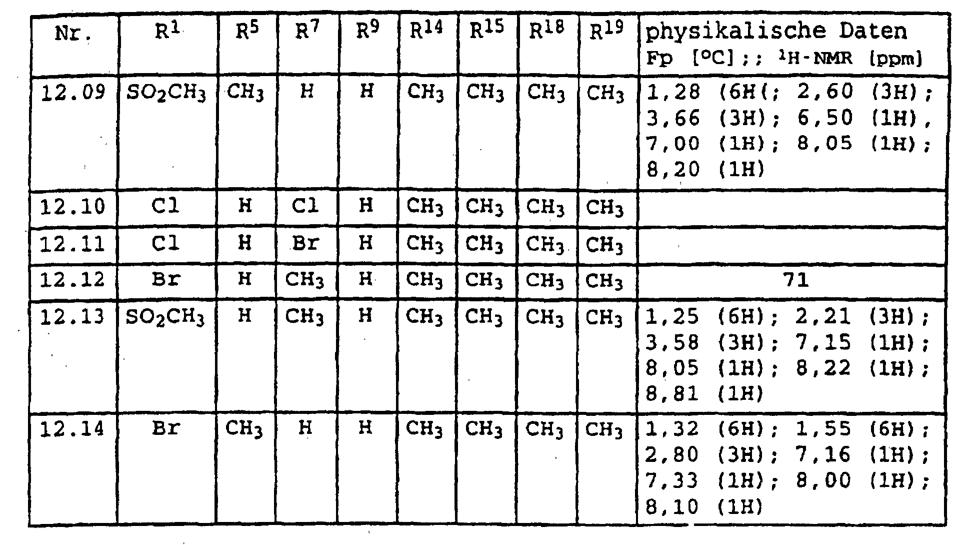

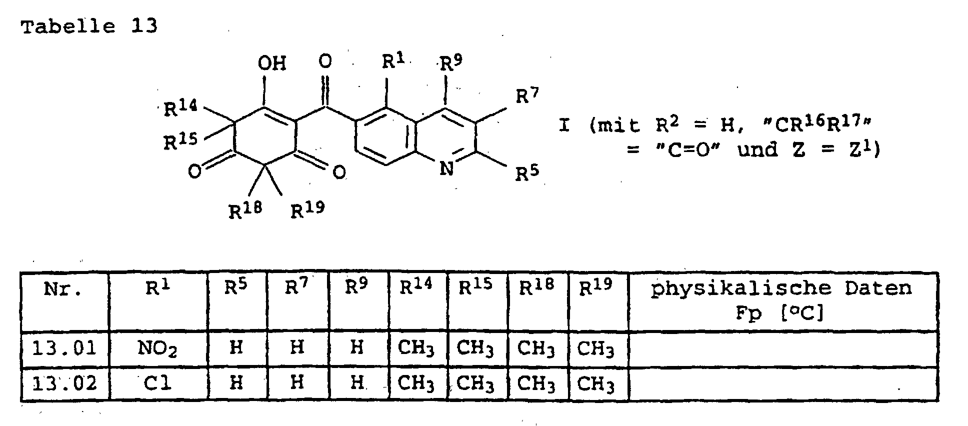

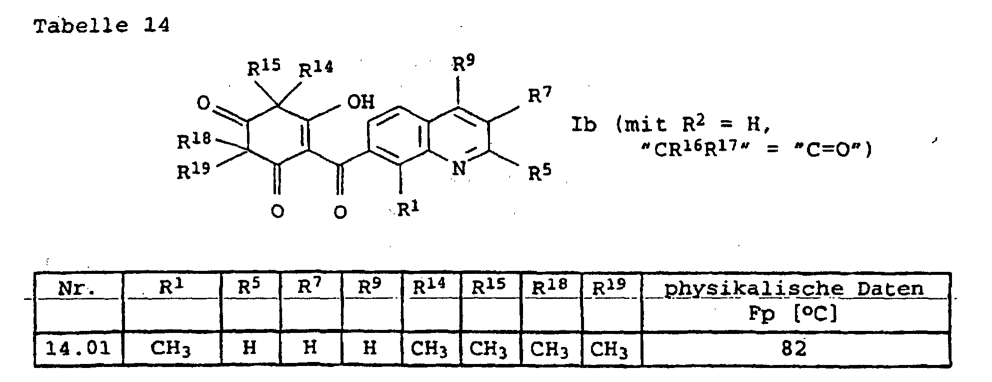

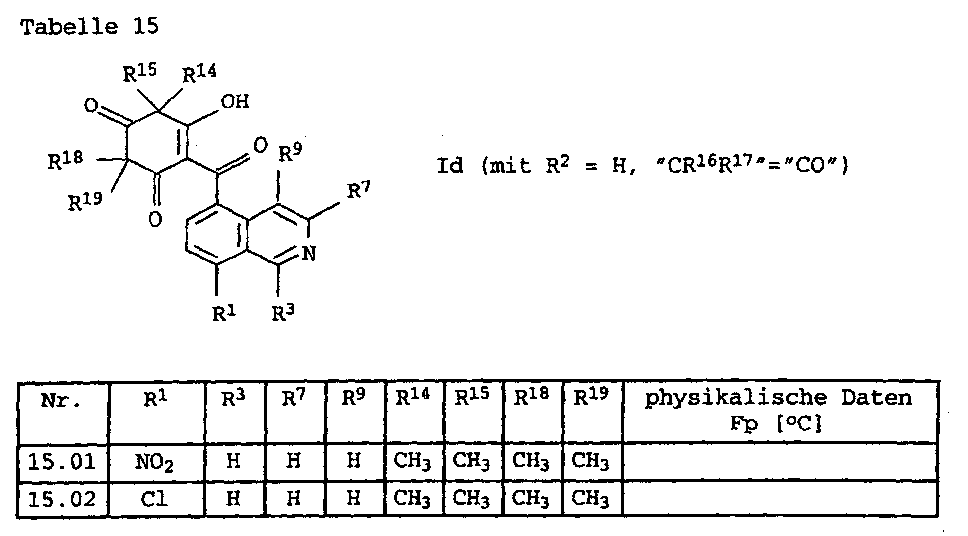

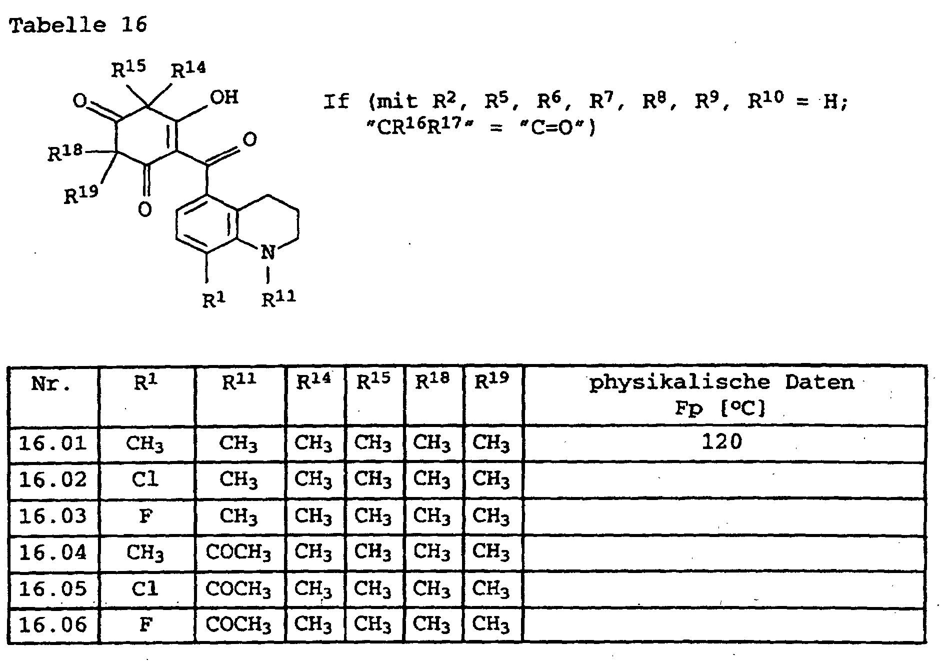

Nachfolgend sind die Synthesen einiger Carbonsäuren der Formel IIIb aufgeführt.

### 8-Methylsulfonyl-5-chinolincarbonsäure (Verbindung 17.06)

- Stufe 1:: 3-Nitro-4-thiomethylbenzoesäure
0,75 mol 4-Fluor-3-nitrobenzoesäure wurden in
2 1 Methanol vorgelegt und 0,75 mol Natriummethylat zugetropft. Danach wurden 0,83 mol Natriumthiomethylat zugegeben und das Reaktionsgemisch 5 Stunden auf 55 bis 60°C erwärmt. Nach Abkühlen wurde 1 l Wasser zugegeben, der Niederschlag abgesaugt und mit 100 ml Methylenchlorid gewaschen. Anschließend wurde der Rückstand in 500 ml 2 N Salzsäure aufgenommen, der sich bildende Niederschlag abgesaugt und mit Wasser gewaschen. Der Rückstand wurde nun in Tetrahydrofuran aufgenommen, über Natriumsulfat getrocknet und das Lösungsmittel abdestilliert.
Ausbeute: 127,6 g (79%) (gelber Feststoff)
(Schmelzpunkt: 245-247°C)
- Stufe 2:: 3-Nitro-4-methylsulfonylbenzoesäure
0,22 mol 3-Nitro-4-thiomethylbenzoesäure wurden zusammen mit 800 ml Eisessig und 5,4 g Na₂WO₄·2 H₂O vorgelegt. Bei einer Temperatur von 55°C wurden 1,32 mol H₂O₂ (30 %ig) zugetropft. Dann 20 Minuten bei 50°C und 2 Stunden bei 70°C gerührt. Man rührte die Reaktionslösung nach Abkühlen in 1 l wasser ein, saugte den Niederschlag ab, wusch den Rückstand mit Wasser und trocknete das Produkt im Vakuum.
Ausbeute: 47,4 g (88%) (weiße Kristalle)
(IR (ν in cm⁻¹): 1699, 1558, 1371, 1322, 1155)
- Stufe 3:: 3-Amino-4-methylsulfonylbenzoesäure
0,447 mol 3-Nitro-4-methylsulfonylbenzoesäure wurden zusammen mit 100 g Raney-Nickel in 2,5 l Methanol mit Wasserstoff reduziert. Danach erhitzte man auf Rückflußtemperatur und saugte heiß ab. Das Filtrat wurde eingeengt.
Ausbeute: 88,1 g (91%)
(¹H-NMR (d₆-DMSO, δ in ppm): 3,18 (3H); 6,25 (2H); 7,21 (1H); 7,48 (1H); 7,72 (1H); 13,8 (1H))
- Stufe 4 :: 8-Methylsulfonyl-5-chinolincarbonsäure
38 ml Wasser und 102 g konz. Schwefelsäure wurden auf 110°C erwärmt. Bei 95°C wurden 0,25 mol 3-Amino-4-methylsulfonylbenzoesäure zugegeben. Danach wurde auf 140°C erhitzt und 0,8 g Natriumiodid und 0,3 mol Glycerin zugegeben. Man erhöhte nun die Reaktionstemperatur auf 150°C. Während des Aufheizens und Nachrührens (1 Stunde) bei 150°C fielen 47 g Destillat an. Nach Abkühlen wurde das Reaktionsgemisch vorsichtig mit 200 ml Wasser versetzt und mit weiteren 800 ml Wasser verdünnt. Anschließend wurde mit 20%iger Natronlague auf pH 13 gestellt, filtriert und mit Schwefelsäure auf pH 3,5 gebracht. Der Prozess wurde wiederholt. Es fiel ein Niederschlag an, der abgesaugt wurde. Das Filtrat wurde auf pH = 2 gebracht und der entstehende Niederschlag abgesaugt, mit Wasser gewaschen und getrocknet.
Ausbeute: 44,9 g (71%)
(¹H-NMR (d₆-DMSO, δ in ppm): 3,70 (3H); 7,82 (1H); 8,40 (1H); 8,68 (1H); 9,32 (1H); 9,66 (1H), 14,01 (1H))

### 8-Bromchinolin-5-carbonsäure (Verbindung 17.05)

- Stufe 1:: 5-Amino-8-bromchinolin
Zu einem Gemisch aus 7,75 g Eisenpulver, 18 ml Eisessig und 9 ml Ethanol wurden bei Rückflußtemperatur 10,0 g 8-Brom-5-nitrochinolin in 68 ml Eisessig und 34 ml Ethanol zugetropft. Nach 45 Minuten Rühren bei Rückflußtemperatur, kühlte man ab und filtrierte über Kieselgur. Das Filtrat wurde eingeengt, in Methylenchlordid aufgenommen, mit Natriumcarbonatlösung gewaschen, getrocknet und eingeengt.
Ausbeute: 7,90 g
(¹H-NMR (CDCl₃; δ in ppm): 4,22 (bs, 2H); 7,71 (m,1H); 7,40 (m,1H); 7,80 (m,1H); 8,18 (m,1H); 9,00 (m,1H))
- Stufe 2:: 8-Brom-5-cyanochinolin
Zu einem Gemisch aus 0,70 g 5-Amino-8-bromchinolin und 3,15 ml Essigsäure tropfte man 0,60 g konz. Salzsäure und rührte 1 Stunde bei Raumtemperatur. Anschließend gab man bei 0-5°C 0,22 g Natriumnitrit in 0,45 ml Wasser zu und rührte 1 Stunde. Nach Zugabe von 20 mg Harnstoff in 0,16 ml Wasser wurde eine weitere Stunde bei 0-5°C gerührt.
Diese Lösung wird zu einem Zweiphasensystem aus Toluol/Kupfer(I)cyanidlösung gegeben, das wie folgt dargestellt wurde. Zu einer Lösung von 1,06 g 10 %iger Ammoniak-Lösung und 0,77 g Natriumcyanid wurde eine Lösung von 0,79 g Kupfer(II)sulfat in 2,2 ml Wasser zugetropft und mit 6 ml Toluol unterschichtet. Nach einer Stunde Rühren bei Raumtemperatur wurde von unlöslichen Bestandteilen abfiltriert und mit Essigsäureethylester extrahiert. Die organische Phase wurde getrocknet und das Lösungsmittel am Vakuum entfernt.
Ausbeute: 0,50 g
(¹-NMR (CDCl₃; δ in ppm): 7,61 (m,1H); 7,76 (m,1H); 8,19 (m,1H); 8,59 (m,1H); 9,17 (m,1H))
- Stufe 3 :: 8-Bromchinolin-5-carbonsäure
Bei 150°C wurden zu 10,10 g 75 %iger Schwefelsäure 5,0 g 8-Brom-5-cyanochinolin portionsweise zugegeben. Nach einer Stunde wurde das Reaktionsgemisch abgekühlt, auf Eiswasser gegossen und mit Essigsäureethylester extrahiert. Die organische Phase wurde getrocknet und eingeengt.
Ausbeute: 3,6 g
(¹H-NMR (d₆-DMSO; δ in ppm): 7,80 (m, 1H); 8,18 (m, 1H); 8,30 (m, 1H); 9,15 (m, 1H); 9,40 (m, 1H))

### 5-Nitro-6-chinolincarbonsäure (Verbindung 18.01)

- Stufe 1:: 5-Nitro-6-methylchinolin
Zu 1 l konzentrierter Schwefelsäure wurden 2,45 mol 6-Methylchinolin zugegeben und bei 0 bis 10°C 2,94 mol 65 %ige Salpetersäure zugetropft. Es wurde eine Stunde nachgerührt, auf Eis gegossen, mit Natronlauge auf pH 2,5 eingestellt, abgesaugt, mit Wasser gewaschen und über Magnesiumsulfat getrocknet.
Ausbeute: 313,0 g farblose Kristalle
(¹H-NMR (CDCl₃; δ in ppm): 2,55 (s, 3H); 7,55 (q, 1H); 7,60 (d, 1H); 8,10 (d, 1H); 8,15 (d, 1H); 8,95 (q, 1H))
- Stufe 2:: 5-Nitrochinolin-6-carbonsäure
Zu 1,3 l Schwefelsäure wurden 20,0 g Vanadiumpentoxid und 0,74 mol 5-Nitro-6-methylchinolin gegeben und bei 140°C mit einer Dosier-Pumpe 200 ml 65 %ige Salpetersäure über 40 Stunden zudosiert. Die Lösung wurde anschließend auf Eis gegossen, mit Natronlauge auf pH 8,0 eingestellt, abgesaugt und über Magnesiumsulfat getrocknet. Man erhielt 81,0 g Edukt zurück. Die Mutterlauge wurde mit Schwefelsäure auf pH 2,5 gestellt, abgesaugt und über Magnesiumsulfat getrocknet.
Ausbeute: 67,0 g farblose Kristalle
(¹H-NMR (d₆-DMSO; δ in ppm): 7,80 (q, 1H); 8,20 (d, 1H); 8,25 (d, 1H); 8,40 (d, 1H); 9,20 (d, 1H))

### 5 -Nitrochinolin-8-carbonsäure (Verbindung 20.03)

- Stufe 1:: 8-Cyano-5-nitrochinolin
5,80 g 8-Brom-5-nitrochinolin und 2,00 g Kupfer(I)cyanid wurden in 15 ml Dimethylformamid 5 Stunden auf 150°C erwärmt. Nach Abkühlen wurde Methylenchlorid zugegeben, unlösliche Bestandteile abfiltriert und das Filtrat eingeengt.
Ausbeute: 3,90 g
(¹H-NMR (CDCl₃; δ in ppm): 7,84 (m, 1H); 8,37 (m, 1H); 8,40 (m, 1H); 9,00 (m, 1H); 9,24 (m, 1H))
- Stufe 2:: 5-Nitrochinolin-8-carbonsäure
Bei 150°C wurden zu 3,50 g 75 %iger Schwefelsäure 1,50 g 8-Cyano-5-nitrochinolin portionsweise zugegeben. Nach einer Stunde Rühren wurde das Reaktionsgemisch abgekühlt, auf Eiswasser gegossen und mit Essigsäureethylester extrahiert. Die organische Phase wurde getrocknet und das Lösungsmittel am Vakuum entfernt.
Ausbeute: 1,1 g
(Schmelzpunkt: 210°C)
(¹H-NMR (d₆-DMSO; δ in ppm): 8,00 (m,1H); 8,49 (m,1H); 8,58 (m,1H); 9,01 (m,1H); 9,22 (m,1H); 15,0 (bs,1H))

### 1,8-Dimethyl-1,2,3,4-tetrahydrochinolin-5-carbonsäure (Verbindung 22.01)

- Stufe 1:: 8-Methyl-1,2,3,4-tetrahydrochinolin-5-carbonsäure 0,1 mol 8-Methylchinolin-5-carbonsäure wurden in 1,5 l Ethanol suspendiert und mit 10,0 g 5 %igem Palladium auf Aktivkohle versetzt. Im Autoklaven wurde bei 50°C mit Wasserstoff (1 bar) innerhalb von 48 Stunden reduziert (HPLC-Kontrolle). Anschließend wurde das Reaktionsgemisch filtriert, der Filterkuchen mit Ethanol gewaschen und die vereinigten organischen Filtrate eingeengt.
Ausbeute: 17,4 g eines gelben Feststoffes .
(¹H-NMR (d₆-DMSO; δ in ppm): 1,75 (m,2H); 2,05 (s,3H); 2,90 (m,2H); 3,25 (m,2H); 5,10 (brs,2H); 6,80 (d,1H); 6,90 (d,1H))
(Schmelzpunkt: 130°C)
- Stufe 2:: 1,8-Dimechyl-1,2,3,4-tetrahydrochinolin-5-carbonsäure
Zu 5 mmol 8-Methyl-1,2,3,4-tetrahydrochinolin-5-carbonsäure und 50 mmol Paraformaldehyd in 30 ml Eisessig wurden 24 mmol Natriumcyanborhydrid gegeben, wobei im Eisbad auf unter 30°C gehalten wurde. Nach 15 Stunden Rühren bei Raumtemperatur wurde auf Eis gegossen und mit Natronlauge auf pH 4 gestellt. Nun wurde mit Essigsäureethylester extrahiert, die organische Phase mit Wasser gewaschen, über Natriumsulfat getrocknet und eingeengt.
Ausbeute: 0,75 g farblose Kristalle
(¹H-NMR (d₆-DMSO; δ in ppm): 1,75 (m,2H); 2,25 (d,3H); 2,65 (s,3H); 3,00 (m,4H); 7,05 (d,1H); 7,30 (d,1H))

### 1-Acetyl-2,3-dihydro-4-chinolon-7-carbonsäure (Verbindung 23.02)

- Stufe 1:: N-(2-Cyanoethyl)-3-aminobenzoesäure
200,0 g 3-Aminobenzoesäure in 2 l Wasser wurden mit 53,2 g Natriumhydroxid versetzt. Bei 30°C wurden 126,6 g Acrylnitril zugetropft und anschließend wurde 22 Stunden auf Rückflußtemperatur erwärmt. Man kühlte dann auf 5°C ab, gab Essisäure zu (pH=5) und saugte den Niederschlag, der mit Wasser gewaschen wurde, ab.
Ausbeute: 266,3 g
(¹H-NMR (d₆-DMSO; δ in ppm): 2,75 (2H); 3,38 (2H); 6,21 (1H); 6,87 (1H); 7,21 (2H); 12,70 (1H))
- Stufe 2:: N-(2-Carboxyethyl)-3-aminobenzoesäure
266,0 g N-(2-Cyanoethyl)-3-aminobenzoesäure wurden zusammen mit 336.0 g Natriumhydroxid in 3 l Wasser 5 Stunden unter Rückfluß erhitzt. Nach Abkühlen stellte man anschließend mit Salzsäure auf pH 3, kühlte und saugte den Niederschlag ab.
Ausbeute: 269,2 g
(Schmelzpunkt: 211°C)
- Stufe 3:: 2,3-Dihydro-4-chinolon-7-carbonsäure
50,0 g der Carbonsäure aus Stufe 2 wurden portionsweise zu 500,0 g Polyphosphorsäure bei 110°C gegeben. Man rührte 1 Stunde nach. Danach gab man den Reaktionsansatz auf Eis, trennte den Niederschlag ab und extrahiert mit Essigsäureethylester. Die organische Phase wurde anschließend getrocknet und eingeengt.
Ausbeute: 9,2 g
(¹H-NMR (d₆-DMSO; δ in ppm) : 2,52 (2H); 3,41 (2H); 7,05 (2H); 7,40 (1H); 7,65 (1H))
- Stufe 4:: 1-Acetyl-2,3-dihydro-4-chinolon-7-carbonsäure
5.0 g 2,3-Dihydro-4-chinolon-7-carbonsäure und 22,5 g Essigsäureanhydrid wurden 1 Stunde auf 100°C erwärmt. Nach dem Abkühlen wurde Wasser zugegeben und mit Methylenchlorid extrahiert. Die organische Phase wurde getrocknet und eingeengt.
Ausbeute: 4,8 g
(Schmelzpunkt: 150°C)

In den folgenden Tabellen 17-24 sind neben den voranstehend beschriebenen Carbonsäuren der Formel IIIb noch weitere aufgeführt, die in analoger Weise hergestellt wurden oder herstellbar sind.

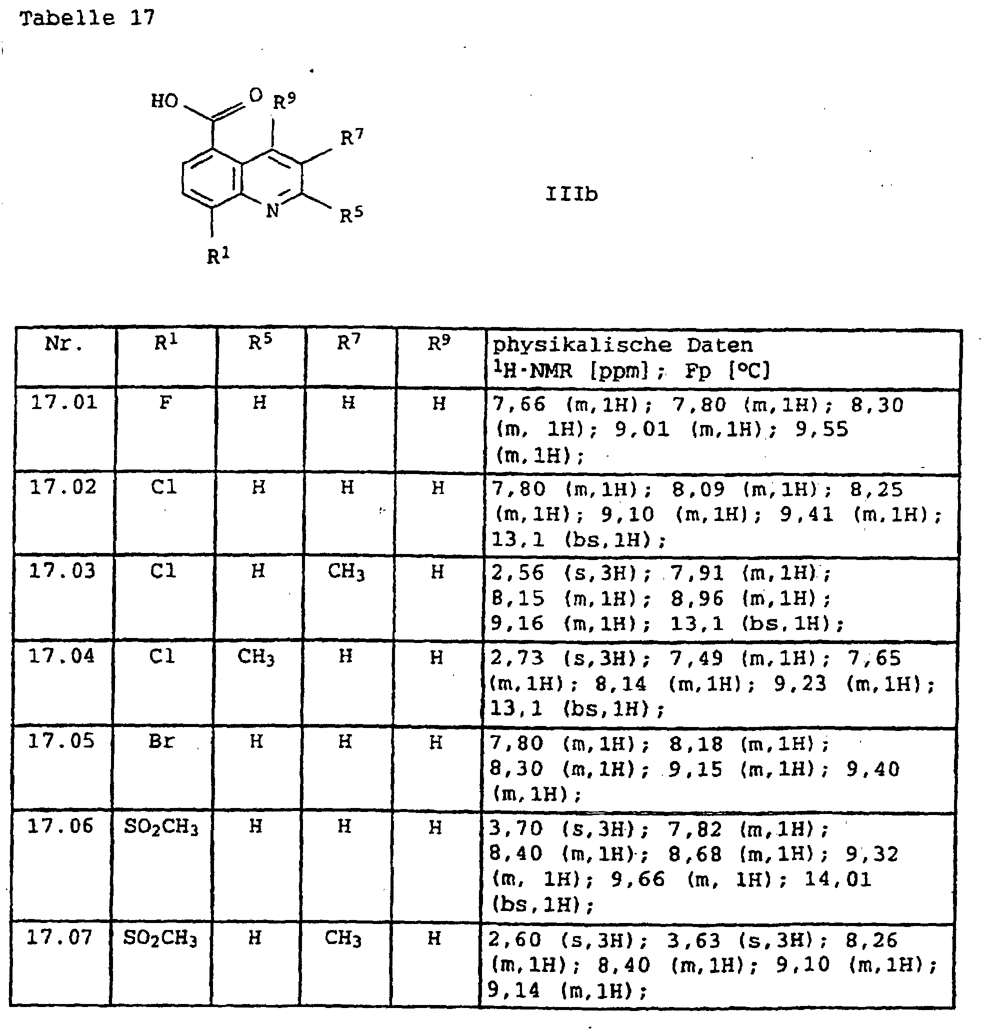

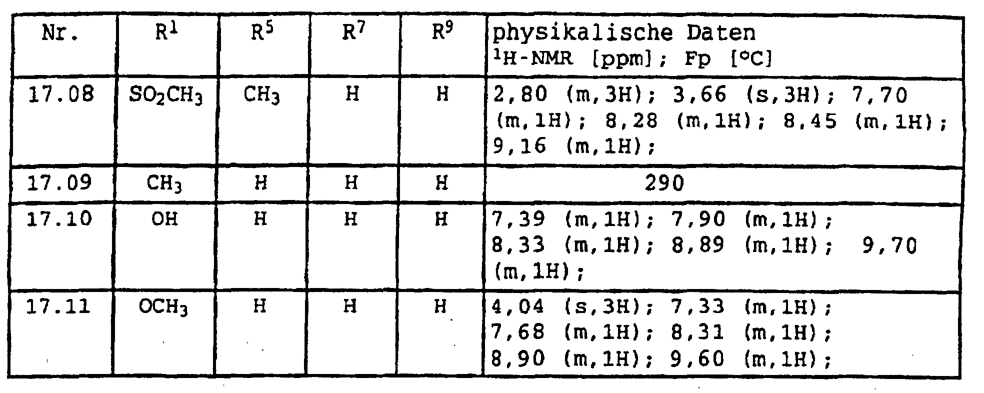

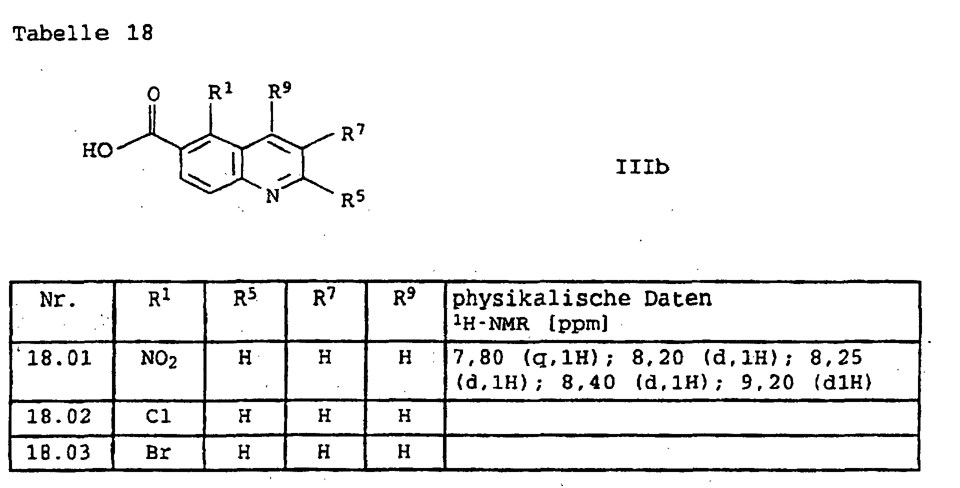

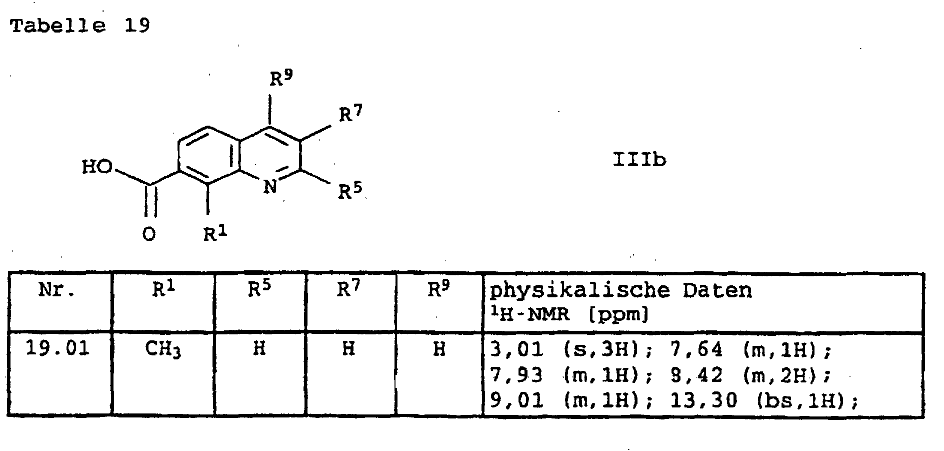

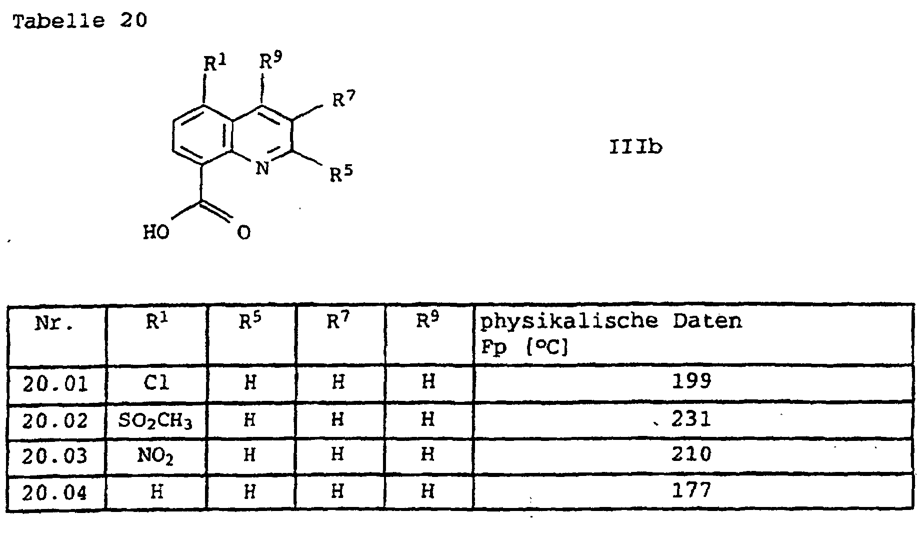

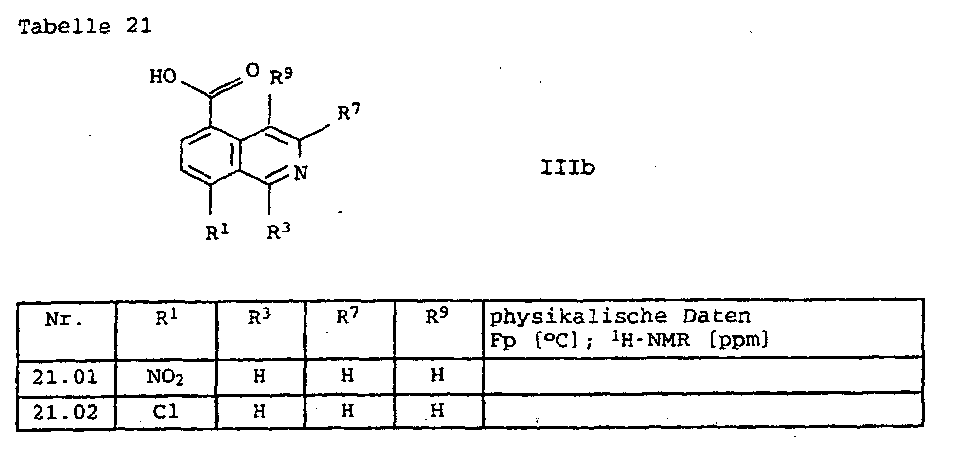

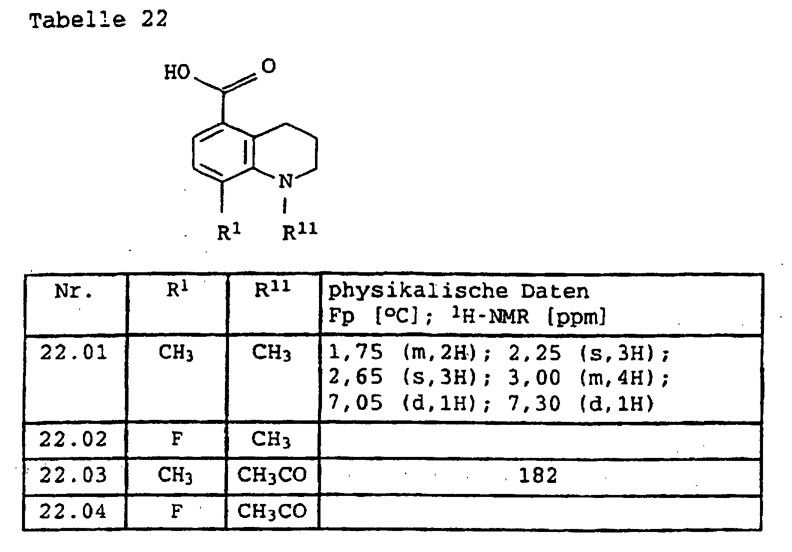

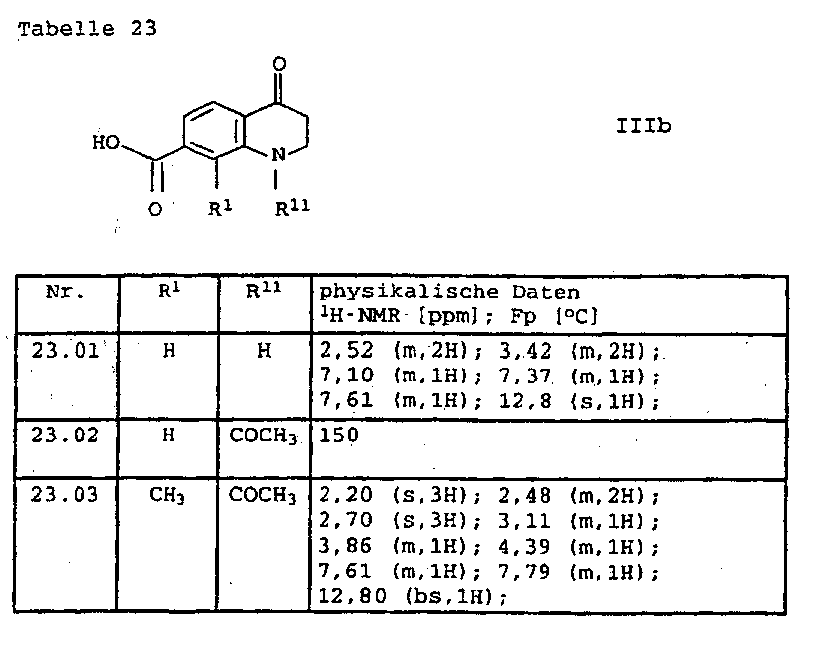

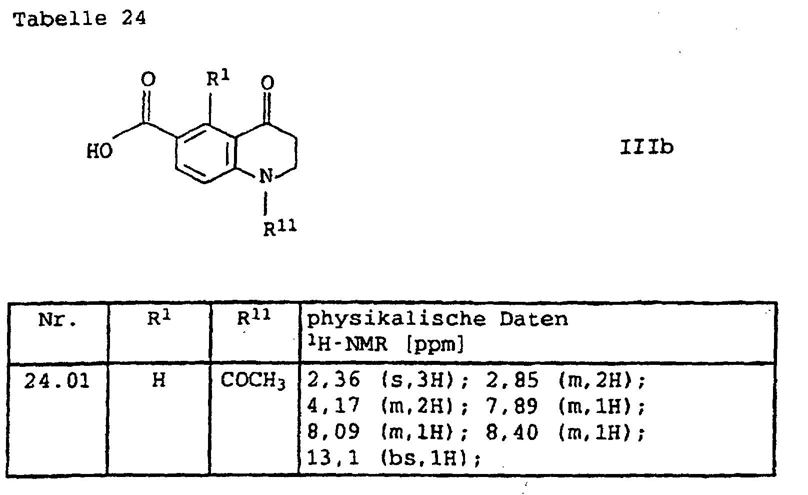

Die Verbindungen der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen. Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus. Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe kommen im Wesentlichen in Betracht:
Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß. Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. 5.02 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. 5.04 werden in einer Mischung gelöst, die aus 40 Gewichtsteilen Cyclohexanon, 30 Gewichtsteilen Isobutanol, 20 Gewichtsteilen des Anlagerüngsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. 5.07 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. 5.11 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile des Wirkstoffs Nr. 7.02 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 8.02 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. 5.20 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. 5.26 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl; BASF AG) besteht. Man erhält ein stabiles Emulsionskonzentrat.

Die Applikation der Wirkstoffe der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff der Formel I betragen je nach Bekämpfungsziel, Jahreszeit, Zielpflanzen und Wachstumsstadium 0,001 bis 3,0, vorzugsweise 0,01 bis 1.0 kg/ha aktive Substanz (a.S.).

Zur Verbreiterung des Wirkungsspektrums und zur Erzielung synergistischer Effekte können die Hetaroylderivate der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate. Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der Hetaroylderivate der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,5 oder 0,25 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Chenopodium album | Weißer Gänsefuß | lambsquarters (goosefoot) |
| Echinochloa crus-galli | Hühnerhirse | barnyardgrass |
| Polygonum persicaria | Flohknöterich | ladysthumb |
| Setaria faberii | Borstenhirse | giant foxtail |
| Solanum nigrum | Schwarzer Nachtschatten | black nightshade |
| Zea mays | Mais | Indian corn |

Bei Aufwandmengen von 0,5 bzw. 0,25 kg/ha zeigte die Verbindung 5.12 (Tabelle 5) im Nachauflauf eine sehr gute Wirkung gegen die oben genannten mono- und dicotylen Schadpflanzen und gute Verträglichkeit in Mais.

## Patentansprüche

1. Hetaroylderivate der Formel I 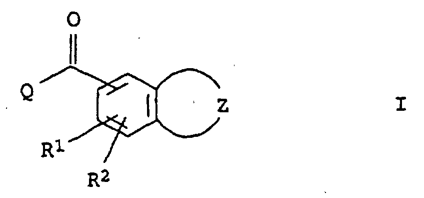 in der die Variablen folgende Bedeutung haben:
R¹, R² Wasserstoff, Nitro, Halogen, Cyano, Rhodano, Hydroxy, Mercapto, C₁-C₆-Alkyl, C₁-C₆-Halogenalkyl, C₂-C₆-Alkenyl, C₂-C₆-Alkinyl, C₁-C₆-Alkoxy, C₁-C₆-Halogenalkoxy, C₂-C₆-Alkenyloxy, C₂-C₆-Alkinyloxy, C₁-C₆-Alkylthio, C₁-C₆-Halogenalkylthio, C₂-C₆-Alkenylthio, C₂-C₆-Alkinylthio, C₁-C₆-Alkylsulfinyl, C₁-C₆-Halogenalkylsulfinyl, C₂-C₆-Alkenylsulfinyl, C₂-C₆-Alkinylsulfinyl, C₁-C₆-Alkylsulfonyl, C₁-C₆-Halogenalkylsulfonyl, C₂-C₆-Alkenylsulfonyl, C₂-C₆-Alkinylsulfonyl, C₁-C₆-Alkoxysulfonyl, C₁-C₆-Halogenalkoxysulfonyl, C₂-C₆-Alkenyloxysulfonyl, C₂-C₆-Alkinyloxysulfonyl, Phenyl, Phenyloxy, Phenylthio, Phenylsulfinyl oder Phenylsulfonyl, wobei die fünf letztgenannten Substituenten partiell oder vollständig halogeniert sein können und ein bis drei der folgenden Gruppen tragen können:
Nitro, Cyano, Hydroxy, C₁-C₄-Alkyl, C₁-C₄-Halogenalkyl, C₁-C₄-Alkoxy, C₁-C₄-Halogenalkoxy;
Z ein Baustein aus der Gruppe Z¹ bis Z¹² 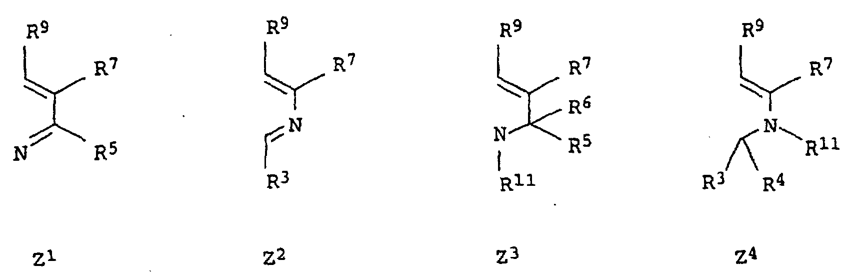 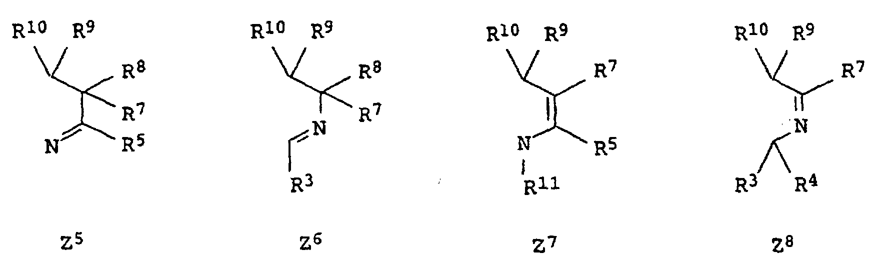 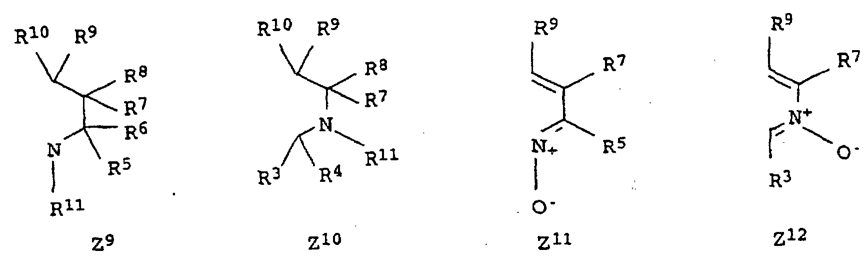 wobei
R³, R⁵, R⁷, R⁹ Nitro, Cyano, Hydroxy, Mercapto, C₂-C₄-Alkenyl, C₂-C₄-Alkinyl, C₂-C₄-Alkenyloxy, C₂-C₄-Alkinyloxy, C₂-C₄-Alkenylthio, C₂-C₄-Alkinylthio, C₁-C₄-Alkylsulfinyl, C₁-C₄-Halogenalkylsulfinyl, C₂-C₄-Alkenylsulfinyl, C₂-C₄-Alkinylsulfinyl, C₁-C₄-Alkylsulfonyl, C₁-C₄-Halogenalkylsulfonyl, C₂-C₄-Alkenylsulfonyl, C₂-C₄-Alkinylsulfonyl, C₁-C₄-Alkoxysulfonyl, C₁-C₄-Halogenalkoxysulfonyl, C₂-C₄-Alkenyloxysulfonyl, C₂-C₄-Alkinyloxysulfonyl, -NR¹²R¹³, -CO₂R¹², -CONR¹²R¹³, Phenyl, Phenoxy, Phenylthio, Phenylsulfinyl oder Phenylsulfonyl bedeuten, wobei die fünf letztgenannten Substituenten partiell oder vollständig halogeniert sein können und ein bis drei der folgenden Gruppen tragen können:
Nitro, Cyano, Hydroxy, C₁-C₄-Alkyl, C₁-C₄-Halogenalkyl, C₁-C₄-Alkoxy, C₁-C₄-Halogenalkoxy;
sowie die unter R⁴ genannten Reste;
R⁴, R^{6,} R⁸, R¹⁰ Wasserstoff, Halogen, C₁-C₄-Alkyl, C₁-C₄-Halogenalkyl, C₁-C₄-Alkoxy, C₁-C₄-Halogenalkoxy, C₁-C₄-Alkylthio oder C₁-C₄-Halogenalkylthio bedeuten;
oder
eine -CR³R⁴-, -CR⁵R⁶-, -CR⁷R⁸-, -CR⁹R¹⁰-Einheit durch C=O oder C=NR¹³ ersetzt sein kann;
R¹¹ Wasserstoff, C₁-C₆-Alkyl, C₁-C₆-Halogenalkyl, C₃-C₆-Alkenyl, C₃-C₆-Alkinyl, C₁-C₆-Alkylcarbonyl, C₁-C₆-Halogenalkylcarbonyl, -CO₂R¹², -CONR¹²R¹³ oder SO₂R¹² bedeutet;
R¹² Wasserstoff, C₁-C₆-Alkyl, C₁-C₆-Halogenalkyl, C₃-C₆-Alkenyl, C₃-C₆-Alkinyl oder Phenyl bedeutet, wobei der letztgenannte Rest partiell oder vollständig halogeniert sein kann und ein bis drei der folgenden Reste tragen kann:
Nitro, Cyano, Hydroxy, C₁-C₄-Alkyl, C₁-C₄-Halogenalkyl, C₁-C₄-Alkoxy, C₁-C₄-Halogenalkoxy;
R¹³ C₁-C₆-Alkoxy, C₁-C₆-Halogenalkoxy, C₃-C₆-Alkenyloxy, C₃-C₆-Alkinyloxy oder ein unter R¹² genannter Rest bedeutet;
Q ein in 2-Stellung verknüpfter Cyclohexan-1,3-dionring der Formel II 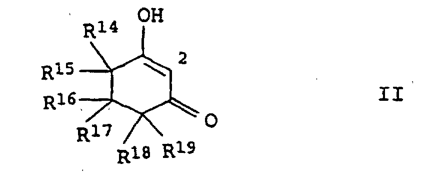
wobei
R¹⁴, R¹⁵, R¹⁷ und R¹⁹ für Wasserstoff oder C₁-C₄-Alkyl stehen;
R¹⁶ für Wasserstoff, C₁-C₄-Alkyl oder C₃-C₄-Cycloalkyl steht, wobei die beiden letztgenannten Gruppen gegebenenfalls einen bis drei der folgenden Substituenten tragen können:
Halogen, C₁-C₄-Alkylthio oder C₁-C₄-Alkoxy;
oder
für Tetrahydropyran-3-yl, Tetrahydropyran-4-yl, Tetrahydrothiopyran-3-yl, 1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die 6 letztgenannten Reste gegebenenfalls durch ein bis drei C₁-C₄-Alkylreste substituiert sein können;
R¹⁸ für Wasserstoff, C₁-C₄-Alkyl oder C₁-C₆-Alkoxycarbonyl steht;
oder
R¹⁶ und R¹⁹ gemeinsam eine Bindung oder einen drei- bis sechsgliedrigen carbocyclischen Ring bilden ;
oder
die -CR¹⁶R¹⁷-Einheit durch C=O ersetzt sein kann,
sowie deren landwirtschaftlich brauchbaren Salze.

2. Hetaroylderivate der Formel I nach Anspruch 1, wobei CR⁹R¹⁰ nicht C=O oder C=NR¹³ bedeutet, wenn Z gleich Z⁹ ist und das "QCO-Fragment" para zum Stickstoff des Bausteines Z steht.

3. Hetaroylderivate der Formel I nach Ansprüchen 1 bis 2, in der die Variablen folgende Bedeutung haben:
R¹, R² Wasserstoff, Nitro, Halogen, Cyano, Rhodano, Hydroxy, Mercapto, C₁-C₆-Alkyl, C₁-C₆-Halogenalkyl, C₂-C₆-Alkenyl, C₂-C₆-Alkinyl, C₁-C₆-Alkoxy, C₁-C₆-Halogenalkoxy, C₂-C₆-Alkenyloxy, C₂-C₆-Alkinyloxy, C₁-C₆-Alkylthio, C₁-C₆-Halogenalkylthio, C₂-C₆-Alkenylthio, C₂-C₆-Alkinylthio, C₁-C₆-Alkylsulfinyl, C₁-C₆-Halogenalkylsulfinyl, C₂-C₆-Alkenylsulfinyl, C₂-C₆-Alkinylsulfinyl, C₁-C₆-Alkylsulfonyl, C₁-C₆-Halogenalkylsulfonyl, C₂-C₆-Alkenylsulfonyl, C₂-C₆-Alkinylsulfonyl, C₁-C₆-Alkoxysulfonyl, C₁-C₆-Halogenalkoxysulfonyl, C₂-C₆-Alkenyloxysulfonyl, C₂-C₆-Alkinyloxysulfonyl, Phenyl, Phenyloxy, Phenylthio, Phenylsulfinyl oder Phenylsulfonyl, wobei die fünf letztgenannten Substituenten partiell oder vollständig halogeniert sein können und ein bis drei der folgenden Gruppen tragen können:
Nitro, Cyano, Hydroxy, C₁-C₄-Alkyl, C₁-C₄-Halogenalkyl, C₁-C₄-Alkoxy, C₁-C₄-Halogenalkoxy;
Z ein Baustein aus der Gruppe Z¹ bis Z¹² 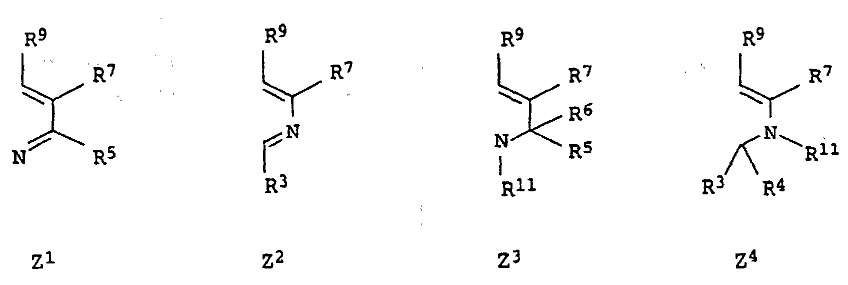 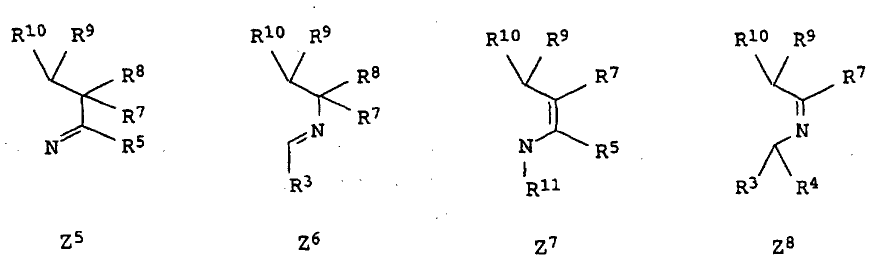 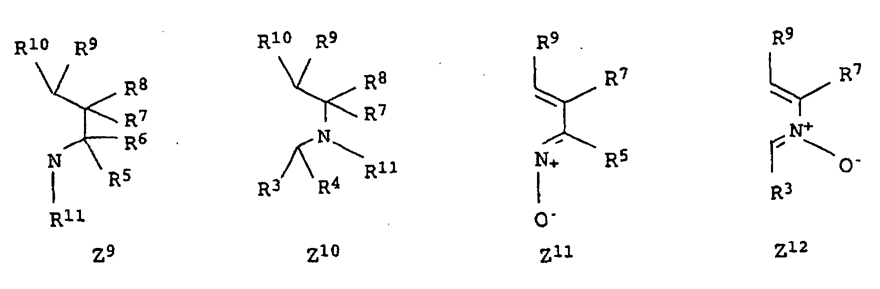 wobei
R³, R⁵, R⁷, R⁹ Wasserstoff, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Nitro, Cyano, Hydroxy, Mercapto, C₂-C₄-Alkenyl, C₂-C₄-Alkinyl, C₂-C₄-Alkenyloxy, C₂-C₄-Alkinyloxy, C₂-C₄-Alkenylthio, C₂-C₄-Alkinylthio, C₁-C₄-Alkylsulfinyl, C₁-C₄-Halogenalkylsulfinyl, C₂-C₄-Alkenylsulfinyl, C₂-C₄-Alkinylsulfinyl, C₁-C₄-Alkylsulfonyl, C₁-C₄-Halogenalkylsulfonyl, C₂-C₄-Alkenylsulfonyl, C₂-C₄-Alkinylsulfonyl, C₁-C₄-Alkoxysulfonyl, C₁-C₄-Halogenalkoxysulfonyl, C₂-C₄-Alkenyloxysulfonyl, C₂-C₄-Alkinyloxysulfonyl, -NR¹²R¹³, -CO₂R¹², -CONR¹²R¹³, Phenyl, Phenoxy, Phenylthio, Phenylsulfinyl oder Phenylsulfonyl bedeuten, wobei die fünf letztgenannten Substituenten partiell oder vollständig halogeniert sein können und ein bis drei der folgenden Gruppen tragen können:
Nitro, Cyano, Hydroxy, C₁-C₄-Alkyl, C₁-C₄-Halogenalkyl, C₁-C₄-Alkoxy, C₁-C₄-Halogenalkoxy;
R⁴, R^{6,} R⁸, R¹⁰ Wasserstoff;
R¹¹ Wasserstoff, C₁-C₆-Alkyl, C₁-C₆-Halogenalkyl, C₃-C₆-Alkenyl, C₃-C₆-Alkinyl, C₁-C₆-Alkylcarbonyl, C₁-C₆-Halogenalkylcarbonyl, -CO₂R¹², -CONR¹²R¹³ oder SO₂R¹² bedeutet;
R¹² Wasserstoff, C₁-C₆-Alkyl, C₁-C₆-Halogenalkyl, C₃-C₆-Alkenyl, C₃-C₆-Alkinyl oder Phenyl bedeutet, wobei der letztgenannte Rest partiell oder vollständig halogeniert sein kann und ein bis drei der folgenden Reste tragen kann:
Nitro, Cyano, Hydroxy, C₁-C₄-Alkyl, C₁-C₄-Halogenalkyl, C₁-C₄-Alkoxy, C₁-C₄-Halogenalkoxy;
R¹³ C₁-C₆-Alkoxy, C₁-C₆-Halogenalkoxy, C₃-C₆-Alkenyloxy, C₃-C₆-Alkinyloxy oder ein unter R¹² genannter Rest bedeutet;

4. Hetaroylderivate der Formel I nach den Ansprüchen 1 bis 3, wobei die Variable Z die Bedeutung Z¹, Z², Z¹¹ oder Z¹² hat.

5. Hetaroylderivate der Formel I, nach den Ansprüchen 1 bis 3, wobei die Variable Z die Bedeutung Z³, Z⁴, Z⁵, Z⁶, Z⁷ oder Z⁸ hat.

6. Hetaroylderivate der Formel I, nach den Ansprüchen 1 bis 3, wobei die Variable Z die Bedeutung Z⁹ oder Z¹⁰ hat.

7. Hetaroylderivate der Formel I, nach den Ansprüchen 1 bis 6, in der
R¹ Nitro, Halogen, Cyano, Rhodano, Hydroxy, Mercapto, C₁-C₆-Alkyl, C₁-C₆-Halogenalkyl, C₂-C₆-Alkenyl, C₂-C₆-Alkinyl, C₁-C₆-Alkoxy, C₁-C₆-Halogenalkoxy, C₂-C₆-Alkenyloxy, C₂-C₆-Alkinyloxy, C₁-C₆-Alkylthio, C₁-C₆-Halogenalkylthio, C₂-C₆-Alkenylthio, C₂-C₆-Alkinylthio, C₁-C₆-Alkylsulfinyl, C₁-C₆-Halogenalkylsulfinyl, C₂-C₆-Alkenylsulfinyl, C₂-C₆-Alkinylsulfinyl, C₁-C₆-Alkyl- sulfonyl, C₁-C₆-Halogenalkylsulfonyl, C₂-C₆-Alkenylsulfonyl, C₂-C₆-Alkinylsulfonyl, C₁-C₆-Alkoxysulfonyl, C₁-C₆-Halogenalkoxysulfonyl, C₂-C₆-Alkenyloxysulfonyl, C₂-C₆-Alkinyloxysulfonyl, Phenyl, Phenyloxy, Phenylthio, Phenylsulfinyl oder Phenylsulfonyl, wobei die fünf letztgenannten Substituenten partiell oder vollständig halogeniert sein können und ein bis drei der folgenden Gruppen tragen können:
Nitro, Cyano, Hydroxy, C₁-C₄-Alkyl, C₁-C₄-Halogenalkyl, C₁-C₄-Alkoxy, C₁-C₄-Halogenalkoxy bedeuten, und
R² für Wasserstoff oder einen unter R¹ genannten Rest steht.

8. Hetaroylderivate der Formel Ia, nach den Ansprüchen 1 bis 4, sowie deren N-Oxide (Formel Ia')

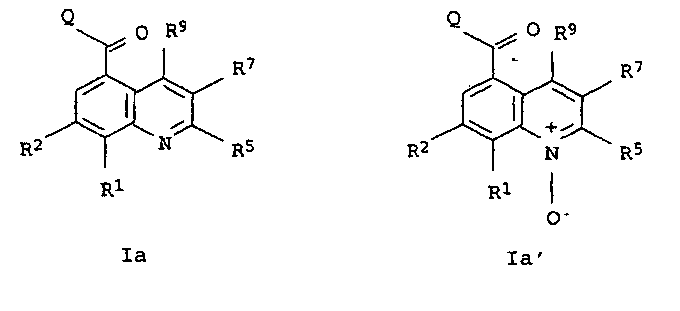

9. Hetaroylderivate der Formel Ib, nach den Ansprüchen 1 bis 4, sowie deren N-Oxide (Formel Ib')

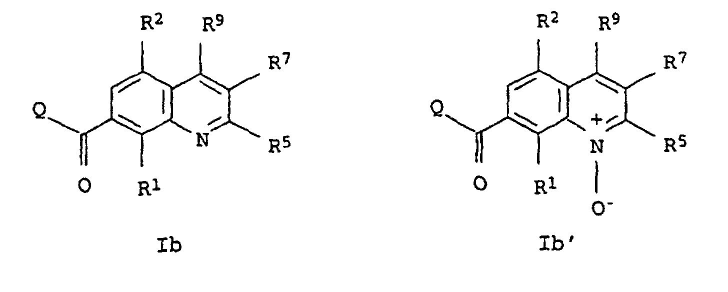

10. Hetaroylderivate der Formel Ic, nach den Ansprüchen 1 bis 4, sowie deren N-Oxide (Formel Ic')

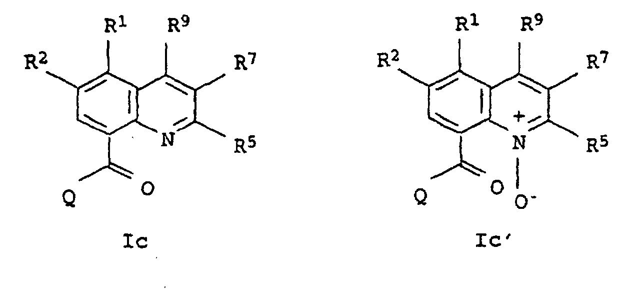

11. Hetaroylderivate der Formel Id, nach den Ansprüchen 1 bis 4, sowie deren N-Oxide (Formel Id')

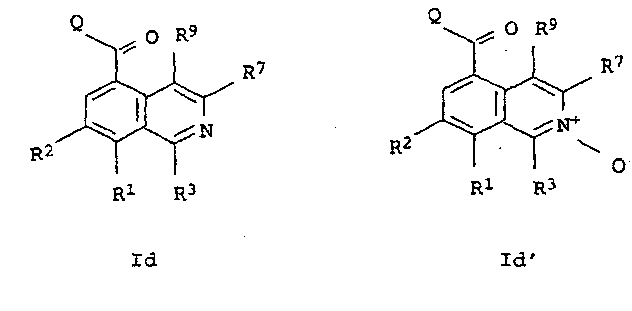

12. Hetaroylderivate der Formel le, nach den Ansprüchen 1 bis 4, sowie deren N-Oxide (Formel Ie')

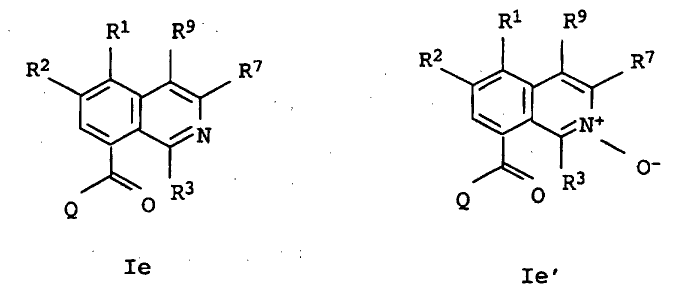

13. Hetaroylderivate der Formel If (mit Z = Z⁹) oder Ig (mit Z = Z¹⁰), nach den Ansprüchen 1 bis 3 oder 6,

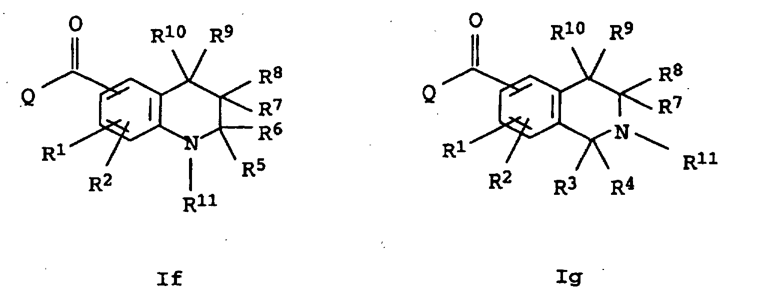

14. Verfahren zur Herstellung von Verbindungen der Formel I gemäß den Ansprüchen 1 bis 13, **dadurch gekennzeichnet, daß** man ein Cyclohexan-1,3-dion der Formel II mit einer aktivierten Carbonsäure IIIa oder mit einer Carbonsäure IIIb, 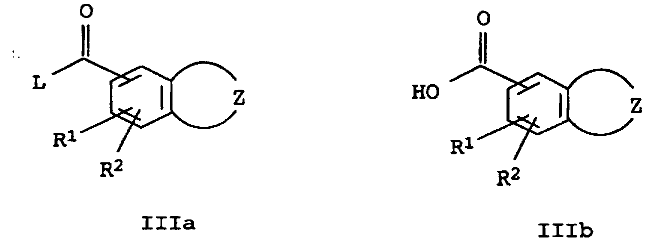 wobei die Variablen R¹, R², R¹⁴ bis R¹⁹ und Z die unter Anspruch 1 genannte Bedeutung haben und L für eine nucleophil verdrängbare Abgangsgruppe steht, acyliert und das Acylierungsprodukt in Gegenwart eines Katalysators zu den Verbindungen I umlagert.

15. Herbizides Mittel, enthaltend eine herbizid wirksame Menge mindestens eines Hetaroylderivates der Formel I oder eines landwirtschaftlich brauchbaren Salzes von I, gemäß den Ansprüchen 1 bis 13, und mindestens einen inerten flüssigen und/oder festen Trägerstoff sowie gewünschtenfalls mindestens einen oberflächenaktiven Stoff.

16. Verfahren zur Herstellung von herbizid wirksamen Mitteln gemäß Anspruch 15, **dadurch gekennzeichnet, daß** man eine herbizid wirksame Menge mindestens eines Hetaroylderivats der Formel I oder eines landwirtschaftlich brauchbaren Salzes von I, gemäß den Ansprüchen 1 bis 13 und mindestens einen inerten flüssigen und/oder festen Trägerstoff sowie gewünschtenfalls mindestens einen oberflächenaktiven Stoff mischt.

17. Verfahren zur Bekämpfung von unerwünschtem Pflanzenwuchs, **dadurch gekennzeichnet, daß** man eine herbizid wirksame Menge mindestens eines Hetaroylderivats der Formel I oder eines landwirtschaftlich brauchbaren Salzes von I, gemäß den Ansprüchen 1 bis 13, auf Pflanzen, deren Lebensraum oder auf Samen einwirken läßt.

18. Verwendung der Hetaroylderviate der Formel I und deren landwirtschaftlich brauchbaren Salze gemäß den Ansprüchen 1 bis 13 als Herbizide.

## Claims

1. A hetaroyl derivative of the formula I 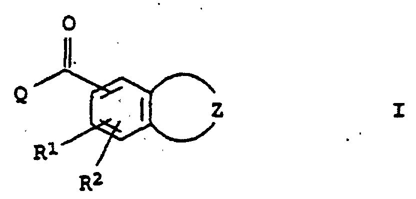 in which the variables have the following meanings:
R¹, R² are hydrogen, nitro, halogen, cyano, thiocyanato, hydroxyl, mercapto, C₁-C₆-alkyl, C₁-C₆-haloalkyl, C₂-C₆-alkenyl, C₂-C₆-alkynyl, C₁-C₆-alkoxy, C₁-C₆-haloalkoxy, C₂-C₆-alkenyloxy, C₂-C₆-alkynyloxy, C₁-C₆-alkylthio, C₁-C₆-haloalkylthio, C₂-C₆-alkenylthio, C₂-C₆-alkynylthio, C₁-C₆-alkylsulfinyl, C₁-C₆-haloalkylsulfinyl, C₂-C₆-alkenylsulfinyl, C₂-C₆-alkynylsulfinyl, C₁-C₆-alkylsulfonyl, C₁-C₆-haloalkylsulfonyl, C₂-C₆-alkenylsulfonyl, C₂-C₆-alkynylsulfonyl, C₁-C₆-alkoxysulfonyl, C₁-C₆-haloalkoxysulfonyl, C₂-C₆-alkenyloxysulfonyl, C₂-C₆-alkynyloxysulfonyl, phenyl, phenyloxy, phenylthio, phenylsulfinyl or phenylsulfonyl, where the five last mentioned substituents may be partially or fully halogenated and may carry one to three of the following groups:
nitro, cyano, hydroxyl, C₁-C₄-alkyl, C₁-C₄-haloalkyl, C₁-C₄-alkoxy, C₁-C₄-haloalkoxy;
Z is a building block selected from the group consisting of Z¹ to Z¹² 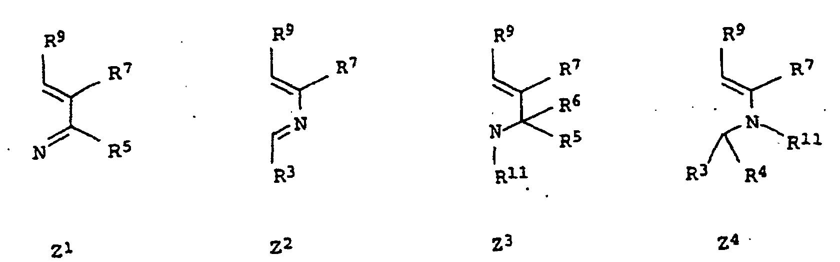 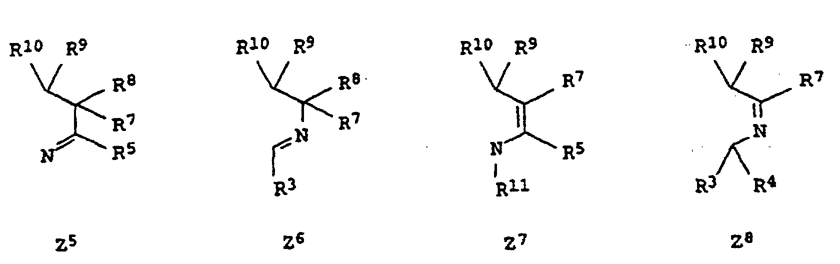 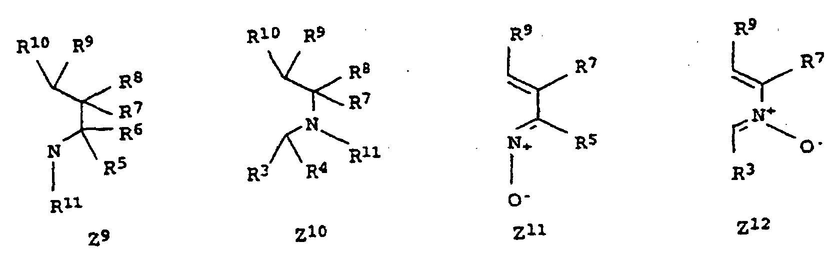
where
R³, R⁵, R⁷, R⁹ are nitro, cyano, hydroxyl, mercapto, C₂-C₄-alkenyl, C₂-C₄-alkynyl, C₂-C₄-alkenyloxy, C₂-C₄-alkynyloxy, C₂-C₄-alkenylthio, C₂-C₄-alkynylthio, C₁-C₄-alkylsulfinyl, C₁-C₄-haloalkylsulfinyl, C₂-C₄-alkenylsulfinyl, C₂-C₄-alkynylsulfinyl, C₁-C₄-alkylsulfonyl, C₁-C₄-haloalkylsulfonyl, C₂-C₄-alkenylsulfonyl, C₂-C₄-alkynylsulfonyl, C₁-C₄-alkoxysulfonyl, C₁-C₄-haloalkoxy-sulfonyl, C₂-C₄-alkenyloxysulfonyl, C₂-C₄-alkynyloxysulfonyl, -NR¹²R¹³, -CO₂R¹², -CONR¹²R¹³, phenyl, phenoxy, phenylthio, phenylsulfinyl or phenylsulfonyl, where the five last mentioned substituents may be partially or fully halogenated and may carry one to three of the following groups:
nitro, cyano, hydroxyl, C₁-C₄-alkyl, C₁-C₄-haloalkyl, C₁-C₄-alkoxy, C₁-C₄-haloalkoxy;
and the radicals mentioned under R⁴;
R⁴, R⁶, R⁸, R¹⁰ are hydrogen, halogen, C₁-C₄-alkyl, C₁-C₄-haloalkyl, C₁-C₄-alkoxy, C₁-C₄-haloalkoxy, C₁-C₄-alkylthio or C₁-C₄-haloalkylthio;
or
a -CR³R⁴-, -CR⁵R⁶-, -CR⁷R⁸-, -CR⁹R¹⁰- unit may be replaced by C=O or C=NR¹³;
R¹¹ is hydrogen, C₁-C₆-alkyl, C₁-C₆-haloalkyl, C₃-C₆-alkenyl, C₃-C₆-alkynyl, C₁-C₆-alkylcarbonyl, C₁-C₆-haloalkylcarbonyl, -CO₂R¹², -CONR¹²R¹³ or SO₂R¹²;
R¹² is hydrogen, C₁-C₆-alkyl, C₁-C₆-haloalkyl, C₃-C₆-alkenyl, C₃-C₆-alkynyl or phenyl, where the last mentioned radical may be partially or fully halogenated and may carry one to three of the following radicals:
nitro, cyano, hydroxyl, C₁-C₄-alkyl, C₁-C₄-haloalkyl, C₁-C₄-alkoxy, C₁-C₄-haloalkoxy;
R¹³ is C₁-C₆-alkoxy, C₁-C₆-haloalkoxy, C₃-C₆-alkenyloxy, C₃-C₆-alkynyloxy or a radical mentioned under R₁₂;
Q is a cyclohexane-1,3-dione ring, attached in the 2-position, of the formula II, 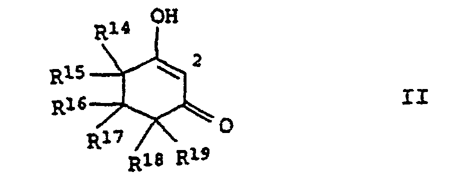
where
R¹⁴, R¹⁵, R¹⁷ and R¹⁹ are hydrogen or C₁-C₄-alkyl;
R¹⁶ is hydrogen, C₁-C₄-alkyl or C₃-C₄-cycloalkyl, where the two last mentioned groups may optionally carry one to three of the following substituents;
halogen, C₁-C₄-alkylthio or C₁-C₄-alkoxy;
or
is tetrahydropyran-3-yl, tetrahydropyran-4-yl, tetrahydrothiopyran-3-yl, 1,3-dioxolan-2-yl, 1,3-dioxan-2-yl, 1,3-oxathiolan-2-yl, 1,3-oxathian-2-yl, 1,3-dithiolan-2-yl or 1,3-dithian-2-yl, where the 6 last mentioned radicals may optionally be substituted by one to three C₁-C₄-alkyl radicals;
R₁₈ is hydrogen, C₁-C₄-alkyl or C₁-C₆-alkoxycarbonyl;
or
R¹⁶ and R¹⁹ together form a bond or a three- to six- membered carbocyclic ring;
or
the -CR¹⁶R¹⁷ unit may be replaced by C=O,
and its agriculturally useful salts.

2. A hetaroyl derivative of the formula I as claimed in claim 1, where CR⁹R¹⁰ is not C=O or C=NR¹³ if Z is Z⁹ and the "QCO fragment" is located para to the nitrogen of building block Z.

3. A hetaroyl derivative of the formula I as claimed in claim 1 or 2, in which the variables have the following meanings:
R¹, R² are hydrogen, nitro, halogen, cyano, thiocyanato, hydroxyl, mercapto, C₁-C₆-alkyl, C₁-C₆-haloalkyl, C₂-C₆-alkenyl, C₂-C₆-alkynyl, C₁-C₆-alkoxy, C₁-C₆-haloalkoxy, C₂-C₆-alkenyloxy, C₂-C₆-alkynyloxy, C₁-C₆-alkylthio, C₁-C₆-haloalkylthio, C₂-C₆-alkenylthio, C₂-C₆-alkynylthio, C₁-C₆-alkylsulfinyl, C₁-C₆-haloalkylsulfinyl, C₂-C₆-alkenylsulfinyl, C₂-C₆-alkynylsulfinyl, C₁-C₆-alkylsulfonyl, C₁-C₆-haloalkylsulfonyl, C₂-C₆-alkenylsulfonyl, C₂-C₆-alkynylsulfonyl, C₁-C₆-alkoxysulfonyl, C₁-C₆-haloalkoxysulfonyl, C₂-C₆-alkenyloxysulfonyl, C₂-C₆alkynyloxysulfonyl, phenyl, phenyloxy, phenylthio, phenylsulfinyl or phenylsulfonyl, where the five last mentioned substituents may be partially or fully halogenated and may carry one to three of the following groups:
nitro, cyano, hydroxyl, C₁-C₄-alkyl, C₁-C₄-haloalkyl, C₁-C₄-alkoxy, C₁-C₄-haloalkoxy;
Z is a building block selected from the group Z¹ to Z¹² 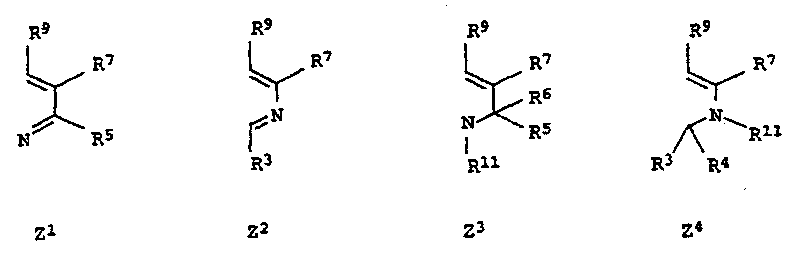 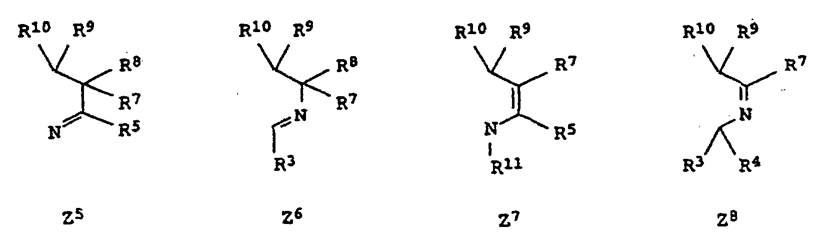 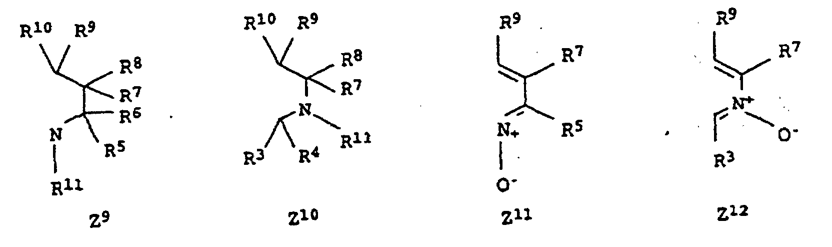
where
R³, R⁵, R⁷,R⁹ are hydrogen, halogen, C₁-C₄-alkyl, C₁-C₄-haloalkyl, C₁-C₄-alkoxy, C₁-C₄-haloalkoxy, C₁-C₄-alkylthio, C₁-C₄-haloalkylthio, nitro, cyano, hydroxyl, mercapto, C₂-C₄-alkenyl, C₂-C₄-alkynyl, C₂-C₄-alkenyloxy, C₂-C₄-alkynyloxy, C₂-C₄-alkenylthio, C₂-C₄-alkynylthio, C₁-C₄-alkylsulfinyl, C₁-C₄-haloalkylsulfinyl, C₂-C₄-alkenylsulfinyl, C₂-C₄-alkynylsulfinyl, C₁-C₄-alkylsulfonyl, C₁-C₄-haloalkylsulfonyl, C₂-C₄-alkenylsulfonyl, C₂-C₄-alkynylsulfonyl, C₁-C₄-alkoxysulfonyl, C₁-C₄-haloalkoxysulfonyl, C₂-C₄-alkenyloxysulfonyl, C₂-C₄-alkynyloxysulfonyl, -NR¹²R¹³, -CO₂R¹², -CONR¹²R¹³, phenyl, phenoxy, phenylthio, phenylsulfinyl or phenylsulfonyl, where the five last mentioned substituents may be partially or fully halogenated and may carry one to three of the following groups:
nitro, cyano, hydroxyl, C₁-C₄-alkyl, C₁-C₄-haloalkyl, C₁-C₄-alkoxy, C₁-C₄-haloalkoxy;
R⁴, R⁶, R⁸, R¹⁰ are hydrogen;
R¹¹ is hydrogen, C₁-C₆-alkyl, C₁-C₆-haloalkyl, C₃-C₆-alkenyl, C₃-C₆-alkynyl, C₁-C₆-alkylcarbonyl, C₁-C₆-haloalkylcarbonyl, -CO₂R¹², -CONR¹²R¹³ or SO₂R¹²;
R¹² is hydrogen, C₁-C₆-alkyl, C₁-C₆-haloalkyl, C₃-C₆-alkenyl, C₃-C₆-alkynyl or phenyl, where the last mentioned radical may be partially or fully halogenated and may carry one to three of the following radicals:
nitro, cyano, hydroxyl, C₁-C₄-alkyl, C₁-C₄-haloalkyl, C₁-C₄-alkoxy, C₁-C₄-haloalkoxy;
R¹³ is C₁-C₆-alkoxy, C₁-C₆-haloalkoxy, C₃-C₆-alkenyloxy, C₃-C₆-alkynyloxy or a radical mentioned under R₁₂.

4. A hetaroyl derivative of the formula I as claimed in any of claims 1 to 3 where the variable Z is Z¹, Z², Z¹¹ or Z¹².

5. A hetaroyl derivative of the formula I as claimed in any of claims 1 to 3 where the variable Z is Z³, Z⁴, Z⁵, Z⁶, Z⁷ or Z⁸.

6. A hetaroyl derivative of the formula I as claimed in any of claims 1 to 3 where the variable Z is Z⁹ or Z¹⁰.

7. A hetaroyl derivative of the formula I as claimed in any of claims 1 to 6 where
R¹ is nitro, halogen, cyano, thiocyanato, hydroxyl mercapto, C₁-C₆-alkyl, C₁-C₆-haloalkyl, C₂-C₆-alkenyl, C₂-C₆-alkynyl, C₁-C₆-alkoxy, C₁-C₆-haloalkoxy, C₂-C₆-alkenyloxy, C₂-C₆-alkynyloxy, C₁-C₆-alkylthio, C₁-C₆-haloalkylthio, C₂-C₆-alkenylthio, C₂-C₆-alkynylthio, C₁-C₆-alkylsulfinyl, C₁-C₆-haloalkylsulfinyl, C₂-C₆-alkenylsulfinyl, C₂-C₆-alkynylsulfinyl, C₁-C₆-alkylsulfonyl, C₁-C₆-haloalkylsulfonyl, C₂-C₆-alkenylsulfonyl, C₂-C₆-alkynylsulfonyl, C₁-C₆-alkoxysulfonyl, C₁-C₆-haloalkoxysulfonyl, C₂-C₆-alkenyloxysulfonyl, C₂-C₆-alkynyloxysulfonyl, phenyl, phenyloxy, phenylthio, phenylsulfinyl or phenylsulfonyl, where the five last mentioned substituents may be partially or fully halogenated and may carry one to three of the following groups:
nitro, cyano, hydroxyl, C₁-C₄-alkyl, C₁-C₄-haloalkyl, C₁-C₄-alkoxy, C₁-C₄-haloalkoxy, and
R² is hydrogen or a radical mentioned under R¹.

8. A hetaroyl derivative of the formula Ia as claimed in any of claims 1 to 4 and its N-oxide (formula Ia')

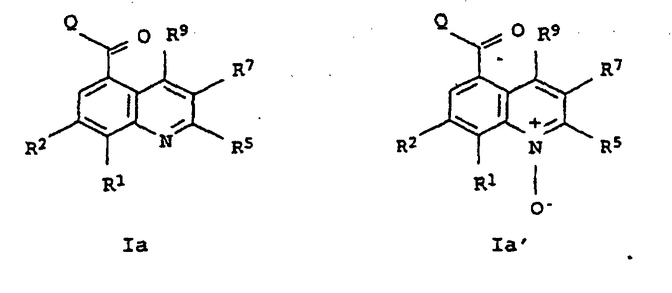

9. A hetaroyl derivative of the formula Ib as claimed in any of claims 1 to 4 and its N-oxide (formula Ib')

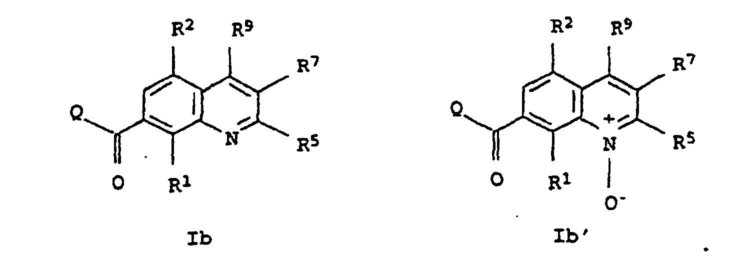

10. A hetaroyl derivative of the formula Ic as claimed in any of claims 1 to 4 and its N-oxide (formula Ic')

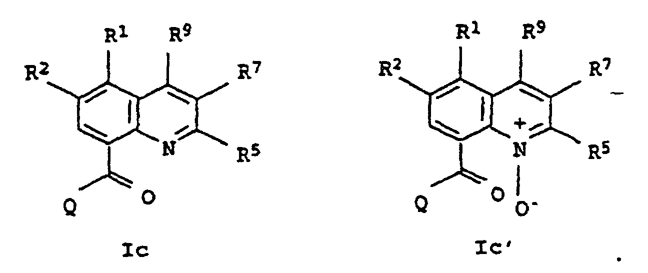

11. A hetaroyl derivative of the formula Id as claimed in any of claims 1 to 4 and its N-oxide (formula Id')

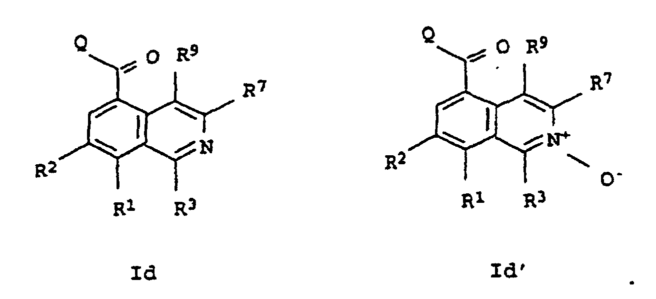

12. A hetaroyl derivative of the formula Ie as claimed in any of claims 1 to 4 and its N-oxide (formula Ie')

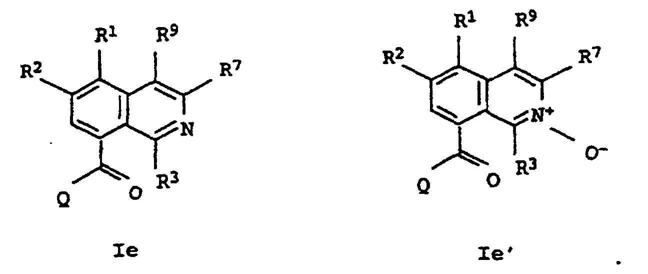

13. A hetaroyl derivative of the formula If (where Z = Z⁹) or Ig (where Z = Z¹⁰) as claimed in any of claims 1 to 3 or 6

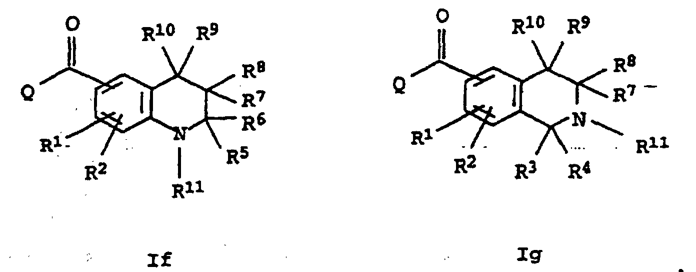

14. A process for preparing compounds of the formula I as claimed in any of claims 1 to 13, which comprises acylating a cyclohexane-1,3-dione of the formula II with an activated carboxylic acid IIIa or with a carboxylic acid IIIb 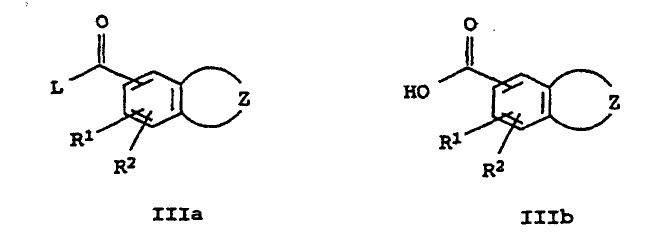 where the variables R¹, R², R¹⁴ to R¹⁹ and Z are as defined in claim 1 and L is a nucleophilically displaceable leaving group and rearranging the acylation product in the presence of a catalyst to give the compounds I.

15. A herbicidal composition, comprising a herbicidally effective amount of at least one hetaroyl derivative of the formula I or an agriculturally useful salt of I as claimed in any of claims 1 to 13 and at least one inert liquid and/or solid carrier and, if desired, at least one surfactant.

16. A process for preparing herbicidally active compositions as claimed in claim 15, which comprises mixing a herbicidally effective amount of at least one hetaroyl derivative of the formula I or an agriculturally useful salt of I as claimed in any of claims 1 to 13 and at least one inert liquid and/or solid carrier and, if desired, at least one surfactant.

17. A method for controlling unwanted vegetation, which comprises allowing a herbicidally effective amount of at least one hetaroyl derivative of the formula I or an agriculturally useful salt of I as claimed in any of claims 1 to 13 to act on plants, their habitat or on seeds.

18. The use of the hetaroyl derivative of the formula I and its agriculturally useful salts as claimed in any of claims 1 to 13 as herbicides.

## Revendications

1. Dérivés hétéroaroyliques de formule I 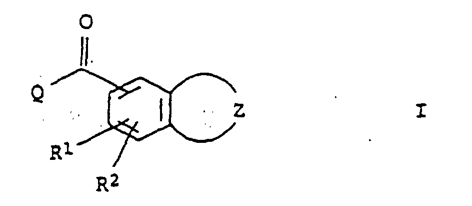 dans laquelle les symboles ont les significations suivantes :
R¹, R² : l'hydrogène, des groupes nitro, des halogènes, des groupes cyano, thiocyano, hydroxy, mercapto, alkyle en C1-C6, halogénoalkyle en C1-C6, alcényle en C2-C6, alcynyle en C2-C6, alcoxy en C1-C6, halogénoalcoxy en C1-C6, alcényloxy en C26C6, alcynyloxy en C2-C6, alkylthio en C1-C6, halogénoalkylthio en C1-C6, alcynylthio en C3-C6, alcynylthio en C2-C6, alkylsulfinyle en C1-C6, halogénoalkylsulfinyle en C1-C6, alcénylsulfinyle en C2-C6, alcynylsulfinyle en C2-C6, alkylsulfonyle en C1-C6, halogénealkylsulfonyle en C1-C6, alcénylsulfonyle en C2-C6, alcynylsulfonyle en C2-C6, alcoxysulfonyle en C1-C6, halogérioalcoxysulfonyle en C1-C6, alcynyloxysulfonyle en C2-C6, alcényloxysulfonyle en C2-C6, phényle, phényloxy, phénylthio, phénylsulfinyle ou phénylsulfonyle, les cinq substituants mentionnés en dernier pouvant être halogénés en totalité ou en partie et pouvant porter eux-mêmes un à trois des groupes suivants :
nitro, cyano, hydroxy, alkyle en C1-C4, halogénoalkyle en C1-C4, alcoxy en C1-C4, halogénoalcoxy en C1-C4 ;
Z : un motif de structure du groupe Z¹ à R¹² 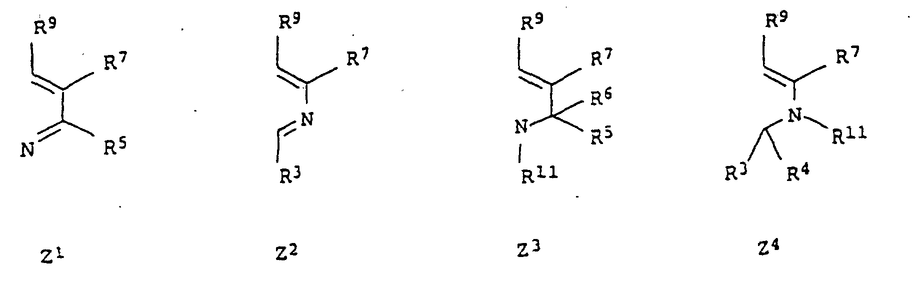 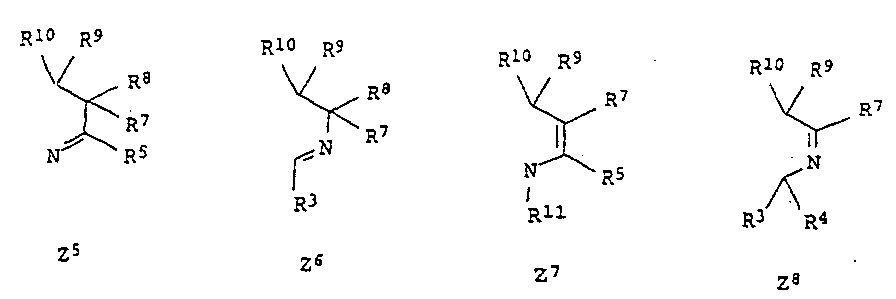 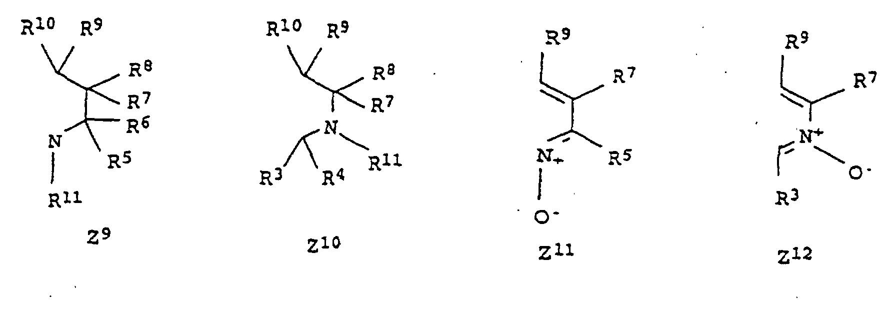 dans lesquels
R³, R⁵, R⁷, R⁹ représentent des groupes nitro, cyano, hydroxy, mercapto, alcynyle en C2-C4, alcynyle en C2-C4, alcynyloxy en C2-C4, alcynyloxy en C2-C4, alcénylthio en C2-C4, alcynylthio en C2-C4, alkylsulfinyle en C1-C4, halogénoalkylsulfinyle en C1-C4, aicénylsulfmyle en C2-C4, alcynylsulfinyle en C2-C4, alkylsulfonyle en C1-C4, halogénoalkylsulfonyle en C1-C4, alcénylsulfonyle en C2-C4, alcynylsulfonyle en C2-C4, alcoxysulfonyle en C1-C4, halogénoalcoxysulfonyle en C1-C4, alcényloxysulfonyle en C2-C4, alcynyloxy. sulfonyle en C2-C4, -NR¹²R¹³, -CO₂R¹², CONR¹²R¹³, phényle, phénoxy, phénylthio, phénylsulfinyle ou phénylsulfonyle, les cinq substituants mentionnés en dernier pouvant eux-mêmes être halogénés en totalité ou en partie et pouvant porter un à trois des groupes suivants :
nitro, cyano, hydroxy, alkyle en C1-C4, halogénoalkyle en C3-C4, alcoxy en C1-C4, halogénoalcoxy en C1-C4 ;
ainsi que les groupes mentionnés en référence à R⁴ ;
R⁴, R⁶, R⁸, R¹⁰ :
l'hydrogène, des halogènes, des groupes alkyle en C1-C4, halogénoalkyle en C1-C4, alcoxy en C1-C4, halogénoalcoxy en C1-C4, alkylthio en C1-C4 ou halogénoalkylthio en C1-C4 ;
ou bien
un motif -CR³R⁴-, -CR⁵R⁶-, -CR⁷R⁸-, -CR⁹R¹⁰- peut être remplacé par C=O ou C=NR¹³ ;
R¹¹ : l'hydrogène, un groupe alkyle en C1-C6, halogénoalkyle en C1-C6, alcényle en C3-C6, alcynyle en C3-C6, (alkyle en C1-C6)carbonyle, (halogénoalkyle en C1-C6)carbonyle, -CO₂R¹², -CONR¹²R¹³ ou SO₂R¹² ;
R¹² : l'hydrogène, un groupe alkyle en C1-C6, halogénoalkyle en C1-C6, alcényle en C3-C6, alcynyle en C3-C6 ou phényle, ce dernier pouvant être halogéné en totalité ou en partie et pouvant porter un à trois des groupes suivants :
nitro, cyano, hydroxy, alkyle en C1-C4, halogénoalkyle en C1-C4, alcoxy en C1-C4, halogénoalcoxy en C1-C4 ;
R¹³ : un groupe alcoxy en C1-C6, halogénoalcoxy en C1-C6, alcényloxy en C3-C6, alcynyloxy en C3-C6 ou l'un des groupes mentionnés en référence à R¹² ;
Q représente un noyau cyclohexane-1,3-dione relié en position 2 et répondant à la formule II 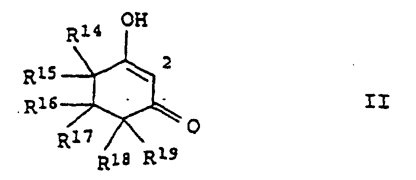 dans laquelle
R¹⁴, R¹⁵, R¹⁷ et R¹⁹ représentent l'hydrogène ou des groupes alkyle en C1-C4 ;
R¹⁶ représente l'hydrogène, un groupe alkyle en C1-C4 ou cycloalkyle en C3-C4, les deux groupes mentionnés en dernier pouvant le cas échéant porter un à trois des substituants suivants :
halogéno, alkylthio en C1-C4 ou alcoxy en C1-C4 ; ou bien
un groupe tétrahydropyran-3-yle, tétrahydropyran-4-yle, tétrahydrothiopyran-3-yle, 1,3-dioxolan-2-yle, 1,3-dioxan-2-yle, 1,3-oxathiolan-2-yle, 1,3-oxathian-2-yle, 1,3-diothiolin-2-yle ou 1,3-dithian-2-yle, les six groupes mentionnés en dernier pouvant le cas échéant porter un à trois substituants alkyle en C1-C4 ;
R¹⁸ représente l'hydrogène, un groupe alkyle en C1-C4 ou (alcoxy en C1-C6)carbonyle ; ou bien
R¹⁶ et R¹⁹ représentent ensemble une liaison ou un noyau carbocyclique de trois à six chaînons ;
ou bien
le motif -CR¹⁶R¹⁷ - peut être remplacé par C=O,
et leurs sels aptes aux applications agricoles.

2. Dérivés hétéroaroyliques de formule I selon revendication 1 dans laquelle CR⁹R¹⁰ a une signification autre que C=O ou C=NR¹³ lorsque Z, simultanément, représente Z⁹ et que le "fragment -QCO" est en position para par rapport à l'azote du motif Z.

3. Dérivés hétéroaroyliques de formule I selon les revendications 1 et 2 dans laquelle les symboles ont les significations suivantes :
R¹, R² : l'hydrogène, des groupes nitro, des halogènes, des groupes cyano, thiocyano, hydroxy, mercapto, alkyle en C1-C6, halogénoalkyle en C1-C6, alcényle en C2-C6, alcynyle en C2-C6, alcoxy en C1-C6, halogénoalcoxy en C1-C6, alcényloxy en C2-C6, alcynyloxy en C2-C6, alkylthio en C1-C6, halogénoalkylthio en C1-C6, alcénylthio en C2-C6, alcynylthio en C2-C6, alkylsulfinyle en C1-C6, halogénoalkylsulfinyle en C1-C6, alcénylsulfinyle en C2-C6, alcynylsulfinyle en C2-C6, alkylsulfonyle en C1-C6, halogénoalkylsulfonyle en C1-C6, alcénylsulfinyle en C2-C6, alcynylsulfinyle en C2-C6, alcoxysulfonyle en C1-C6, halogénoalcoxysulfonyle en C1-C6, alcényloxysulfonyle en C2-C6, alcynyloxysulfonyle en C2-C6, phényle, phényloxy, phénylthio, phénylsulfinyle ou phénylsulfonyle, les cinq substituants mentionnés en dernier pouvant être halogénés en totalité ou en partie et pouvant porter un à trois des groupes suivants :
nitro, cyano, hydroxy, alkyle en C1-C4, halogénoalkyle en C1-C4, alcoxy en C1-C4, halogénoalcoxy en C1-C4 ;
Z : un motif de structure du groupe Z¹ à Z¹² 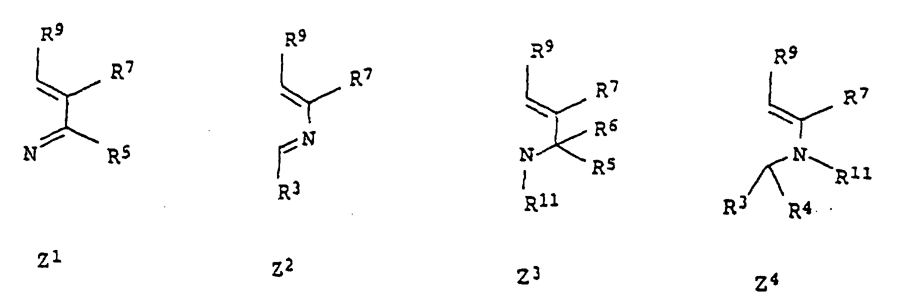 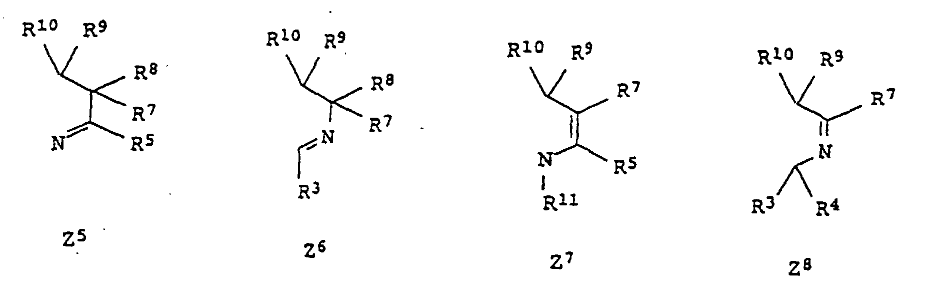 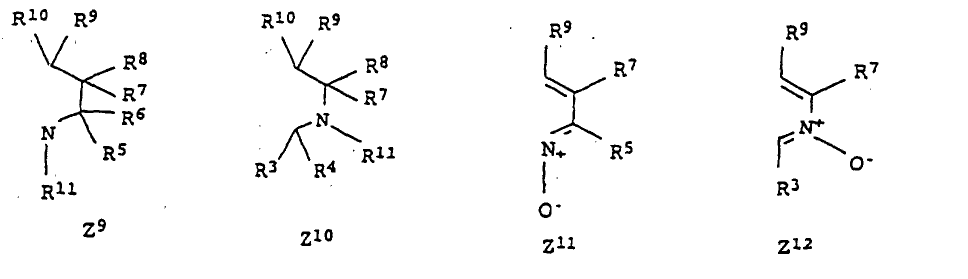 dans lesquels
R³, R⁵, R⁷, R⁹ représentent l'hydrogène, des halogènes, des groupes alkyle en C1-C4, halogénoalkyle en C1-C4, alcoxy en C1-C4, halogénoalcoxy en C1-C4, alkylthio en C1-C4, halogénoalkylthio en C1-C4, nitro, cyano, hydroxy, mercapto, alcényle en C2-C4, alcynyle en C2-C4, alcényloxy en C2-C4, alcynyloxy en C2-C4, alcénylthio en C2-C4, alcynylthio en C2-C4, alkylsulfinyle en C1-C4, halogénoalkylsulfinyle en C1-C4, alcénylsulfinyle en C2-C4, alcynylsulfinyle en C2-C4, alkylsulfonyle en C1-C4, halogénoalkylsulfonyle en C1-C4, alcénylsulfonyle en C2-C4, alcynylsulfonyle en C2-C4, alcoxy sulfonyle en C1-C4, halogénoalcoxysulfonyle en C1-C4, alcényloxysulfonyle en C2-C4, alcynyloxysulfonyle en C2-C4, -NR¹²R¹³, -CO₂R¹², -CONR¹²R¹³, phényle, phénoxy, phénylthio, phénylsulfinyle ou phénylsulfonyle, les cinq substituants mentionnés en dernier pouvant être halogénés en totalité ou en partie et pouvant porter un à trois des groupes suivants :
nitro, cyano, hydroxy, alkyle en C1-C4, halogénoalkyle en C1-C4, alcoxy en C 1-C4, halogénoalcoxy en C 1-C4 ;
R⁴, R⁶, R⁸, R¹⁰ l'hydrogène ;
R¹¹: l'hydrogène, un groupe alkyle en C1-C6, halogénoalkyle en C1-C6, alcényle en C3-C6, alcynyle en C3-C6, (alkyle en C1-C6)carbonyle, (halogénoalkyle en C1-C6)carbonyle, -CO₂R¹², -CONR¹²R¹³ ou SO₂R¹² ;
R¹²: l'hydrogène, un groupe alkyle en C1-C6, halogénoalkyle en C1-C6, alcényle en C3-C6, alcynyle en C3-C6 ou phényle, ce dernier pouvant être halogéné en totalité ou en partie et pouvant porter un à trois des groupes suivants :
nitro, cyano, hydroxy, alkyle en C1-C4, halogénoalkyle en C1-C4, alcoxy en C1-C4, halogénoalcoxy en C1-C4 ;
R¹³ : un groupe alcoxy en C1-C6, halogénoalcoxy en C1-C6, alcényloxy en C3-C6, alcynyloxy en C3-C6 ou l'un des groupes mentionnés en référence à R¹² ;

4. Dérivés hétéroaroyliques de formule I selon les revendications 1 à 3, dans laquelle Z représente Z¹, Z², Z¹¹ ou Z¹².

5. Dérivés hétéroaroyliques de formule I selon les revendications 1 à 3, dans laquelle Z représente Z³, Z⁴, Z⁵, Z⁶, Z⁷ ou Z⁸.

6. Dérivés hétéroaroyliques de formule I selon les revendications 1 à 3, dans laquelle Z représente Z⁹ ou Z¹⁰.

7. Dérivés hétéroaroyliques de formule I selon les revendications 1 à 6, dans laquelle
R¹ représente un groupe nitro, un halogène, un groupe cyano, thiocyano, hydroxy, mercapto, alkyle en C1-C4, halogénoalkyle en C1-C6, alcényle en C2-C6, alcynyle en C2-C6, alcoxy en C1-C6, halogénoalcoxy en C1-C6, alcényloxy en C2-C6, alcynyloxy en C2-C6, alkylthio en C1-C6, halogénoalkylthio en C1-C6, alcénylthio en C2-C6, alcynylthio en C2-C6, alkylsulfinyle en C1-C6, halogénoalkylsulfinyle en C1-C6, alcénylsulfinyle en C2-C6, alcynylsulfinyle en C2-C6, alkylsulfonyle en C1-C6, halogénoalkylsulfonyle en C1-C6, alcénylsulfonyle en C2-C6, alcynylsulfonyle en C2-C6, alcoxysulfonyle en C1-C6, halogénoalcoxysulfonyle en C1-C6, alcényloxysulfonyle en C2-C6, alcynyloxysulfonyle en C2-C6, phényle, phényloxy, phénylthio, phénylsulfonyle ou phénylsulfonyle, les cinq substituants mentionnés en dernier pouvant être halogénés en totalité ou en partie et pouvant porter un à trois des substituants suivants :
nitro, cyano, hydroxy, alkyle en C1-C4, halogénoalkyle en C1-C4, alcoxy en C1-C4, halogénoalcoxy en C1-C4 et
R² représente l'hydrogène ou l'un des groupes mentionnés en référence à R¹.

8. Dérivés hétéroaroyliques de formule Ia, selon les revendications 1 à 4, et leurs N-oxydes (de formule Ia')

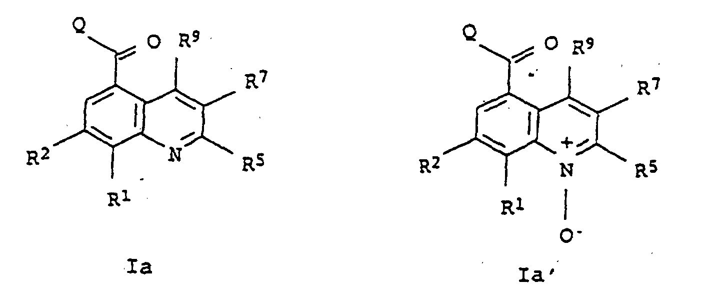

9. Dérivés hétéroaroyliques de formule Ib, selon les revendications 1 à 4, et leurs N-oxydes (de formule Ib')

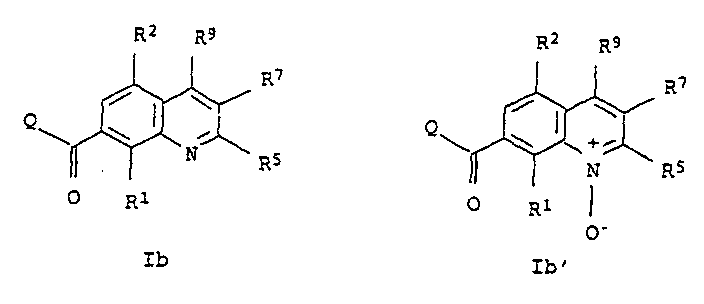

10. Dérivés hétéroaroyliques de formule Ic, selon les revendications 1 à 4, et leurs N-oxydes (de formule Ic')

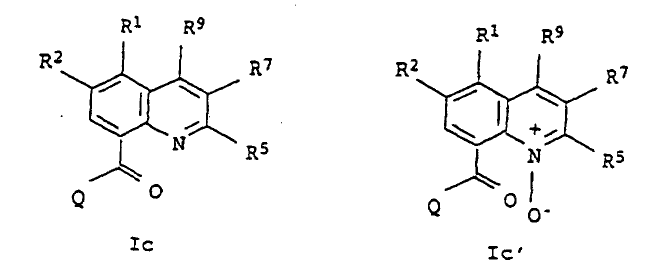

11. Dérivés hétéroaroyliques de formule Id, selon les revendications 1 à 4, et leurs N-oxydes (de formule Id')

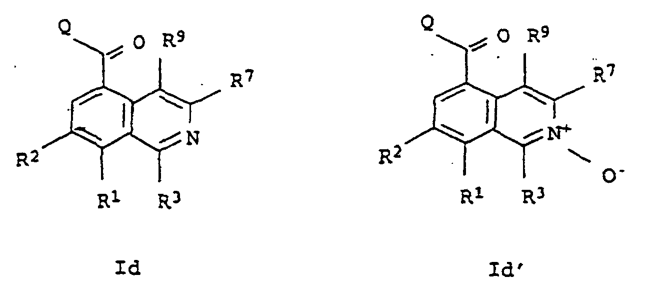

12. Dérivés hétéroaroyliques de formule Ie, selon les revendications 1 à 4, et leurs N-oxydes (de formule Ie')

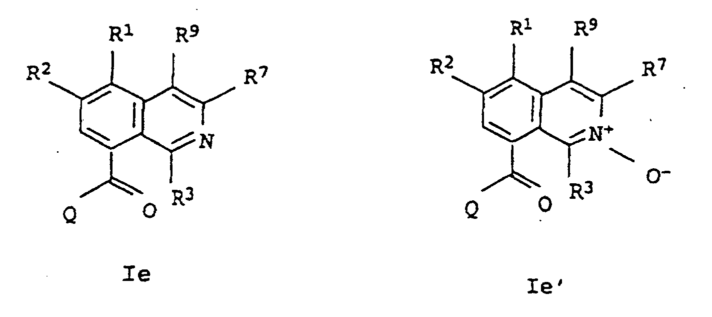

13. Dérivés hétéroaroyliques de formule If (avec Z = Z⁹) ou Ig (avec Z = Z¹⁰) selon les revendications 1 à 3 ou 6

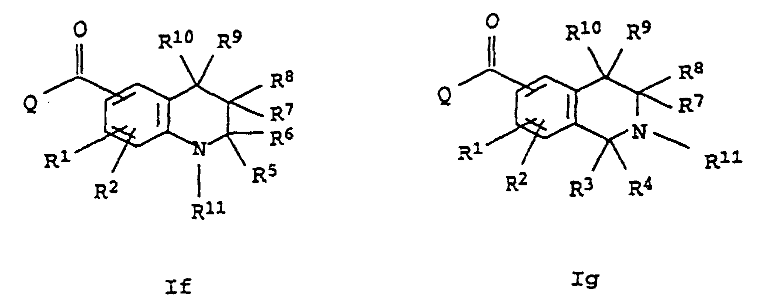

14. Procédé pour la préparation des composés de formule I selon les revendications 1 à 13, **caractérisé par le fait que** l'on acyle une cyclohexane-1,3-dione de formule II à laide d'un acide carboxylique activé IIIa ou d'un acide carboxylique IIIb 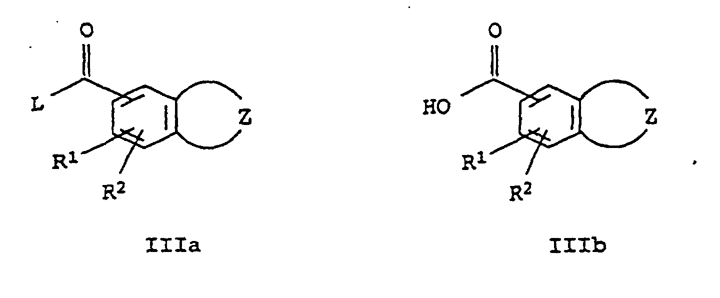 pour lesquels les symboles R¹, R², R¹⁴ à R¹⁹ et Z ont les significations indiquées dans la revendication 1 et L est un groupe éliminable par échange nucléophile, puis on soumet le produit d'acylation à transposition en les composés I en présence d'un catalyseur.

15. Produit herbicide contenant une quantité herbicide efficace d'au moins un dérivé hétéroaroylique de formule I ou d'un sel de I apte aux applications agricoles, selon les revendications 1 à 13, et au moins un véhicule liquide et/ou solide inerte ainsi que, si on le désire, au moins une substance tensio-active.

16. Procédé pour la préparation des produits herbicides selon la revendication 15, **caractérisé par le fait que** l'on mélange une quantité herbicide efficace d'au moins un dérivé hétéroaroylique de formule I ou d'un sel de I apte aux applications agricoles selon les revendications 1 à 13 et au moins un véhicule liquide et/ou solide inerte ainsi que, si on le désire, au moins une substance tensio-active.

17. Procédé pour combattre les croissances de végétaux indésirables, **caractérisé par le fait que** l'on fait agir une quantité herbicide efficace d'au moins un dérivé hétéroaroylique de formule I ou d'un sel de I apte aux applications agricoles selon les reven dications 1 à 13 sur les végétaux, leur habitat ou leurs semences.

18. Utilisation des dérivés hétéroaroyliques de formule I et de leurs sels aptes aux applications agricoles selon les revendications 1 à 13 en tant qu'herbicides.
